# EUROPEAN PATENT APPLICATION

(11) **EP 4 610 233 A1**
(43) Date of publication of application: **03.09.2025**
(21) Application number: 23882181.3
(22) Date of filing: 03.08.2023
(51) Int. Cl.: C02F 1/00, A01N 59/06, A01N 59/16, A01N 65/08, A01P 1/00, A01P 3/00, A61G 12/00, A61M 1/00, C02F 11/00

(54) **TREATMENT AGENT, WASTE LIQUID TREATMENT IMPLEMENT, AND DOWNSTREAM-SIDE WASTE LIQUID TREATMENT IMPLEMENT**

(30) Priority: 28.10.2022 JP 2022173428; 28.10.2022 JP 2022173431
(71) Applicant: EXCELSIOR INC., Tokyo 154-0023 (JP)
(72) Inventor: ADACHI, Kanichi, Tokyo 154-0023 (JP); URANO, Shin-ya, Yokohama-shi, Kanagawa 227-0044 (JP)
(74) Representative: Winter, Brandl - Partnerschaft mbB
(86) International application number: PCT/JP2023/028444
(87) International publication number: WO 2024/089965

(57) **Abstract**

Provided is a novel treatment agent that can reduce foul odors including indole and skatole, and also provided is a novel treatment agent that can be applied to at least one of operation rooms, endoscopy rooms, emergency evacuation sites where flushing toilets cannot be used, infrastructure-lacking regions such as mountains and beaches, living rooms for people who require nursing care and cannot use a toilet by themselves, and the like. The treatment agent is a treatment agent for treating waste liquid and contains slaked lime and pepper.

## Description

### TECHNICAL FIELD

The present invention relates to a treatment agent, a waste liquid treatment implement, and a downstream-side waste liquid treatment implement.

### BACKGROUND ART

Proper disposal of excrement is important for maintaining a hygienic and pleasant environment; however, that may be difficult in a variety of situations and places. Examples thereof include emergency evacuation sites in the event of an emergency disaster where a flushing toilet cannot be used, infrastructure-lacking regions such as mountains and beaches, living rooms for persons who require nursing care and cannot use a toilet by themselves, and inside hospitals such as operation rooms and endoscopy rooms.

For example, to take the endoscopy room as an example, endoscopic examinations are divided into upper examinations, which are performed to examine the stomach and the like through the mouth or the nose, and lower examinations, which are performed to examine the rectum, large intestine, and small intestine through the anus. In both the upper and lower examinations, suction of body fluids is performed, and in some cases, since the examination is performed while rinsing with water, waste liquid mixed with excrement, blood, and body fluids are discharged. Waste liquid containing body fluids thus generated is subjected to waste disposal.

Patent Document 1 discloses a configuration in which a treatment agent for absorbing waste liquid that has flowed down through a waste liquid inlet pipe into a collecting container, the treatment agent being composed entirely of a macromolecular water-absorbing polymer or having a macromolecular water-absorbing polymer as a main component, is placed inside the collecting container. As a result, the waste liquid collected by suction in the collecting container in a sealed state is supplied with the treatment agent that has been placed inside the collecting container and is successively gelled. For this reason, there is no risk that the waste liquid collected by suction may scatter outside the collecting container, and the system is excellent in terms of safety.

### CITATION LIST

### PATENT DOCUMENT

Patent Document 1: JP 02-157083 A

### DISCLOSURE OF THE INVENTION

Patent Document 1 mentions only about the problem of waste liquid treatment; however, there is also a problem of foul odors originating from excrement. Particularly, the foul odors of indole, skatole, and the like are very unpleasant and cause headaches for medical workers.

Thus, an object of the invention is to provide a novel treatment agent capable of reducing foul odors including indole and skatole, and to eventually provide a novel treatment agent that can be applied to at least one of waste liquid treatments in operation rooms, endoscopy rooms, emergency evacuation sites where flushing toilets cannot be used, infrastructure-lacking regions such as mountains and beaches, living rooms for people who require nursing care and cannot use a toilet by themselves, and the like.

An aspect of the invention is a treatment agent containing slaked lime and pepper.

According to the treatment agent related to an aspect of the present invention, a novel treatment agent capable of reducing foul odors including indole and skatole is provided, and eventually, a novel treatment agent that can be applied to at least one of waste liquid treatments in operation rooms, endoscopy rooms, emergency evacuation sites where a flushing toilet cannot be used, infrastructure-lacking regions such as mountains and beaches, living rooms of persons who require nursing care and cannot use a toilet by themselves, and the like, can be provided. Furthermore, such a treatment agent can also be used to treat excrement, blood, vomit, or body fluids discharged from humans or animals, and to treat organic sludge such as food waste.

### BRIEF DESCRIPTION OF DRAWINGS

Fig. 1 is a color photograph showing a proliferation inhibitory effect of sample A on spore-forming bacteria;
Fig. 2 is a schematic perspective view illustrating a waste liquid treatment implement and a downstream-side waste liquid treatment implement according to embodiments of the present invention;
Fig. 3 is a front view of Fig. 2;
Fig. 4 is an exploded perspective view showing a configuration of the waste liquid treatment implement according to Fig. 2;
Fig. 5 is a cross-sectional view taken along the height direction, illustrating the inside of the waste liquid treatment implement according to Fig. 3;
Fig. 6 is a perspective view illustrating a lid part and a partition member constituting the waste liquid treatment implement according to Fig. 3;
Fig. 7 is a perspective view illustrating a lid part and a partition member cut at a position different from that in Fig. 6;
Fig. 8 is an exploded perspective view illustrating the configuration of the partition member;
Fig. 9 is a plan view illustrating the partition member;
Fig. 10 is an exploded perspective view illustrating the configuration of the downstream-side waste liquid treatment implement shown in Fig. 2;
Fig. 11 is a cross-sectional view taken along the height direction, illustrating the inside of the downstream-side waste liquid treatment implement;
Fig. 12 a perspective view illustrating a lid part, a connecting member, and an attachment member constituting the downstream-side waste liquid treatment implement, with the lid part shown in a cutaway state;
Fig. 13 is an enlarged view of Fig. 11 illustrating the lid part, the connecting member, the attachment member, and a displacement member, and showing a situation in which a gas or the like utilized for suction flows through the inside of the attachment member;
Fig. 14 is an enlarged view illustrating a state in which the position of the displacement member in Fig. 13 is different from that in Fig. 13;
Fig. 15 is an exploded perspective view of a waste liquid treatment implement showing a modification example of Fig. 4;
Fig. 16 is an exploded perspective view illustrating a downstream-side waste liquid treatment implement according to a modification example of Fig. 10;
Fig. 17 is a perspective view illustrating a partition member according to a modification example;
Fig. 18 is an exploded perspective view of Fig. 17;
Fig. 19 is a plan view of Fig. 17;
Fig. 20 is a perspective view illustrating a partition member according to a modification example;
Fig. 21 is a diagram illustrating a modification example of Fig. 13;
Fig. 22 is a diagram illustrating a modification example of Fig. 14;
Fig. 23 is a perspective view illustrating a modification example of Fig. 1;
Fig. 24 is an exploded perspective view illustrating a capture assembly attached to a waste liquid treatment implement according to a modification example; and
Fig. 25 is a diagram illustrating the inside of the capture assembly.

### BEST MODES FOR CARRYING OUT THE INVENTION

### <First invention>

First, a first invention will be described.

In the present specification, the expression "X to Y" representing a range means "X or more and Y or less". When a plurality of the expression "X to Y" are described, for example, it is described such that "X1 to Y1, or X2 to Y2", the description includes all of the disclosure showing each numerical value as an upper limit, the disclosure showing each numerical value as a lower limit, and the disclosure showing combinations of those upper limits and lower limits (that is, a lawful basis for amendment). Specifically, amendments to X1 or more, amendments to Y2 or less, amendments to X1 or less, amendments to Y2 or more, amendments to X1 through X2, amendments to X1 through Y2, and the like must all be deemed lawful. Furthermore, unless particularly stated otherwise, the operations and measurement of physical properties and the like are carried out under the conditions of room temperature (20°C to 25°C) and relative humidity of 40% to 50%RH. Unless particularly described otherwise, the unit % means % by mass.

### <Treatment agent>

An aspect of the present invention is a treatment agent for treating waste liquid, which contains slaked lime and pepper. Such an aspect can reduce foul odors including indole and skatole, and can be eventually applied to at least one of waste liquid treatments in operation rooms, endoscopy rooms, emergency evacuation sites where flushing toilets cannot be used, infrastructure-lacking regions such as mountains and beaches, living rooms for people who require nursing care and cannot use a toilet by themselves, and the like, where this problem is prevalent.

In an embodiment of the present invention, the above-described waste liquid is excrement, blood, vomit, or body fluids, which are discharged from a human being or an animal. In an embodiment of the present invention, the waste liquid is a liquid (excrement, blood, body fluids, or the like) discharged from a patient during medical practice such as endoscopic examinations (upper examinations and lower examinations). In an embodiment of the present invention, the waste liquid may be human excrement (feces and/or urine) (in the case of feces only, the waste liquid may contain liquid such as water).

In an embodiment of the present invention, the above-described waste liquid contains at least one selected from the group consisting of n-butyric acid, n-valeric acid, isovaleric acid, indole, and skatole as a foul odor component. In an embodiment of the present invention, the waste liquid contains at least one of indole and skatole as the foul odor component. The treatment agent according to the present invention is effective for deodorization (including odor reduction) of fatty acids such as n-butyric acid, n-valeric acid, and isovaleric acid as well as at least one of indole and skatole in particular. In an embodiment of the present invention, the treatment agent is a treatment agent for deodorizing at least one of indole and skatole.

### (Slaked lime)

The treatment agent according to an aspect of the present invention contains slaked lime. In an embodiment of the present invention, the content ratio (% by mass) of slaked lime is 0.1% by mass or more, 0.5% by mass or more, 1% by mass or more, 2% by mass or more, 3% by mass or more, 5% by mass or more, 6% by mass or more, 7% by mass or more, 8% by mass or more, 9% by mass or more, 10% by mass or more, 12% by mass or more, 15% by mass or more, 17% by mass or more, 20% by mass or more, or 30% by mass or more, in the treatment agent. In an embodiment of the present invention, the content ratio (% by mass) of slaked lime is 70% by mass or less, 60% by mass or less, 50% by mass or less, 40% by mass or less, 35% by mass or less, 30% by mass or less, 28% by mass or less, 25% by mass or less, 20% by mass or less, 15% by mass or less, 14% by mass or less, 13% by mass or less, 10% by mass or less, 9% by mass or less, 8% by mass or less, 7% by mass or less, 6% by mass or less, 5% by mass or less, 4% by mass or less, or 3% by mass or less, in the treatment agent. In an embodiment of the present invention, the content ratio (% by mass) of slaked lime is 0.1% to 40% by mass, 0.5% to 35% by mass, 1% to 30% by mass, 3% to 28% by mass, 5% to 25% by mass, or 7% to 15% by mass, in the treatment agent.

In an embodiment of the present invention, the average particle size of slaked lime is, for example, 10 µm or more, or 50 µm or more. In an embodiment of the present invention, the average particle size of slaked lime is, for example, 1000 µm or less, 500 µm or less, or 300 µm or less. Incidentally, unless particularly stated otherwise, the "average particle size" described in the present specification means an average value obtained by arbitrarily selecting a statistically reliable number of particles (or 100, 200, 300, or 1000 particles), measuring the longest particle size for each particle under a microscope, and determining the arithmetic mean of those particle sizes. In order to obtain a desired average particle size, the particles may be sieved or the like as appropriate. In an embodiment of the present invention, a method for preparing slaked lime is preferably a method of purchasing a commercially available product, and for example, a commercially available product available from Ube Material Industries, Ltd. is preferred.

### (Pepper)

The treatment agent according to an aspect of the present invention contains pepper. Pepper is the fruit of Piper nigrum pepper tree. In an embodiment of the present invention, pepper may be any of black pepper, white pepper, green pepper, and pink pepper.

In an embodiment of the present invention, pepper is not in the form of piperine extract.

In an embodiment of the present invention, the average particle size of pepper is, for example, 10 µm or more, or 50 µm or more. In an embodiment of the present invention, the average particle size of pepper is, for example, 1000 µm or less, 500 µm or less, or 300 µm or less.

In an embodiment of the present invention, the content ratio (% by mass) of pepper is 0.05% by mass or more, 0.1% by mass or more, 0.5% by mass or more, 1% by mass or more, 1.5% by mass or more, 1.6% by mass or more, 1.7% by mass or more, 1.8% by mass or more, 2% by mass or more, 2.5% by mass or more, 3% by mass or more, 3.2% by mass or more, 3.5% by mass or more, 3.8% by mass or more, 4% by mass or more, 5% by mass or more, 6% by mass or more, 7% by mass or more, 8% by mass or more, 9% by mass or more, 10% by mass or more, 12% by mass or more, 14% by mass or more, 16% by mass or more, 18% by mass or more, or 20% by mass or more, in the treatment agent. According to an embodiment of the present invention, the content ratio (% by mass) of pepper is 55% by mass or less, 45% by mass or less, 40% by mass or less, 30% by mass or less, 25% by mass or less, 20% by mass or less, 15% by mass or less, 10% by mass or less, 9% by mass or less, 8% by mass or less, 7% by mass or less, 6% by mass or less, 5.5% by mass or less, 5% by mass or less, 4% by mass or less, 3.8% by mass or less, 3.5% by mass or less, or 3% by mass or less, in the treatment agent.

According to an embodiment of the present invention, the content ratio (% by mass) of pepper is 0.05% to 30% by mass, 0.1% to 25% by mass, 0.5% to 20% by mass, 1% to 15% by mass, 1.5% to 10% by mass, 1.6% to 9% by mass, 1.7% to 8% by mass, 1.8% to 7% by mass, 1.8% to 6% by mass, 1.8% to 5% by mass, 1.8% to 4.5% by mass, or 1.8% to 3.8% by mass, in the treatment agent.

According to an embodiment of the present invention, these content ratios (% by mass) may be values obtained by calculation including a binder in the treatment agent, or values obtained by calculation excluding a binder from the treatment agent. Incidentally, the content ratios (% by mass) of components in the treatment agent described in the present specification may be values obtained by calculation including a binder in the treatment agent, or values obtained by calculation excluding a binder from the treatment agent, in the same manner as described above.

According to an embodiment of the present invention, the treatment agent may or may not contain a binder. A preferred range of the content ratio (% by mass) of each component in the treatment agent may vary depending on the presence or absence of a binder.

### (Water-absorbing polymer)

The treatment agent of an embodiment of the present invention contains a water-absorbing polymer. As a water-absorbing polymer is included, a treatment target material that has been treated by absorbing moisture and the like is likely to solidify (likely to become jelly-like). The term "water-absorbing polymer (water-absorbing resin)" in the present specification refers to a macromolecular gelling agent having a water swelling capacity (CRC) of 5 g/g or more as defined by ERT441.2-02, and containing 50% by mass or less of a water-soluble component (Ext) defined by ERT470.2-02.

In an embodiment of the present invention, specific examples of the water-absorbing polymer include, for example, starch-based water-absorbing polymers such as a starch-acrylonitrile graft polymer hydrolysate, and a starch-acrylic acid graft polymer; cellulose-based water-absorbing polymers such as a cellulose-acrylonitrile graft polymer and a cellulose-styrene sulfonic acid graft copolymer; polysaccharide-based water-absorbing polymers such as pectin; protein-based water-absorbing polymers such as collagen; polyvinyl alcohol-based water-absorbing polymers such as a crosslinked polyvinyl alcohol polymer; acrylic water-absorbing polymers such as a sodium polyacrylate crosslinked body, an acrylic acid polymer partial sodium salt crosslinked product, and a sodium acrylate-vinyl alcohol copolymer; maleic anhydride-based water-absorbing polymers, vinylpyrrolidone-based water-absorbing polymers; and polyether-based water-absorbing polymers such as a crosslinked polyethylene glycol-diacrylate polymer. These water-absorbing polymers may be used singly, or two or more kinds thereof may be used in combination. Furthermore, these water-absorbing polymers may be synthesized, or commercially available products may be used. Examples of the commercially available products include, for example, AQUA KEEP (registered trademark) SA (manufactured by Sumitomo Seika Chemicals Co.**,** Ltd.), AQUALIC (registered trademark) CA (manufactured by Nippon Shokubai Co., Ltd.), SANFRESH (ST-250, ST-100, ST-573), AQUAPEARL (manufactured by San-Dia Polymer, Ltd.), HIMOSAB HS-960 (manufactured by Hymo Corporation), and EF POLYMER (EF Polymer Private Limited).

In an embodiment of the present invention, the water-absorbing polymer may be carboxymethyl cellulose. Carboxymethyl cellulose is a derivative of cellulose, in which carboxymethyl groups (-CH₂-COOH) are bonded to some of the hydroxy groups of gluconopyranose monomers constituting the skeleton of cellulose. Furthermore, this carboxymethyl cellulose may also be a carboxymethyl cellulose salt. Carboxymethyl cellulose is a thickening agent that has a high affinity for water and becomes a gel-like high viscosity body when mixed with water. In the present invention, when the treatment agent thickens, the effect of suppressing foul odors is enhanced, and the suppressive effect can be further maintained.

According to an embodiment of the present invention, the average particle size of the water-absorbing polymer is 1 to 1200 µm, 50 to 1100 µm, 10 to 1000 µm, 80 to 850 µm, 100 to 600 µm, 150 to 500 µm, or 200 to 400 µm.

According to an embodiment of the present invention, the content ratio (% by mass) of the water-absorbing polymer is 1% by mass or more, 5% by mass or more, 10% by mass or more, 15% by mass or more, 20% by mass or more, 25% by mass or more, 28% by mass or more, 30% by mass or more, 35% by mass or more, 40% by mass or more, 45% by mass or more, 50% by mass or more, 55% by mass or more, 60% by mass or more, or 65% by mass or more, in the treatment agent. According to an embodiment of the present invention, the content ratio (% by mass) of the water-absorbing polymer is 99% by mass or less, 95% by mass or less, 90% by mass or less, 85% by mass or less, 80% by mass or less, 75% by mass or less, 70% by mass or less, 65% by mass or less, 60% by mass or less, 55% by mass or less, 50% by mass or less, 45% by mass or less, 40% by mass or less, or 35% by mass or less, in the treatment agent.

According to an embodiment of the present invention, the content ratio (% by mass) of the water-absorbing polymer is 1% to 99% by mass, 5% to 95% by mass, 10% to 90% by mass, 15% to 85% by mass, 20% to 80% by mass, 25% to 75% by mass, 30% to 70% by mass, 35% to 65% by mass, 40% to 60% by mass, or 45% to 55% by mass, in the treatment agent. According to an embodiment of the present invention, the content ratio (% by mass) of the water-absorbing polymer is 25% to 60% by mass.

### (Zinc oxide)

According to an embodiment of the present invention, the treatment agent contains zinc oxide. Zinc oxide is an oxide of zinc represented by ZnO, and has an effect of adsorbing foul odor components such as ammonia and sulfides and deodorizing and eliminating odors. The zinc oxide can be freely selected from commercially available products. For example, zinc oxide type I (manufactured by Hakusui Tech Co., Ltd.), zinc oxide type II (manufactured by Hakusui Tech Co., Ltd.), PAZET AB, PAZET AK, and PAZET CK (all manufactured by Hakusui Tech Co., Ltd.).

According to an embodiment of the present invention, the content ratio (% by mass) of zinc oxide is 0.05% by mass or more, 0.1% by mass or more, 0.25% by mass or more, 0.5% by mass or more, 1% by mass or more, 1.5% by mass or more, 2% by mass or more, 2.5% by mass or more, 3% by mass or more, 3.5% by mass or more, 3.8% by mass or more, 4% by mass or more, 4.2% by mass or more, 4.4% by mass or more, 4.6% by mass or more, 4.8% by mass or more, 5% by mass or more, 5.2% by mass or more, 5.4% by mass or more, 5.6% by mass or more, 5.8% by mass or more, 6% by mass or more, 6.2% by mass or more, or 6.4% by mass or more, in the treatment agent.

According to an embodiment of the present invention, the content ratio (% by mass) of zinc oxide is 30% by mass or less, 25% by mass or less, 23% by mass or less, 20% by mass or less, 18% by mass or less, 15% by mass or less, 10% by mass or less, 9% by mass or less, 8% by mass or less, 7% by mass or less, 6% by mass or less, 5% by mass or less, 4% by mass or less, or 3% by mass or less, in the treatment agent.

According to an embodiment of the present invention, the content ratio (% by mass) of zinc oxide is 0.05% to 30% by mass, 0.1% to 25% by mass, 0.5% to 20% by mass, 1% to 15% by mass, 2% to 10% by mass, 2.5% to 8% by mass, 3% to 6% by mass, 3.5% to 5% by mass, or 3.8% to 4% by mass. According to an embodiment of the present invention, the content ratio (% by mass) of zinc oxide is 2.5% to 30% by mass.

### (Lignin)

According to an embodiment of the present invention, the treatment agent contains lignin. Lignin is a macromolecular phenolic compound involved in the lignification of higher plants and is also called a ligneous material. Lignin can exhibit an operating effect of adsorbing and decomposing ammonia and other odorous substances.

According to an embodiment of the present invention, the content ratio (% by mass) of lignin is 0.01% by mass or more, 0.05% by mass or more, 0.1% by mass or more, 0.5% by mass or more, 0.8% by mass or more, 1% by mass or more, 1.2% by mass or more, 1.5% by mass or more, or 1.8% by mass or more, in the treatment agent. According to an embodiment of the present invention, the content ratio (% by mass) of lignin is 30% by mass or less, 25% by mass or less, 20% by mass or less, 15% by mass or less, 10% by mass or less, 8% by mass or less, 6% by mass or less, 4% by mass or less, 3.5% by mass or less, 3.3% by mass or less, 3.1% by mass or less, 3.0% by mass or less, 2.8% by mass or less, 2.6% by mass or less, or 2.4% by mass or less, in the treatment agent.

According to an embodiment of the present invention, the content ratio (% by mass) of lignin is 0.01% to 30% by mass, 0.05% to 25% by mass, 1% to 20% by mass, 1.5% to 15% by mass, 1.5% to 10% by mass, 1.5% to 8% by mass, 1.5% to 6% by mass, 1.5% to 4% by mass, or 1.5% to 3% by mass, in the treatment agent. According to an embodiment of the present invention, the content ratio (% by mass) of lignin is 1% to 3.5% by mass.

According to an embodiment of the present invention, the average particle size of lignin is 1 to 500 µm, 5 to 300 µm, 10 to 100 µm, or 40 to 80 µm.

In an embodiment of the present invention, the method for preparing lignin is preferably a method of purchasing a commercially available product, and examples thereof include SAN X and VANILLEX (all manufactured by Nippon Paper Group).

### (Bentonite)

According to an embodiment of the present invention, the treatment agent contains bentonite. Bentonite contains a large amount of layered aluminum phyllosilicate, and has properties such as high viscosity, tacky adhesiveness, water absorption, and adsorption. Bentonite adsorbs cations of ammonia or the like, and suppresses the generation of ammonia and other odorous substances and adsorbs ammonia and other odorous substances. Furthermore, by combining lignin and bentonite, the cation adsorbing effect of bentonite and the unclarified, complicated three-dimensional network structure of lignin can work together to further exhibit effects of suppressing the generation of ammonia and other foul odor substances and adsorbing and decomposing ammonia and other foul odor substances.

According to an embodiment of the present invention, the content ratio (% by mass) of bentonite is 0.01% by mass or more, 0.05% by mass or more, 0.1% by mass or more, 0.5% by mass or more, 0.8% by mass or more, 1% by mass or more, 1.2% by mass or more, 1.5% by mass or more, or 1.8% by mass or more, in the treatment agent. According to an embodiment of the present invention, the content ratio (% by mass) of bentonite is 30% by mass or less, 25% by mass or less, 20% by mass or less, 15% by mass or less, 10% by mass or less, 8% by mass or less, 6% by mass or less, 4% by mass or less, 3.5% by mass or less, 3.3% by mass or less, 3.1% by mass or less, 2.8% by mass or less, 2.6% by mass or less, or 2.4% by mass or less, in the treatment agent.

According to an embodiment of the present invention, the content ratio (% by mass) of bentonite is 0.01% by mass to 30% by mass, 0.05% to 25% by mass, 0.1% to 20% by mass, 1% to 15% by mass, 1.5% to 10% by mass, 1.5% to 8% by mass, 1.5% to 6% by mass, 1.5% to 4% by mass, or 1.5% to 3.5% by mass, in the treatment agent. According to an embodiment of the present invention, the content ratio (% by mass) of bentonite is 1% to 3.5% by mass in the treatment agent.

According to an embodiment of the present invention, the average particle size of bentonite is 0.05 to 300 µm, 0.5 to 200 µm, 10 to 150 µm, 50 to 148 µm, or 80 to 145 µm. According to an embodiment of the present invention, the volume average particle size (D50) of bentonite is 0.05 to 300 µm, 0.5 to 200 µm, 10 to 150 µm, 50 to 148 µm, or 80 to 145 µm. According to an embodiment of the present invention, the mode diameter of bentonite is 0.05 to 300 µm, 0.5 to 200 µm, 10 to 150 µm, 50 to 148 µm, or 80 to 145 µm.

In an embodiment of the present invention, the method for preparing bentonite is preferably a method of purchasing a commercially available product, and examples thereof include KUNIPIA-F, KUNIMINE F, MOISTNITE S, MOISTNITE U (all manufactured by Kunimine Industries Co., Ltd.), and 250SA-B (all manufactured by Hoyo Bentonite Mining Co., Ltd.).

### (Zeolite)

According to an embodiment of the present invention, the treatment agent contains zeolite. The term "zeolite" is a generic term for minerals called zeolites, and about 40 types of natural zeolites have been discovered. By including zeolite, the pores of the zeolite can capture foul odors and suppress foul odors. The zeolite may be a natural substance or may be an artificial substance; however, from the viewpoint of availability, it is preferable that the zeolite is an artificial substance. Furthermore, the zeolite of the present invention is preferably a zeolite called mordenite in which extremely small cavities having a size of 5.5 to 8 Å, which are slightly larger than water or nitrogen molecules, are formed in a tunnel shape. In a case where a commercially available product of zeolite is purchased, Zeolite 2460, Zeolite 60, Zeolite CP, ZEOFILL (registered trademark) series, and the like manufactured by Shin Tohoku Chemical Industry Co., Ltd. are preferred. The average particle size of zeolite is not particularly limited, but is about 5 µm to 1.5 mm, about 8 µm to 1.2 mm, or about 10 to 100 µm. Zeolite contains SiO₂ (silicon oxide), Al₂O₃ (aluminum oxide), CaO (calcium oxide), Na₂O (sodium oxide), K₂O (potassium oxide), Fe₂O₃ (iron oxide), MgO (magnesium oxide), water of adhesion (H₂O), bound water (H₂O), and others in the amounts of about 70.5% by mass, 11.3% by mass, 2.6% by mass, 1.6% by mass, 1.3% by mass, 0.7% by mass, 0.1% by mass, 8.0% by mass, and 3.9% by mass, respectively; however, the zeolite is definitely not limited to such a composition, and a product prepared such that the content of each component is approximately 1% to 20%, and the total content is 100%, may be used. In the present invention, for example, a zeolite such as K[AlSi₂O₆] may be used.

According to an embodiment of the present invention, the content ratio (% by mass) of zeolite is 0.01% by mass or more, 0.05% by mass or more, 0.1% by mass or more, 0.5% by mass or more, 1% by mass or more, 1.5% by mass or more, 2% by mass or more, 3% by mass or more, 3.5% by mass or more, 4% by mass or more, 4.5% by mass or more, 5% by mass or more, 5.5% by mass or more, 6% by mass or more, 6.5% by mass or more, 7% by mass or more, 7.5% by mass or more, or 8% by mass or more, in the treatment agent. According to an embodiment of the present invention, the content ratio (% by mass) of zeolite is 40% by mass or less, 30% by mass or less, 25% by mass or less, 20% by mass or less, 15% by mass or less, 13% by mass or less, 12% by mass or less, 10% by mass or less, 9% by mass or less, or 8.5% by mass or less, in the treatment agent.

According to an embodiment of the present invention, the content ratio (% by mass) of zeolite is 0.01% to 40% by mass, 0.05% to 30% by mass, 0.1% to 20% by mass, 0.5% to 15% by mass, 1% to 13% by mass, 1.5% to 12% by mass, 2% to 9% by mass, 3% to 9% by mass, or 3.5% to 8.5% by mass, in the treatment agent. According to an embodiment of the present invention, the content ratio (% by mass) of zeolite is 1% to 20% by mass.

### (Humic acid)

According to an embodiment of the present invention, the treatment agent contains humic acid. Humic acid may be a final product formed through the decomposition of plants and the like by microorganisms, and may be an organic fraction that is not classified as sugar, carbohydrate, protein, lipid, or the like. Humic acid has strong chelating power for metal ions, has many functional groups, and can produce sugars and amino acids through hydrolysis.

According to an embodiment of the present invention, the average particle size of a material containing humic acid is about 100 nm to 3 mm, about 0.01 to 1 mm, about 100 to 900 µm, about 200 to 800 µm, or about 400 to 600 µm. According to an embodiment of the present invention, 1% to 15% by mass, 5% to 14% by mass, or 7% to 12% by mass of the material containing humic acid passes through a sieve having a mesh size of 42 mm and remains on a sieve having a mesh size of 0.6 mm; 15% to 45% by mass, 30% to 43% by mass, or 40% to 42% by mass of the material passes through a sieve having a mesh size of 0.6 mm and remains on a sieve having a mesh size of 0.3 mm; and 20% to 60% by mass, 35% to 55% by mass, or 46% to 52% by mass of the material passes through a sieve having a mesh size of 0.03 mm.

In an embodiment of the present invention, the method for preparing a material containing humic acid is preferably a method of purchasing a commercially available product.

According to an embodiment of the present invention, the content ratio (% by mass) of the material containing humic acid is 0.01% by mass or more, 0.05% by mass or more, 0.1% by mass or more, 0.5% by mass or more, 1% by mass or more, 1.5% by mass or more, 3% by mass or more, 4% by mass or more, 5% by mass or more, 6% by mass or more, 6.5% by mass or more, 7% by mass or more, 7.5% by mass or more, 8% by mass or more, 8.5% by mass or more, or 9% by mass or more, in the treatment agent. According to an embodiment of the present invention, the content ratio (% by mass) of the material containing humic acid is 30% by mass or less, 25% by mass or less, 20% by mass or less, 15% by mass or less, 13% by mass or less, 12% by mass or less, 10% by mass or less, 8% by mass or less, or 7% by mass or less, in the treatment agent.

According to an embodiment of the present invention, the content ratio (% by mass) of the material containing humic acid is 0.01% to 30% by mass, 0.05% to 25% by mass, 0.1% to 20% by mass, 3% to 15% by mass, 4% to 13% by mass, or 5% to 12% by mass, in the treatment agent. According to an embodiment of the present invention, the content ratio (% by mass) of the material containing humic acid is 1% to 20% by mass.

According to an embodiment of the present invention, the treatment agent contains lignin, bentonite, and humic acid. By employing such a combination, foul odors of ammonia and the like of the treatment target material can be suppressed more efficiently.

### (Disodium hydrogen phosphate)

According to an embodiment of the present invention, the treatment agent contains disodium hydrogen phosphate. According to an embodiment of the present invention, the average particle size of disodium hydrogen phosphate is about 100 nm to 3 mm, about 0.01 to 1 mm, about 100 to 900 µm, about 200 to 800 µm, or about 400 to 600 µm; however, the average particle size is not limited to these and may be outside the ranges described on the left-side, and materials having the sizes of commercially available products can be used as appropriate.

According to an embodiment of the present invention, the content ratio (% by mass) of disodium hydrogen phosphate is 0.01% by mass or more, 0.05% by mass or more, 0.1% by mass or more, 0.5% by mass or more, 1% by mass or more, 1.5% by mass or more, 3% by mass or more, 4% by mass or more, or 5% by mass or more, in the treatment agent. According to an embodiment of the present invention, the content ratio (% by mass) of disodium hydrogen phosphate is 30% by mass or less, 25% by mass or less, 20% by mass or less, 15% by mass or less, 13% by mass or less, 12% by mass or less, 10% by mass or less, 8% by mass or less, or 7% by mass or less, in the treatment agent.

According to an embodiment of the present invention, the content ratio (% by mass) of disodium hydrogen phosphate is 0.01% to 30% by mass, 0.05% to 25% by mass, 0.1% to 20% by mass, 3% to 15% by mass, 4% to 13% by mass, or 5% to 12% by mass, in the treatment agent. According to an embodiment of the present invention, the content ratio (% by mass) of disodium hydrogen phosphate is 1% to 10% by mass.

### (Sodium dihydrogen phosphate)

According to an embodiment of the present invention, the concentration of sodium dihydrogen phosphate is less than 1% by mass. According to an embodiment of the present invention, the treatment agent substantially does not contain sodium dihydrogen phosphate.

### (Sodium bicarbonate)

According to an embodiment of the present invention, the treatment agent contains sodium bicarbonate (sodium hydrogen carbonate). Sodium bicarbonate is a hydrogen carbonate of sodium represented by the composition formula: NaHCO₃. Sodium bicarbonate is so safe to the human body that it may be used as a food additive. Therefore, even from the viewpoint of being less likely to cause chemical injury, sodium bicarbonate is suitable. Regarding the method for preparing sodium bicarbonate, a commercially available product may be purchased, and for example, industrial grade sodium bicarbonates manufactured by Tosoh Corporation, QINDAO HAI WAN GROUP IMP. & EXP. CO., and Zichuan Antou Alum Factory, and the like are preferred. According to an embodiment of the present invention, the average particle size of sodium bicarbonate is not particularly limited, but is preferably 30 to 150 µm, and more preferably 80 to 100 µm.

According to an embodiment of the present invention, the content ratio (% by mass) of sodium bicarbonate is 0.01% by mass or more, 0.05% by mass or more, 0.1% by mass or more, 0.5% by mass or more, 1% by mass or more, 1.5% by mass or more, 2% by mass or more, 3% by mass or more, 3.5% by mass or more, 4% by mass or more, or 4.5% by mass or more, in the treatment agent. According to an embodiment of the present invention, the content ratio (% by mass) of sodium bicarbonate is 40% by mass or less, 38% by mass or less, 35% by mass or less, 33% by mass or less, 30% by mass or less, 29% by mass or less, 28% by mass or less, 25% by mass or less, 20% by mass or less, 18% by mass or less, 15% by mass or less, 13% by mass or less, 12% by mass or less, 11% by mass or less, or 10% by mass or less, in the treatment agent.

According to an embodiment of the present invention, the content ratio (% by mass) of sodium bicarbonate is 0.01% to 40% by mass, 0.05% to 38% by mass, 0.1% to 35% by mass, 0.5% to 30% by mass, 1% to 28% by mass, 1.5% to 25% by mass, 2% to 20% by mass, 3% to 18% by mass, 3.5% to 15% by mass, 4% to 12% by mass, or 4.5% to 10% by mass, in the treatment agent. According to an embodiment of the present invention, the content ratio (% by mass) of sodium bicarbonate is 1% to 20% by mass in the treatment agent.

### (Limonite)

According to an embodiment of the present invention, the treatment agent contains limonite.

The chemical composition of limonite is FeO(OH)·nH₂O; however, limonite may contain hematite (Fe₂O₃), clay minerals, manganese (II) oxide, and the like as impurities. As limonite is included, decomposition and adsorption of sulfur-based compounds such as mercaptan, methyl mercaptan, and hydrogen sulfide, which are foul odor components in feces, as well as fatty acid-based compounds can be carried out.

According to an embodiment of the present invention, the content ratio (% by mass) of limonite is 0.01% by mass or more, 0.05% by mass or more, 0.1% by mass or more, 0.5% by mass or more, 1% by mass or more, 1.5% by mass or more, 2% by mass or more, 3% by mass or more, 3.5% by mass or more, 4% by mass or more, or 4.5% by mass or more, in the treatment agent. According to an embodiment of the present invention, the content ratio (% by mass) of limonite is 40% by mass or less, 38% by mass or less, 35% by mass or less, 30% by mass or less, 28% by mass or less, 25% by mass or less, 20% by mass or less, 18% by mass or less, 15% by mass or less, 13% by mass or less, 12% by mass or less, 10% by mass or less, 5% by mass or less, 2% by mass or less, or less than 1% by mass, in the treatment agent.

According to an embodiment of the present invention, the content ratio (% by mass) of limonite is 0.01% to 40% by mass, 0.05% to 38% by mass, 0.1% to 35% by mass, 0.5% to 30% by mass, 1% to 28% by mass, 1.5% to 25% by mass, 2% to 20% by mass, 3% to 18% by mass, 3.5% to 15% by mass, 4% to 12% by mass, or 4.5% to 10% by mass, in the treatment agent. According to an embodiment of the present invention, the concentration of limonite in the treatment agent is less than 1% by mass. According to an embodiment of the present invention, the treatment agent substantially does not contain limonite.

According to an embodiment of the present invention, the volume average particle size (D50) in the particle size distribution measurement of limonite is preferably 0.1 to 200 µm, more preferably 1 to 150 µm, even more preferably 5 to 100 µm, and still more preferably 10 to 50 µm. As the volume average particle size is in these ranges, the expected effects of the present invention can be efficiently provided.

Limonite can be freely selected from commercially available products. Examples thereof include LMB50 and LMB300 (all manufactured by Japan Limonite Co., Ltd.).

According to an embodiment of the present invention, the treatment is in an air-dried state (a state in which the treatment agent in the air loses moisture through natural drying and maintains an equilibrium with the humidity in the air).

### <Lump-like treatment agent>

According to an embodiment of the present invention, the treatment agent may be granulated using binders such as water, PVA, cellulose, water-soluble cellulose, and sodium alginate. That is, according to an embodiment of the present invention, the treatment agent is a lump-like treatment agent. Furthermore, the treatment agent according to an embodiment of the present invention may be in the form of a granular treatment agent or a lump-like treatment agent using the technologies of JP 2013-6137 A and JP 2011/162244 A. According to an embodiment of the present invention, the treatment agent contains a lubricating agent. The lubricating agent is used when producing a lump-like treatment agent, particularly in order to facilitate smooth feeding into the mortar of a tableting machine. Therefore, regarding the type of the lubricating agent, conventionally known lubricating agents may be used by appropriately selecting or combining them. For example, ester-based agents, silicon-based agents, stearic acid esters, calcium stearate, potassium stearate, calcium, and zinc are used. The content of the lubricating agent is about 0.3% to 5% by mass, about 0.8% to 4.5% by mass, or 0.8% to 3% by mass, with respect to the total amount of the treatment agent.

According to an embodiment of the present invention, the average diameter of the treatment agent is 0.01 to 100 mm, 0.1 to 50 mm, 1 to 30 mm, 3 to 20 mm, or 5 to 20 mm. In the present specification, unless particularly stated otherwise, the term "average diameter" means an average value obtained by arbitrarily selecting a statistically reliable number of particles (or 10, 100, 200, 300, or 1000 particles), measuring the longest particle size for each particle using a vernier caliper, and taking the arithmetic mean of those values.

According to an embodiment of the present invention, the treatment agent is produced using a desktop manual tableting machine. In order to produce a lump-like treatment agent with a desktop manual tableting machine (one pestle and one mortar), first, each component to be solidified is placed in the pestle by hand using a spoon. Thereafter, a lever is manually (hydraulic) lowered to apply pressure, each component is solidified, and a lump-like treatment agent is produced. Incidentally, for example, when a pestle having a small opening diameter (diameter 7 mm; upper and lower circular plate type) is used, since higher pressure per area is applied as the opening diameter is smaller, even materials that are difficult to solidify tend to be solidified. On the other hand, for example, when a pestle having a large opening diameter (diameter 15 mm; upper and lower flat plate type) is used, since lower pressure per area is applied as the opening diameter is larger, it is disadvantageous for materials that are difficult to solidify; however, as the opening diameter is larger, feeding into the pestle is facilitated, and productivity is improved.

According to an embodiment of the present invention, the treatment agent is produced using a continuous type tableting machine. The continuous type tableting machine may or may not be a direct compression type; however, from the viewpoint that the effort for pretreatment can be saved, it is also preferable to adopt a direct compression type, which improves productivity. Furthermore, in order to facilitate feeding of fine powders, it is preferable to incorporate a lubricating agent. On the other hand, when pretreatment is carried out, for example, a lump-like treatment agent may be produced by applying high pressure using a roller compactor and performing rolling to granulate a fine powder, mixing a binder (binding agent) with the granulation product, and tableting the mixture. In view of mass production, it is preferable to use, for example, a tableting machine (Model AP18-SS or the like) manufactured by Hata Tekkosho Co., Ltd. The diameter of the pestle and mortar at this time is about 13 mm, and the number of punching is about 18. When such a tableting machine is used, it is possible to produce the treatment agent simply by preparing raw materials in a desired ratio, mixing the raw materials, placing the mixed raw material in the hopper of the tableting machine, and tableting the mixture in a rotary manner with the tableting machine, which is preferable.

According to an embodiment of the present invention, the treatment agent is produced using a continuous rotary type tableting machine. As described above, there are no particular limitations on the method for producing the treatment agent (lump-like treatment agent). The treatment agent may be prepared by mixing the components constituting the treatment agent in any order, or may be produced by mixing desired components including a binder while tableting the components using a tableting machine.

### (Binder)

According to an embodiment of the present invention, the binder is preferably a cellulose-based binder. According to an embodiment of the present invention, the binder has an action of binding some or all of the components included in the treatment agent.

According to an embodiment of the present invention, the binder may be prepared by purchasing a commercially available product. As the commercially available product, PH-102, TG-101, ST-02, TG-101 manufactured by Asahi Kasei Chemicals Corporation; PVPK-15, PVPK-30, PVPK-90 (polyvinylpyrrolidone) manufactured by Higuchi, Inc.; KC series (KC FLOCK W-50S, W-50, W-100/100G, W-200/200G, W-250, W-300G, W-400G) manufactured by Nippon Paper Chemicals Co., Ltd.; CEOLUS series (CEOLUS K2) manufactured by Asahi Kasei Chemicals Co., Ltd.; and the like are suitably used.

The main function of the binder is to contribute to solidification. Therefore, it is more preferable as the amount of the binder is smaller; however, according to an embodiment of the present invention, the content ratio (% by mass) of the binder is 5% by mass or more, 10% by mass or more, 15% by mass or more, 20% by mass or more, or 25% by mass or more, in the treatment agent. According to an embodiment of the present invention, the content ratio (% by mass) of the binder is 60% by mass or less, 55% by mass or less, 50% by mass or less, or 45% by mass or less, in the treatment agent. According to an embodiment of the present invention, the content ratio (% by mass) of the binder is 10% to 50% by mass in the treatment agent.

According to an embodiment of the present invention, the treatment agent is not in a lump-like form. According to an embodiment of the present invention, the treatment agent may be in the form of a mixture in which the components constituting the treatment agent are simply mixed without applying pressure or the like. According to an embodiment of the present invention, the treatment agent substantially does not contain a binder having a function of binding together some or all of the components constituting the treatment agent. In the present specification, the expression "substantially does not contain" means to include a concept that the treatment agent does not at all contain the component, as well as a concept that the treatment agent contains 0.005% by mass or less of the component.

According to an embodiment of the present invention, the lump-like treatment agent is in an air-dried state (a state in which the treatment agent in the air loses moisture through natural drying and maintains an equilibrium with the humidity in the air).

### <Treatment with treatment agent>

There are no particular limitations on the specific method of treatment; however, according to an embodiment of the present invention, a method of bringing waste liquid and the treatment agent into contact with each other is suitable. According to an embodiment of the present invention, a method of adding the treatment agent to waste liquid may be mentioned. According to an embodiment of the present invention, a method of adding waste liquid to the treatment agent may be mentioned. According to an embodiment of the present invention, the treatment agent is placed in advance in a bottle that collects the waste liquid discharged from a person during a surgical procedure (for example, endoscopic procedure). The waste liquid discharged by suction or the like is introduced into a bottle in which the treatment agent of the present invention has been placed in advance, and the waste liquid is treated.

For example, in the endoscopic examination, waste liquid (excrement, blood, body fluids, and the like) is discharged from the digestive organs (stomach, rectum, large intestine, small intestine, and the like) after the examination, and this waste liquid is subjected to waste disposal. Furthermore, in hospitals, waste fluids such as urine from hospitalized patients are also subjected to waste disposal. These waste liquids may contain spore-forming bacteria such as bacteria of the genus Bacillus, the genus Clostridium, and the like. Spore-forming bacteria are representative of opportunistic infection causative bacteria. Spore-forming bacteria are heat-resistant bacteria that can withstand heat of 100°C or higher, and form spores within the bacterial body when nutrients are insufficient, or the environment is deteriorated. These spores are resistant not only to heat but also to desiccation and disinfectants, and can therefore survive even in a harsh environment until the spores find an environment suitable for growth. Then, when an appropriate environment for growth such as moisture, temperature, and nutrients is available, the spores germinate, become vegetative cells, and begin to proliferate. Therefore, spore-forming bacteria require special attention in the control of hospital infection. The treatment agent according to the present invention can suppress and inhibit spore germination or proliferation of spore-forming bacteria when brought into contact with such waste liquid. For this reason, it is particularly useful in hospitals where large amounts of patient waste liquid needs to be treated. Here, the reason why the treatment agent according to the present invention exhibits a suppressive and inhibitory effect on spore germination or proliferation of spore-forming bacteria is unclear; however, the reason is presumed to be as follows. Incidentally, the present invention is not limited by the following presumption. The treatment agent according to the present invention contains slaked lime and pepper. Among these, the presence of slaked lime causes waste liquid to become alkaline. For this reason, the spore coat (spore coat outer layer and spore coat inner layer) of spore-forming bacteria placed in an alkaline atmosphere becomes soft, and intrusion of materials from the outside world is made easier. For this reason, it is easier for pepper and zinc oxide (in the case of being further contained) in the treatment agent to act directly on the core, and spore germination and bacterial proliferation (growth) are effectively suppressed and inhibited.

That is, the treatment agent according to the present invention is used to suppress and inhibit spore germination or proliferation of spore-forming bacteria. Furthermore, the present invention also provides a method for suppressing and inhibiting spore germination or proliferation of spore-forming bacteria, the method including bringing waste liquid into contact with the treatment agent according to the present invention. Here, examples of the spore-forming bacteria include known spore-forming bacteria of the genus Amphibacillus, the genus Bacillus, the genus Clostridium, the genus Sporosarcina, and the like. Among these, the treatment agent according to the present invention exhibits an excellent ability for suppressing and inhibiting spore germination or proliferation against the genus Clostridium, particularly Clostridioides difficile.

According to an embodiment of the present invention, the amount of the treatment agent with respect to the treatment target material (for example, waste liquid discharged in an endoscopic examination: about 1000 mL to about 4000 mL) can be appropriately adjusted as necessary; however, as a guideline, the amount is 1 to 1000 g, 2 to 900 g, 3 to 800 g, 5 to 750 g, 10 to 700 g, 20 to 650 g, or 30 to 600 g. According to an embodiment of the present invention, the treatment target material and the treatment agent may be brought into contact with each other (thereafter, mixed as necessary) and then left to stand or left unattended for, for example, 5 minutes to 30 days, 120 minutes to 20 days, 300 minutes to 10 days, 200 minutes to 5 days, 400 minutes to 3 days, or 500 minutes to 2 days. According to the treatment agent of the present invention, an effect of suppressing foul odors from the treatment target material for a long period of time is also provided.

In the present embodiment, the treatment agent may further contain other components. Here, examples of the other components include an antibacterial agent. Here, the antibacterial agent in a case where the treatment agent further contains an antibacterial agent is not particularly limited; however, examples thereof include inorganic antibacterial agents such as silver and copper; naturally occurring antibacterial agents such as chitin, chitosan, allyl isothiocyanate, hinokitiol, and limonene; and penicillin-based and macrolide-based antibacterial agents. The amount of the antibacterial agent in a case where the treatment agent further contains an antibacterial agent is not particularly limited, and can be appropriately selected in accordance with the desired effect of suppressing and inhibiting spore germination or proliferation.

### EXAMPLES

### <Preparation of artificial waste liquid (Control (i))>

4 g of soybean protein hydrolysate (high polypeptone), 0.8 g of a garlic paste, and 0.2 g of urea were dissolved in 1 L of pure water. A mixture obtained by adding 1.0 g-wet of black soil to 25 mL of this solution was used as Control (i).

### <Production of Controls (ii) and (iii)>

A product obtained by adding one tablet (0.72 g) of reference treatment agent 1 to the Control (i) was used as Control (ii), and a product obtained by adding 0.72 g of reference treatment agent 2 to the Control (i) was used as Control (iii).

### (Production of reference treatment agent 1)

With regard to the reference treatment agent 1 (average diameter: 15 mm, average thickness: 5 mm), 70 tablets of the treatment agent were obtained by placing each of the following components in a mortar of a desktop tableting machine (manufactured by Kikusui Seisakusho, Ltd.), and tableting the mixture by applying a pressure of 40 kN. Incidentally, the pestle used was an upper and lower flat plate type pestle having a diameter of 15 mm.

**[Table 1]**

| Reference treatment agent 1 | | |
|---|---|---|
| Substance name | % | (g) |
| KC200 (KC FLOCK) | 38.0% | 19 |
| Sodium bicarbonate | 8.0% | 4 |
| Disodium hydrogen phosphate | 3.0% | 1.5 |
| Slaked lime | 12.0% | 6 |
| Zeolite | 2.0% | 1 |
| Zinc oxide | 0.5% | 0.25 |
| Water-absorbing polymer | 35.5% | 17.75 |
| Calcium stearate | 1.0% | 0.5 |
| | 100.0% | 50 |

| | | |
|---|---|---|
| KC200 (KC FLOCK): manufactured by Nippon Paper Group average particle size 32 µm Sodium bicarbonate: manufactured by Tosoh Corporation average particle size 90 µm Disodium hydrogen phosphate: manufactured by Mitejima Chemical Co., Ltd. average particle size 90 µm Slaked lime: manufactured by Ube Material Industries, Ltd. 100-mesh pass Zeolite: ZEOFILL W1 volume average particle size (D50): 13 µm Zinc oxide: zinc oxide type 1 manufactured by Hakusui Tech Co., Ltd. average particle size 0.6 µm Water-absorbing polymer (SANFRESH ST-573): average particle size: 380 µm (more than 850 µm about 1% by mass, more than 106 µm 850 µm Calcium stearate: SUNACE SAK-CS-P average particle size 7 µm (including a portion of particles having a size of 50 µm or more). | | |

### (Production of reference treatment agent 2)

Each of the components constituting the treatment agent were mixed so as to obtain the composition shown in the following table, and 100 g of the treatment agent was produced.

**[Table 2]**

| Reference treatment agent 2 | | | | |
|---|---|---|---|---|
| Limonite | Lignin | Bentonite | Slaked lime | Water-absorbing polymer |
| LMB-50 | SAN-X P-202 | MOISNITE S | Ube Material Industries | Polyacrylic acid-based polymer |
| 13.70% | 27.40% | 8.22% | 4.11% | 46.57% |

| | | | | |
|---|---|---|---|---|
| Limonite: LMB-50 volume average particle size (D50): 22 µm Lignin: SAN-X P-202 average particle size: 60 µm Bentonite: MOISNITE S average particle size: 139 µm Slaked lime: manufactured by Ube Material Industries: 100-mesh pass Water-absorbing polymer polyacrylic acid-based polymer SANFRESH ST-250 average particle size: 380 µm (more than 850 µm about 1% by mass, more than 106 µm and 850 µm or less 89% by mass, 106 µm or less about 10% by mass). | | | | |

### <Addition of various chemicals>

Various chemicals and the like shown in the following table in the amounts and concentrations shown in the following table were further added to the Control (ii) and Control (iii).

**[Table 3]**

| Various chemicals added to Control (ii) | |
|---|---|
| CaO₂ (powder) | 0.2 (g) |
| Iron oxide | 0.2 (g) |
| Activated carbon | 0.1 (g) |
| Copper salt (copper sulfate) | Amount to reach 1mg-Cu/L (15.7µM) is added in solution |
| Silver salt (silver nitrate) | Amount to reach 1mg-Ag/L (9.3µM) is added in solution |
| Alum (AINH₄ (SO4)₂) | Amount to reach 2mg/L (8.4µM) is added in solution |
| Dichloroisocyanuric acid | 0.025 (g) |
| Benzalkonium chloride (invert soap) | Amount to reach 100mg/L is added in solution |

| | |
|---|---|
| *Various chemicals have been added to 25 mL of artificial waste liquid. Therefore, the actual amounts of addition of the various chemicals for which only the concentrations are disclosed can be converted from the above-described concentrations. | |

First, the results of measuring the odor intensity of an experiment based on the Control (ii) are shown in Table 5.

Incidentally, the odor intensity was determined by allowing a plurality of persons (three persons) who had passed an odor panel selection test, to score samples according to the following criteria, and calculating an arithmetic mean of the scores.

**[Table 4]**

| Foul odor intensity | Content |
|---|---|
| 0 | Odorless |
| 1 | Smell that can be barely detected |
| 2 | Faint smell that allows to realize what smell it is |
| 3 | Smell that can be easily detected |
| 4 | Strong smell |
| 5 | Very strong smell |

**[Table 5-1]**

| | Number of days | Measurer U | Measurer K | Measurer D | Odor intensity average |
|---|---|---|---|---|---|
| Control (i) | 1^{st} day | 3 | 3.5 | 3 | 3.17 |
| | 4^{th} day | 4 | 3 | 4 | 3.67 |
| | 5^{th} day | 3 | 3 | 3.5 | 3.17 |
| | 6^{th} day | | | 4 | 4.00 |
| | 8^{th} day | 3.5 | 4 | 4 | 3.83 |
| | 11^{th} day | 4 | 4 | 3 | 3.67 |
| Control (ii) | 1^{st} day | 1.5 | 2 | 2.5 | 2.00 |
| | 4^{th} day | 2.5 | 3 | 3 | 2.83 |
| | 5^{th} day | 3 | 3 | 3.5 | 3.17 |
| | 6^{th} day | | | 3.5 | 3.50 |
| | 8^{th} day | 3.5 | 4 | 4 | 3.83 |
| | 11^{th} day | 3.5 | 4 | | 3.75 |
| Control (ii)+CaO₂ | 1^{st} day | 2 | 3 | 3 | 2.67 |
| | 4^{th} day | 3 | 4 | 3 | 3.33 |
| | 5^{th} day | 3.5 | 3.5 | 3.5 | 3.50 |
| | 6^{th} day | | | 3 | 3.00 |
| | 8^{th} day | 3.5 | 3 | 3.5 | 3.33 |
| | 11^{th} day | 3.5 | 3.5 | 3.5 | 3.50 |
| Control (ii) +iron oxide | 1^{st} day | 1.5 | 2.5 | 2 | 2.00 |
| | 4^{th} day | 2.5 | 3 | 2 | 2.50 |
| | 5^{th} day | 3.5 | 2.5 | 3 | 3.00 |
| | 6^{th} day | | | 2.5 | 2.50 |
| | 8^{th} day | 3 | 3 | 3 | 3.00 |
| | 11^{th} day | 3.5 | 3 | | 3.25 |
| Control (ii) +activated carbon | 1^{st} day | 1.5 | 2 | 1 | 1.50 |
| | 4^{th} day | 2 | 2 | 2.5 | 2.17 |
| | 5^{th} day | 3 | 3 | 3 | 3.00 |
| | 6^{th} day | | | 3 | 3.00 |
| | 8^{th} day | 3 | | | 3.00 |
| | 11^{th} day | | | | |
| Control (ii)+copper salt | 1^{st} day | 1.5 | 3.5 | 2 | 2.33 |
| | 4^{th} day | 2.5 | 3.5 | 2 | 2.67 |
| | 5^{th} day | 2.5 | 3 | 3 | 2.83 |
| | 6^{th} day | | | 3 | 3.00 |
| | 8^{th} day | 3 | | | 3.00 |
| | 11^{th} day | | | | |
| Control (ii)+silver salt | 1^{st} day | 1.5 | 2 | 1.5 | 1.67 |
| | 4^{th} day | 2.5 | 3 | 2.5 | 2.67 |
| | 5^{th} day | 2.5 | 2 | 2.5 | 2.33 |
| | 6^{th} day | | | 2.5 | 2.50 |
| | 8^{th} day | 3.5 | 3.5 | 3.5 | 3.50 |
| | 11^{th} day | 3 | 3.5 | 3.5 | 3.33 |
| Control (ii)+alum | 1^{st} day | 2.5 | 2 | 2 | 2.17 |
| | 4^{th} day | 3 | 3 | 2.5 | 2.83 |
| | 5^{th} day | 2.5 | 2 | 3 | 2.50 |
| | 6^{th} day | | | 3 | 3.00 |
| | 8^{th} day | 2.5 | 3 | 3 | 2.83 |
| | 11^{th} day | 3 | 3.5 | 3.5 | 3.33 |

**[Table 5-2]**

| | | | | | |
|---|---|---|---|---|---|
| Control (ii)+dichloroisocyanuric acid (chlorine agent) | 1^{st} day | 4 | 2 | 4 | 3.33 |
| | 4^{th} day | 2.5 | 2 | 2.5 | 2.33 |
| | 5^{th} day | 4 | 2 | 3.5 | 3.17 |
| | 6^{th} day | | | 3 | 3.00 |
| | 8^{th} day | 3 | 3 | 3 | 3.00 |
| | 11^{th} day | 2.5 | 2 | | 2.25 |
| Control (ii)+benzalkonium chloride (invert soap) | 1^{st} day | 1.5 | 2.5 | 1.5 | 1.83 |
| | 4^{th} day | 1.5 | 2 | 1.5 | 1. 67 |
| | 5^{th} day | 2 | 2 | 1.5 | 1.83 |
| | 6^{th} day | | | 1.5 | 1.50 |
| | 8^{th} day | 3 | 2.5 | 3 | 2.83 |
| | 11^{th} day | 3 | 3 | 3 | |

When the change in the odor intensity in Table 5 is viewed, the odor intensity of the Control (i) remained at around 3.5 from the first day to the eleventh day. The Control (ii) showed a significant effect with an odor intensity of 2 on the first day; however, the intensity gradually increased, reaching a level equal to the Control (i) on the fifth day and the highest level among all the systems on the eighth day.

In the systems in which various chemicals were added to the Control (ii), CaO and iron oxide, which were added for the purpose of improving the reducing atmosphere in which foul odor substances are easily generated, had a low effect, being worse than or equal to the effect of the Control (ii). In the system to which activated carbon was added, the odor intensity remained lower by about 0.5 than the odor intensity of the Control (ii) until the eighth day, and it was suggested that the system was effective to a certain extent; however, on the eighth day, mold began to grow and emitted an unpleasant odor. Therefore, testing of the system to which activated carbon was added was stopped. In addition, there were cases where the quality of the smell had changed due to the chemicals themselves or the growth of mold, causing an increase in the unpleasant feeling or the like. In the systems to which a copper salt, a silver salt, alum, a chlorine agent, or a disinfectant was added for the purpose of sterilization, the fecal odor and putrid odor were all suppressed more than in the Control (ii); however, in some cases, the odor intensity was increased because the odor of chemicals such as a chlorine agent was strong, or satisfactory results were not obtained.

With regard to the quality of the smell, the fecal odor and the odor of a pit toilet began to be felt from around the fourth day in the Control (i), whereas although the odor intensity of the Control (ii) was lower, the odor of a pit toilet began to be felt from around the same time.

In the systems to which Cao or a silver salt was added, there was no significant difference from the Control (ii) in terms of the change in the quality of smell, while in the systems to which iron oxide or alum was added, the odor of a pit toilet was suppressed for about several days; however, a similar smell was emitted thereafter. Furthermore, in the systems to which activated carbon or a copper salt was added, the odor of a pit toilet was slightly suppressed; however, since a different odor was emitted, or mold was caused to grow, the systems were not considered to be quite satisfactory. In the system to which dichloroisocyanuric acid was added, the odor of a pit toilet was not much felt; however, a relatively strong chlorine smell or burnt odor was felt.

On the other hand, with regard to the state during use, in the Control (ii), water started to come out from the top on the fourth day, and on the sixth day, the system was separated into three layers, with water being observed in the middle layer. In contrast, in the system to which CaO was added, the system was divided into three layers on the fourth day, and a large amount of water was also present. This is considered to be because a macromolecular water-absorbing resin (water-absorbing polymer) is coordinated to calcium, and the amount of water absorption was decreased. A similar phenomenon was also observed in the system to which a copper salt was added. In the other systems, a significant difference from the Controls was not observed in terms of water retention properties.

Next, the results of measuring the odor intensity of the reference treatment agent 2 are shown in Table 7.

**[Table 6]**

| Various chemicals added to Control (iii) | |
|---|---|
| Zeolite (powder) | 0.2 (g) |
| CaO₂ (powder) | 0.2 (g) |
| Iron oxide | 0.2 (g) |
| Activated carbon | 0.1 (g) |
| Copper salt | Amount to reach 1mg-Cu/L(15.7µM) is added in solution |
| Silver salt | Amount to reach 1mg-Ag/L(9.3µM) is added in solution |
| Alum | Amount to reach 2mg/L(8.4µM) is added in solution |
| Dichloroisocyanuric acid | 0.025 (g) |
| Benzalkonium chloride (invert soap) | Amount to reach 100mg/L is added in solution |

| | |
|---|---|
| *Various chemicals have been added to 25 mL of artificial waste liquid. Therefore, the actual amounts of addition of the various chemicals for which only the concentrations are disclosed can be converted from the above-described concentrations. | |

**[Table 7-1]**

| | Number of days | Measurer U | Measurer K | Measurer D | Odor intensity average |
|---|---|---|---|---|---|
| Control (iii) | 1^{st} day | 2 | 2.5 | 3 | 2.5 |
| | 4^{th} day | 4 | 4 | 4 | 4.0 |
| | 5^{th} day | 3.5 | 3 | 3.5 | 3.3 |
| | 6^{th} day | - | - | 3 | 3.0 |
| | 8^{th} day | 3 | 3 | 3.5 | 3.2 |
| | 11^{th} day | 3.5 | 2.5 | 3.5 | 3.2 |
| Control (iii)+zeolite | 1^{st} day | 1.5 | 3 | 3 | 2.5 |
| | 4^{th} day | 4 | 3.5 | 4 | 3.8 |
| | 5^{th} day | 3.5 | 3.5 | 3.5 | 3.5 |
| | 6^{th} day | - | - | 2.5 | 2.5 |
| | 8^{th} day | 3 | 3 | 3 | 3.0 |
| | 11^{th} day | 3.5 | 3 | 3 | 3.2 |
| Control (iii) +CaO₂ | 1^{st} day | 2 | 3 | 3 | 2.7 |
| | 4^{th} day | 4 | 4.5 | 4 | 4.2 |
| | 5^{th} day | 3.5 | 3.5 | 3.5 | 3.5 |
| | 6^{th} day | - | - | 3 | 3.0 |
| | 8^{th} day | 3 | 3.5 | 3 | 3.2 |
| | 11^{th} day | 3 | 3 | 3 | 3.0 |
| Control (iii) +iron oxide | 1^{st} day | 2.5 | 3 | 3 | 2.8 |
| | 4^{th} day | 3.5 | 3.5 | 3 | 3.3 |
| | 5^{th} day | 3 | 3 | 3.5 | 3.2 |
| | 6^{th} day | - | - | 2.5 | 2.5 |
| | 8^{th} day | 2 | 3 | 3 | 2.7 |
| | 11^{th} day | 3 | 3 | 3 | 3.0 |
| Control (iii)+activated carbon | 1^{st} day | 1 | 2.5 | 2.5 | 2.0 |
| | 4^{th} day | 3.5 | 3 | 3.5 | 3.3 |
| | 5^{th} day | 3 | 3 | 3.5 | 3.2 |
| | 6^{th} day | - | - | 3 | 3.0 |
| | 8^{th} day | 2 | 3 | 2.5 | 2.5 |
| | 11^{th} day | 2.5 | 2.5 | 2.5 | 2.5 |
| Control (iii) +copper salt | 1^{st} day | 2 | 3.5 | 3 | 2.8 |
| | 4^{th} day | 3.5 | 3.5 | 3.5 | 3.5 |
| | 5^{th} day | 3 | 3 | 3 | 3.0 |
| | 6^{th} day | - | - | 3 | 3.0 |
| | 8^{th} day | 2.5 | 2 | 3 | 2.5 |
| | 11^{th} day | 2.5 | 2.5 | 2.5 | 2.5 |
| Control (iii) +silver salt | 1^{st} day | 2 | 3 | 3 | 2.7 |
| | 4^{th} day | 3 | 4 | 3 | 3.3 |
| | 5^{th} day | 3 | 2.5 | 3 | 2.8 |
| | 6^{th} day | - | - | 2.5 | 2.5 |
| | 8^{th} day | 2.5 | 3 | 2.5 | 2.7 |
| | 11^{th} day | 3 | 3 | 2.5 | 2.8 |
| Control (iii) +alum | 1^{st} day | 2 | 2.5 | 3 | 2.5 |
| | 4^{th} day | 3 | 3.5 | 3.5 | 3.3 |
| | 5^{th} day | 2.5 | 2.5 | 3 | 2.7 |
| | 6^{th} day | - | - | 4 | 4.0 |
| | 8^{th} day | 2 | 3 | 3.5 | 2.8 |
| | 11^{th} day | 3 | 3 | 2.5 | 2.8 |

**[Table 7-2]**

| | | | | | |
|---|---|---|---|---|---|
| Control (iii)+dichloroisocyanuric acid (chlorine agent) | 1^{st} day | 4 | 4 | 5 | 4.3 |
| | 4^{th} day | 3 | 2 | 4 | 3.0 |
| | 5^{th} day | 2 | 2 | 4 | 2.7 |
| | 6^{th} day | - | - | 4 | 4.0 |
| | 8^{th} day | 3 | 3 | 4 | 3.3 |
| | 11^{th} day | 3 | 3 | 3.5 | 3.2 |
| Control (iii)+benzalkonium (invert soap) | 1^{st} day | 2.5 | 3 | 3 | 2.8 |
| | 4^{th} day | 1.5 | 3 | 2.5 | 2.3 |
| | 5^{th} day | 2.5 | 2.5 | 3 | 2.7 |
| | 6^{th} day | - | - | 3 | 3.0 |
| | 8^{th} day | 3.5 | 3 | 3.5 | 3.3 |
| | 11^{th} day | 3 | 3.5 | 3 | 3.2 |

The reference treatment agent 2 (Control (iii)) suppressed smell second only to activated carbon on the first day alone; however, the odor intensity fairly increased on the fourth day and the fifth day. Perhaps due to strong emission of the smell, the odor intensity slightly decreased from the eighth day onwards; however, the reference treatment agent 2 (Control (iii)) was considered to have a low long-term effect of suppressing the generation of foul odor.

The systems to which various chemicals and the like were added to the Control (iii), generally showed low odor intensities except for the systems to which CaO or zeolite was added. Among these, the silver salt-added system, the activated carbon-added system, or the cationic surfactant-added system (disinfectant-added system) showed relatively satisfactory results.

However, in terms of the quality of smell, in the Control (iii), a strong fecal odor, the odor of a pit toilet, and irritation were felt on the fourth day, whereas in the systems to which zeolite, CaO, iron oxide, activated carbon, a copper salt, a silver salt, or alum was added, the odor of a pit toilet was felt from around the same time, although the intensity varied.

In the system to which dichloroisocyanuric acid was added, a chemical odor was felt continuously at a constant intensity. In the system to which benzalkonium chloride was added, the garlic odor remained for a long time, the decomposition of organic matter was suppressed, and the generation of a fecal odor was also suppressed; however, on the eleventh day, the odor of a pit toilet was emitted.

With regard to the state during use, the system to which CaO was added hardly retained water and was in a soggy state. The other systems were not significantly different from the Control (iii).

In the above-described tests, in the system to which CaO was added, since the effect of water absorption was weakened, and there was no effect on smell, further investigation was terminated, while in the system to which iron oxide was added, since there was only a slight effect, further investigation was terminated. The effect of a copper salt was equal to the effect of silver; however, because the copper salt inhibited the water-absorbing resin, further investigation was terminated.

The above-described testing was defined as the first test, and consequently, in the second test, the effect was verified by varying the concentration in the systems to which a silver salt, alum, dichloroisocyanuric acid, or benzalkonium chloride was added. Furthermore, it was decided to test CMC (sodium carboxymethyl cellulose) paste, which was predicted to have an effect of increasing viscosity and suppressing mass transfer; sodium chloride, which was predicted to have an effect of increasing the salt concentration and reducing microbial activity; a soluble oxidizing iron salt; benzethonium chloride, which is a cationic surfactant similar to benzalkonium chloride; and sodium percarbonate, which generates hydrogen peroxide in water, singly as chemicals to be added to the Control (ii) (single systems). In addition, it was decided to verify the effects of the combinations of benzalkonium chloride and activated carbon, sodium chloride and activated carbon, sodium percarbonate and activated carbon, sodium percarbonate and benzalkonium chloride, and sodium percarbonate and a silver salt, as chemicals to be added to the Control (ii) (complex systems).

The conditions for these second odor suppression tests are shown in the following table.

**[Table 8]**

| Various chemicals added to Control (ii) | |
|---|---|
| Single system | |
| Silver salt (silver nitrate) | 5 mg/L |
| Alum | 10 mg/L |
| Benzalkonium chloride | 300 mg/L |
| CMC | 10 g/L |
| Sodium chloride | 10 g/L |
| Iron(III) oxide | 20 mg/L |
| Benzethonium chloride | 300 mg/L |
| Sodium percarbonate | 10 g/L |

| Complex system | |
|---|---|
| Benzalkonium chloride and activated carbon | 300 mg/L +0.1 g/25 mL |
| Sodium chloride and activated carbon | 10 g/L +0.1 g/25 mL |
| Sodium percarbonate and activated carbon | 10 g/L +0.1 g/25 mL |
| Sodium percarbonate and benzalkonium chloride | 10 g/L +300 mg/L |
| Sodium percarbonate and silver salt (silver nitrate) | 10 g/L +5 mg/L |

| | |
|---|---|
| *Various chemicals have been added to 25 mL of artificial waste liquid. Therefore, the actual amounts of addition of the various chemicals for which only the concentrations are disclosed can be converted from the above-described concentrations. | |

The odor pleasantness was determined by allowing a plurality of persons (three persons) who had passed an odor panel selection test, to score samples according to the following criteria, and calculating an arithmetic mean of the scores.

**[Table 9]**

| Odor pleasantness | Content |
|---|---|
| 2 | Pleasant |
| 1 | Slightly pleasant |
| 0 | Neither pleasant nor unpleasant (No unpleasant odor) |
| - 1 | Slightly unpleasant |
| - 2 | Unpleasant |
| - 3 | Very unpleasant |
| - 4 | Extremely unpleasant |

| | |
|---|---|
| *Ratings with negative numbers are associated with fecal odor and putrid odor. | |

**[Table 10-1]**

| Added chemicals | Number of days | Average odor intensity | Average odor pleasantness |
|---|---|---|---|
| Control (i) | 1 | 3.5 | - |
| | 2 | 3.8 | - |
| | 3 | 3.8 | - |
| | 4 | 4.0 | - 2.3 |
| | 7 | 3.8 | - 2.7 |
| | 8 | 3.8 | - 2.7 |
| | 9 | 4.0 | - 3.0 |
| | 11 | 3.7 | - 2.0 |
| | 14 | 3.3 | - 2.0 |
| | 21 | 3.8 | - 2.3 |
| Control (ii) | 1 | 2.3 | - |
| | 2 | 2.7 | - |
| | 3 | 3.3 | - |
| | 4 | 3.5 | - 2.3 |
| | 7 | 3.3 | - 2.3 |
| | 8 | 2.8 | - 1.7 |
| | 9 | 3.0 | - 2.0 |
| | 11 | 3.2 | - 1.7 |
| | 14 | 3.5 | - 1.7 |
| | 21 | 3.0 | - 1.5 |
| Control (ii) +silver salt | 1 | 1.8 | - |
| | 2 | 2.2 | - |
| | 3 | 3.0 | - |
| | 4 | 3.2 | - 2.3 |
| | 7 | 3.0 | - 1.7 |
| | 8 | 2.7 | - 1.0 |
| | 9 | 2.5 | - 1.0 |
| | 11 | 3.0 | - 1.3 |
| | 14 | 4.0 | - 3.0 |
| | 21 | - | - |
| Control (ii) +alum | 1 | 2.5 | - |
| | 2 | 2.2 | - |
| | 3 | 3.2 | - |
| | 4 | 3.0 | - 1.5 |
| | 7 | 3.3 | - 2.3 |
| | 8 | 2.8 | - 2.0 |
| | 9 | 3.5 | - 2.0 |
| | 11 | 3.2 | - 2.0 |
| | 14 | - | - |
| | 21 | - | - |
| Control (ii) +benzalkonium chloride | 1 | 2.2 | - |
| | 2 | 3.0 | - |
| | 3 | 2.2 | - |
| | 4 | 2.2 | - 0.7 |
| | 7 | 3.0 | - 0.7 |
| | 8 | 3.2 | - 1.3 |
| | 9 | 2.0 | - 1.0 |
| | 11 | 3.0 | - 1.3 |
| | 14 | 3.0 | - 1.0 |
| | 21 | 3.0 | - 1.3 |

**[Table 10-2]**

| | | | |
|---|---|---|---|
| Control (ii) +CMC | 1 | 1.5 | - |
| | 2 | 1.7 | - |
| | 3 | 2.5 | - |
| | 4 | 2.0 | - 1.3 |
| | 7 | 3.0 | - 1.0 |
| | 8 | 3.3 | - 1.3 |
| | 9 | 3.5 | - 2.0 |
| | 11 | 3.5 | - 2.0 |
| | 14 | 3.3 | - 1.7 |
| | 21 | 3.0 | - 1.7 |
| Control (ii) +sodium chloride | 1 | 2.7 | - |
| | 2 | 2.0 | - |
| | 3 | 2.8 | - |
| | 4 | 3.0 | - 1.3 |
| | 7 | 3.2 | - 1,3 |
| | 8 | 3.5 | - 2.0 |
| | 9 | - | - |
| | 11 | - | - |
| | 14 | - | - |
| | 21 | - | - |
| Control (ii) +iron (III) chloride | 1 | 2.3 | - |
| | 2 | 2.2 | - |
| | 3 | 2.8 | - |
| | 4 | 3.0 | - 2.5 |
| | 7 | 3.0 | - 2.0 |
| | 8 | - | - |
| | 9 | - | - |
| | 11 | - | - |
| | 14 | - | - |
| | 21 | - | - |
| | 1 | 2.5 | - |
| | 2 | 2.7 | - |
| | 3 | 2.0 | - |
| | 4 | 1.8 | - 0.5 |
| Control (ii) +benzethonium chloride | 7 | 2.0 | - 0.3 |
| | 8 | 2.3 | - 0.5 |
| | 9 | 2.0 | - 0.3 |
| | 11 | 2.7 | - 0.3 |
| | 14 | 2.5 | - 0.3 |
| | 21 | 2.3 | - 0.3 |
| Control (ii) +sodium percarbonate | 1 | 2.3 | - |
| | 2 | 2.5 | - |
| | 3 | 2.8 | - |
| | 4 | 2.7 | - 1.8 |
| | 7 | 2.8 | - 1.7 |
| | 8 | 3.0 | - 1.7 |
| | 9 | 3.0 | - 1.0 |
| | 11 | 3.2 | - 1.3 |
| | 14 | 3.3 | - 1.7 |
| | 21 | 3.3 | - 1.3 |

In the Control (ii), the odor of a pit toilet was strong from the third day to the seventh day, and perhaps because the toilet-related odor components had volatilized to some extent during that time, an odor of rotting vegetables and the like were felt thereafter.

In the systems to which a silver salt, alum, sodium chloride, or iron chloride was added, the odor of a pit toilet and the like were felt on around the third day, and on around the seventh day to the eleventh day, the test was stopped because mold began to grow, resulting in a change in the odor.

In the system to which CMC paste was added, because there was an odor reminiscent of plastics or rubber, the odor of a pit toilet was not much felt until about the eighth day; however, after that, the odor of a pit toilet and a putrid odor became stronger.

In the systems to which benzalkonium chloride or benzethonium chloride was added, a chemical odor was felt all the time; however, the odor of a pit toilet was relatively suppressed, and the effect was greater in the system into which benzethonium chloride was incorporated. In the system into which sodium percarbonate was incorporated, a distinctive and unpleasant chemical odor was felt; however, the odor of a pit toilet itself was relatively suppressed until about the second week.

**[Table 11]**

| Added chemicals | Number of days | Average odor intensity | Average odor pleasantness |
|---|---|---|---|
| Control (ii) +benzalkonium chloride +activated carbon | 1 | 1.2 | - |
| | 2 | 1.3 | - |
| | 3 | 2.0 | - |
| | 4 | 2.8 | - 1.7 |
| | 7 | 2.3 | - 1.3 |
| | 8 | 3.0 | - 2.0 |
| | 9 | - | - |
| | 11 | - | - |
| | 14 | - | - |
| | 21 | - | - |
| Control (ii) +sodium chloride +activated carbon | 1 | 1.7 | - |
| | 2 | 1.7 | - |
| | 3 | 2.0 | - |
| | 4 | 2.8 | - 2.0 |
| | 7 | 2.8 | - 1.3 |
| | 8 | 3.0 | - 2.0 |
| | 9 | - | - |
| | 11 | - | - |
| | 14 | - | - |
| | 21 | - | - |
| Control (ii) +sodium percarbonate +activated carbon | 1 | 2.2 | - |
| | 2 | 2.2 | - |
| | 3 | 2.5 | - |
| | 4 | 2.0 | -0.7 |
| | 7 | 3.0 | - 1.3 |
| | 8 | 2.7 | - 1.3 |
| | 9 | 3.0 | - 1.0 |
| | 11 | 2.8 | - 1.0 |
| | 14 | - | - |
| | 21 | - | - |
| Control (ii) +sodium percarbonate +benzalkonium chloride | 1 | 3.2 | - |
| | 2 | 2.8 | - |
| | 3 | 3.7 | - |
| | 4 | 3.0 | - 2.0 |
| | 7 | 3.0 | - 1.3 |
| | 8 | 3.0 | - 1.7 |
| | 9 | 3.0 | - 1.0 |
| | 11 | 3.3 | - 2.0 |
| | 14 | 3.3 | - 2.0 |
| | 21 | 3.0 | - 1.3 |
| Control (ii) +sodium percarbonate+silver salt | 1 | 3.2 | - |
| | 2 | 2.8 | - |
| | 3 | 2.7 | - |
| | 4 | 3.0 | - 2.0 |
| | 7 | 3.0 | - 1.3 |
| | 8 | 3.0 | - 1.7 |
| | 9 | 3.0 | - 3.0 |
| | 11 | 2.8 | - 1.3 |
| | 14 | 3.3 | - 1.3 |
| | 21 | 2.5 | - 1.0 |

In the systems to which chemicals were added in combination, there were many cases where mold grew during the test, and no particular effect of adding chemicals in combination to the present system could be found.

The odor intensity is an index of the strength of an odor, and in some cases, an odor that is strong may not be necessarily felt unpleasant, or even a weak odor may be felt unpleasant. Thus, further investigation was carried out by focusing on the pleasantness or unpleasantness of odors.

Systems were each prepared by incorporating tea (100% green tea leaves), incorporating perilla, incorporating chili pepper, incorporating black pepper, incorporating Houttuynia cordata, incorporating powdered mustard, or incorporating powdered wasabi, as various natural chemicals into the Control (ii), and the pleasantness or unpleasantness of odors was evaluated in the same manner as described above. The amount of these chemicals added to the Control (ii) was all set to 0.1 g.

**[Table 12]**

| Added chemicals | Number of days | Average odor pleasantness |
|---|---|---|
| Control (ii) +tea | After 5 days | - 0.67 |
| | After 7 days | - 0.67 |
| | After 9 days | - 0.33 |
| Control (ii) +perilla | After 5 days | - 1.67 |
| | After 7 days | - 1.00 |
| | After 9 days | - 1.33 |
| Control (ii) +chili pepper | After 5 days | - 1.33 |
| | After 9 days | -0.33 |
| Control (ii) +black pepper | After 5 days | 0 |
| | After 7 days | 0 |
| | After 9 days | 0 |
| Control (ii) +Houttuynia cordata | After 5 days | - 1.67 |
| | After 9 days | - 1.33 |
| Control (ii) +Houttuynia cordata | After 5 days | - 1.67 |
| | After 9 days | - 1.33 |
| Control (ii) +powdered mustard | After 3 days | - 0.33 |
| | After 7 days | - 1.00 |
| Control (ii) +powdered wasabi | After 7 days | -0.33 |

As a result, in terms of the odor pleasantness, the system into which pepper was incorporated was the best, with almost no unpleasantness felt for about 10 days. In view of the odor quality (quality of smell), in the system into which pepper was incorporated, where the unpleasantness was not high, the odor was perceived mainly as a pepper odor. The composition of the system into which pepper was incorporated is summarized below.

**[Table 13]**

| Substance name % | |
|---|---|
| Substance name | % |
| KC200 (KC FLOCK) | 33.3 |
| Sodium bicarbonate | 7.0 |
| Disodium hydrogen phosphate | 2.6 |
| Slaked lime | 10.5 |
| Zeolite | 1.8 |
| Zinc oxide | 0.4 |
| Water-absorbing polymer | 31.2 |
| Calcium stearate | 0.9 |
| Black pepper | 12.3 |
| | 100.0% |

Incidentally, in the above-described table, when the binder (KC200 (KC FLOCK)) is excluded and then the content ratio of each component is converted, the content ratio is as follows.

**[Table 14]**

| Substance name % | |
|---|---|
| Substance name | % |
| Sodium bicarbonate | 10.5 |
| Disodium hydrogen phosphate | 3.9 |
| Slake lime | 15.7 |
| Zeolite | 2.7 |
| Zinc oxide | 0.6 |
| Water-absorbing polymer | 46.8 |
| Calcium stearate | 1.4 |
| Black pepper | 18.4 |
| | 100.0% |

Subsequently, further investigation was conducted to find whether there was a better alternative system than pepper. Specifically, since slaked lime (alkali component) is included in the Control (ii), it was investigated to find whether further adding an alkali would suppress suppress putrefaction to thereby suppress foul odor, and whether potassium iodide would react with organic matter and exhibit a sterilizing action as an iodine agent to suppress foul odor. Specifically, systems were each prepared by incorporating sodium sesquicarbonate, incorporating sodium bicarbonate, or incorporating potassium iodide, as various chemicals into the Control (ii), and the pleasantness or unpleasantness of odors was evaluated in the same manner as described above. The amount of these chemicals added to the Control (ii) was all set to 0.1 g.

**[Table 15]**

| Added chemicals | Number of days | Average unpleasantness |
|---|---|---|
| Control (ii) +Sodium sesquicarbonate | After 5 days | - 1.67 |
| | After 9 days | - 1.67 |
| | After 14 days | - 1.33 |
| Control (ii) +sodium bicarbonate | After 5 days | - 1.67 |
| | After 9 days | - 1.67 |
| | After 14 days | - 1.67 |
| Control (ii) +potassium iodide | After 5 days | - 1.00 |
| | After 9 days | - 1.67 |
| | After 14 days | - 2.00 |

As shown in Table 15, the odor pleasantness of these chemicals was equal to or lower than that of the Control (ii), and their effects were not recognized. There was also no significant difference in the odor quality.

Subsequently, an experiment was conducted, in which a thickening agent having a thickening effect was added aiming for an effect of decreasing the amount of volatilization of foul odor by decreasing the mobility of foul odor components in a liquid due to an increase in viscosity. Specifically, systems were each prepared by incorporating hi METOLOSE, incorporating PVA, and incorporating starch, as various chemicals into the Control (ii), and the pleasantness or unpleasantness of odors was evaluated in the same manner as described above. The amount of the agent added to the control (ii) was set to 0.1 g for hi METOLOSE and starch, and was set to 0.1 ml for PVA.

**[Table 16]**

| Added chemicals | Number of days | Average unpleasantness |
|---|---|---|
| Control (ii) +hi METOLOSE | After 5 days | - 0.67 |
| | After 7 days | - 1.67 |
| | After 9 days | - 1.67 |
| | After 14 days | - 1.00 |
| Control (ii) +PVA | After 5 days | - 1.00 |
| | After 9 days | - 1.67 |
| | After 14 days | - 1.33 |
| Control (ii) +starch | After 5 days | - 1.00 |
| | After 9 days | - 1.67 |
| | After 14 days | - 1.33 |

As such, results as expected could not be obtained.

Subsequently, experiments were performed using various surfactants, aiming for a foul odor suppressing effect of a technique for reducing the number of live bacteria, such as a surfactant. Specifically, systems in which 0.1 g of TRITON X100 (Kishida Chemical Co., Ltd.), which is representative of nonionic surfactants, was incorporated, 0.1 g of a soap (a commercially available fatty acid ester soap shaved into a powder) was incorporated, and two kinds of cationic surfactant samples (CATION G50: benzalkonium chloride solution, OSMORIN DA-50: didecyldimethylammonium adipate) were incorporated as various chemicals into the Control (ii), were used. Furthermore, the two kinds of cationic surfactant samples were used at the following various concentrations. Incidentally, since various surfactants were added to 25 mL of artificial waste liquid, the actual amounts of addition can be calculated relatively from the above-described concentrations.

**[Table 17]**

| Added chemicals | Number of days | Average odor pleasantness |
|---|---|---|
| Control (ii) +TRITON X | After 5 days | - 0.67 |
| | After 7 days | - 1.00 |
| | After 9 days | - 1.00 |
| | After 14 days | - 1.00 |
| Control (ii) +soap | After 5 days | - 0.67 |
| | After 7 days | - 1.33 |
| | After 9 days | - 2.00 |
| Control (ii) +CATION G-50 100 mg/L | After 5 days | - 1.00 |
| | After 7 days | - 1.67 |
| | After 9 days | - 1.33 |
| | After 14 days | - 1.33 |
| Control (ii) +OSMORIN DA-50 30 mg/L | After 5 days | - 0.33 |
| | After 7 days | - 1.67 |
| | After 9 days | - 1.00 |
| | After 14 days | - 1.33 |
| Control (ii) +OSMORIN DA-50 100 mg/L | After 5 days | - 1.00 |
| | After 7 days | - 1.33 |
| | After 9 days | - 1.33 |
| | After 14 days | - 1.00 |
| Control (ii) +OSMORIN DA-50 300 mg/L | After 5 days | - 0.67 |
| | After 7 days | - 0.67 |
| | After 9 days | - 1.33 |
| | After 14 days | - 1.33 |

When the test results are viewed in terms of the odor pleasantness, the system into which 300 mg/L of TRITON X or DA-50 was incorporated showed slightly more satisfactory results than the Control (ii). When the systems into which G-50 and DA-50 were incorporated at a concentration of 100 mg/L were compared, the system into which DA-50 was incorporated was slightly better. Incidentally, a benzalkonium chloride solution is known to be used as a disinfectant for hands and fingers or an antiseptic solution for contaminated environments.

Taking all the results obtained thus far into consideration, it was found that the results of "pepper" give the most excellent effect of reducing foul odor.

Thus, each of the components of the following composition including pepper was placed in a mortar of a tabletop tableting machine (manufactured by Kikusui Seisakusho, Ltd.), the mixture was tableted by applying a pressure of 40 kN, and 50 g (70 tablets) each of lump-like treatment agents KS-22-α1, KS-22-α2, and KS-22-α3 were obtained. Furthermore, 50 g (70 tablets) of tab22, which did not contain pepper, was obtained (average diameter: 15 mm, average thickness: 5 mm). Incidentally, the pestle used was an upper and lower flat plate type pestle having a diameter of 15 mm.

**[Table 18]**

| KS-22-α1 | | |
|---|---|---|
| Substance name | % | g |
| KC200 (KC FLOCK) | 40.0% | 20 |
| Water-absorbing polymer (ST-573) | 30.0% | 15 |
| Lignin ST-202 | 2.0% | 1 |
| Slaked lime | 8.0% | 4 |
| KUNIPIA F | 2.0% | 1 |
| Humic acid | 6.0% | 3 |
| Zinc oxide type 1 | 4.0% | 2 |
| Black pepper | 2.0% | 1 |
| Zeolite | 5.0% | 2.5 |
| Calcium stearate | 1.0% | 0.5 |
| | 100.00 | 50 |

Incidentally, in the above-described Table 18, when the binder (KC200 (KC FLOCK)) is excluded and then the content ratio of each component is converted, the content ratio is as follows.

**[Table 19]**

| Component | % |
|---|---|
| Water-absorbing polymer (ST-573) | 50.0 |
| Lignin ST-202 | 3.3 |
| Slaked lime | 13.3 |
| KUNIPIA F | 3.3 |
| Humic acid | 10.0 |
| Zinc oxide type 1 | 6.7 |
| Black pepper | 3.3 |
| Zeolite | 8.3 |
| Calcium stearate | 1.8 |

**[Table 20]**

| KS-22-α2 | | |
|---|---|---|
| Substance name | % | g |
| KC200 (KC FLOCK) | 40.0% | 20 |
| Water-absorbing polymer (ST-573) | 30.0% | 15 |
| Lignin ST-202 | 2.0% | 1 |
| Slaked lime | 8.0% | 4 |
| KUNIPIA F | 2.0% | 1 |
| Humic acid | 6.0% | 3 |
| Zinc oxide type 1 | 2.0% | 1 |
| Black pepper | 4.0% | 2 |
| Zeolite | 5.0% | 2.5 |
| Calcium stearate | 1.0% | 0.5 |
| | 100.0% | 50 |

Incidentally, in the above-described Table 20, when the binder (KC200 (KC FLOCK)) is excluded and then the content ratio of each component is converted, the content ratio is as follows.

**[Table 21]**

| Component | % |
|---|---|
| Water-absorbing polymer (ST-573) | 50.0 |
| Lignin ST-202 | 3.3 |
| Slaked lime | 13.3 |
| KUNIPIA F | 3.3 |
| Humic acid | 10.0 |
| Zinc oxide type 1 | 3.3 |
| Black pepper | 6.7 |
| Zeolite | 8.3 |
| Calcium stearate | 1.8 |

**[Table 22]**

| KS-22-α3 | | |
|---|---|---|
| Substance name | % | g |
| KC200 (KC FLOCK) | 40.0% | 20 |
| Water-absorbing polymer (ST-573) | 30.0% | 15 |
| Lignin ST-202 | 2.0% | 1 |
| Slaked lime | 8.0% | 4 |
| KUNIPIA F | 2.0% | 1 |
| Humic acid | 6.0% | 3 |
| Zinc oxide type 1 | 0.0% | 0 |
| Black pepper | 6.0% | 3 |
| Zeolite | 5.0% | 2.5 |
| Calcium stearate | 1.0% | 0.5 |
| | 100.0% | 50 |

Incidentally, in the above-described Table 22, when the binder (KC200 (KC FLOCK)) is excluded and then the content ratio of each component is converted, the content ratio is as follows.

**[Table 23]**

| Component | % |
|---|---|
| Water-absorbing polymer (ST-573) | 50.0 |
| Lignin ST-202 | 3.3 |
| Slaked lime | 13.3 |
| KUNIPIA F | 3.3 |
| Humic acid | 10.0 |
| Zinc oxide type 1 | 0.0 |
| Black pepper | 10.0 |
| Zeolite | 8.3 |
| Calcium stearate | 1.8 |

**[Table 24]**

| tab22 | | |
|---|---|---|
| Substance name | % | g |
| KC200 (KC FLOCK) | 40.0% | 20 |
| Water-absorbing polymer (ST-573) | 30.0% | 15 |
| Lignin ST-202 | 4.0% | 2 |
| Slaked lime | 8.0% | 4 |
| KUNIPIA F | 2.0% | 1 |
| Humic acid | 6.0% | 3 |
| Zinc oxide type 1 | 4.0% | 2 |
| Zeolite | 5.0% | 2.5 |
| Calcium stearate | 1.0% | 0.5 |
| | 100.0% | 50 |

| | | |
|---|---|---|
| In the above-described tables, KC200 (KC FLOCK): manufactured by Nippon Paper Group average particle size 32 µm Water-absorbing polymer (SANFRESH ST-573): average particle size: 380 µm (more than 850 µm about 1% by mass, more than 106 µm and 850 µm or less 89% by mass, 106 µm or less about 10% by mass) Lignin: manufactured by Nippon Paper Group SAN-X P-202 average particle size: 60 µm Slaked lime: manufactured by Ube Material Industries, Ltd.; 100-mesh pass KUNIPIA F: average particle size: 139 µm Humic acid: manufactured by PIC BIO, Inc. Canadian humin (42 mm-through to 0.6 mm-on 9%, 0.6 mm-through to 0.3 mm-on 41%, 0.03 mm-through 49%) Zinc oxide type 1: manufactured by Hakusui Tech Co., Ltd. average particle size 0.6 µm Black pepper: SB FOOD Co., Ltd. table pepper Zeolite ZEOFILL W1 volume average particle size (D50): 13 µm Calcium stearate: SUNACE SAK-CS-P average particle size 7 µm (including a portion of particles having a size of 50 µm or more) | | |

Then, one tablet (0.72 g) of each of the above-described lump-like treatment agents was added to the Control (i).

In these experiments, the odor pleasantness was determined by allowing a plurality of persons (three persons) who had passed an odor panel selection test, in the same manner as in the previous experiments.

**[Table 25]**

| Test system | Number of days passed (d) | | |
|---|---|---|---|
| | 6 | 13 | 17 |
| tab22 | -0.33 | - 2.5 | - 2.5 |
| KS-22-α1 | 0.33 | 0.33 | 0.33 |
| KS-22-α2 | 0.33 | 0.33 | 0.33 |
| KS-22-α3 | 0.0 | 0.0 | 0.0 |

### <Production of imitation feces>

Subsequently, imitation feces used for tests were prepared by sequentially adding fatty acids, indole, and skatole, each in an amount sufficient to contribute to a certain extent to the odor, to stone powder clay having a texture and a specific gravity similar to those of feces, and mixing the components. The preparation concentrations are shown in the following table.

**[Table 26]**

| | Preparation concentration (mg/kg) |
|---|---|
| n-Butyric acid | 5,523 |
| n-Valeric acid | 5,887 |
| Isovaleric acid | 5,874 |
| Indole | 275 |
| Skatole | 275 |

| | |
|---|---|
| *Preparation concentration is the weight of addition of each component with respect to 1 kg of imitation feces. | |

In the test, 65 g of imitation feces was placed in a plastic bag, a specified amount (50 g) of each of the reference treatment agent 1, KS-22-α1, and KS-22-α1 powder type + a silver salt (product obtained by adding 0.5% by mass of silver nitrate to the total mass of KS-22-α1 powder type (not tableted)) was added thereto, subsequently 200 mL of pure water was added thereto, and the bag was immediately installed inside a container having a capacity of 17 L. In this way, evaluation of the control (treatment agent not added), the reference treatment agent 1, KS-22-α1, and KS-22-α1 powder type + silver salt was carried out.

### <Test results>

The measurement results for the concentrations of n-butyric acid, n-valeric acid, isovaleric acid, indole, and skatole, and the suppression rates of odorous substances with respect to the control are shown in the following table.

### (n-Butyric acid)

Regarding n-butyric acid, the volatilization concentration of the control (obtained by adding 200 mL of pure water to imitation feces) was 0.34 ppm on the fourth hour, and changed around 0.7 ppm from the third day. In contrast, the volatilization concentration of the reference treatment agent 1 was 0.15 ppm in four hours, 0.07 ppm on the third day, and about 0.04 ppm after one week. In the systems of KS-22-α1 and KS-22-α1 powder type + silver salt, the volatilization concentration was in low levels, such as 0.03 ppm or less on the fourth hour, and approximately 0.01 ppm or less from the first day onwards. In terms of the suppression rate, the system of KS-22-α1 powder type + silver salt showed 98% or more from the first day onwards.

The system of the reference treatment agent 1 showed a suppression rate of approximately 90% or more from the first day onwards; however, the system of KS-22-α1 showed performance with a suppression rate that was about 10% higher. Although this may seem like only about 10%, it implies that the odor was reduced to 1/10 or 1/50 of the odor of the control, and when considering that human response to odors is exponential, and that the guidelines for the odor intensity and concentration of the Offensive Odor Control Law are met, it is believed that an improvement of lowering the sensory evaluation (odor intensity) by about one level could be attempted with n-butyric acid.

### (n-Valeric acid)

Regarding n-valeric acid, the volatilization concentration of the control was 0.4 ppm in four hours, and around 1.3 ppm from the first day onwards. In contrast, the reference treatment agent 1 showed low levels such as 0.08 ppm in four hours and 0.03 ppm from the first day onwards. In the systems of KS-22-α1 and KS-22-α1 powder type + silver salt, the volatilization concentration was approximately 0.01 ppm or less from the fourth hour, and the systems showed higher performance than the reference treatment agent 1. In terms of the suppression rate with respect to the control, while the reference treatment agent 1 showed 80% on the fourth hour, the systems of KS-22-α1 and KS-22-α1 powder type + silver salt showed 95% or more on the fourth hour, and from the first day onwards, the systems showed a suppression rate of 99% or more whereas the reference treatment agent 1 showed a suppression rate in the range of 97% to 99%.

### (Isovaleric acid)

Isovaleric acid also showed a tendency similar to that of valeric acid, and the control showed a volatilization concentration of 0.33 ppm on the fourth hour and around 1.1 ppm from the first day onwards. The reference treatment agent 1 showed a volatilization concentration of 0.04 ppm on the fourth hour and 0.01 ppm or less from the first day onwards, and the systems of KS-22-α1 and KS-22-α1 powder type + silver salt showed a volatilization concentration of 0.01 ppm or less from the fourth hour. Even in terms of the suppression rate, isovaleric acid showed a tendency similar to that of n-valeric acid.

From the measurement results of these three kinds of fatty acids, it was shown that KS-22-α1 rapidly suppresses volatilization of acidic fecal odor gases in a short period of time. Furthermore, it can be said that these components were maintained at higher levels than conventional products and continuously suppressed volatilization over a period of two weeks.

### (Indole and skatole)

Regarding indole and skatole, which are representative fecal odor components having different chemical properties, even the controls showed a tendency that the volatilization concentration gradually increased, and indole showed a volatilization concentration of 0.017 ppm initially and 0.090 ppm in the second week. Furthermore, skatole showed a volatilization concentration of 0.005 ppm initially and 0.029 ppm in the second week.

In the reference treatment agent 1, the concentrations were lower than those in the control; however, indole showed a volatilization concentration of 0.010 to 0.027 ppm, and skatole showed a volatilization concentration of 0.004 to 0.016 ppm, showing a mild tendency of concentration increase up to the first week or the second week. In the systems of KS-22-α1 and KS-22-α1 powder type + silver salt, the volatilization concentration of indole was 0.002 to 0.010 ppm, and that of skatole was 0.001 to 0.003 ppm, which were clearly suppressed to low levels as compared with the Control and the reference treatment agent 1.

In view of the suppression rate, indole showed a suppression rate of 40% initially and 60% to 70% from the first day onwards, while skatole showed a suppression rate that greatly fluctuated but changed between 10% to 50%, showing a low suppressive effect. In particular, skatole is a substance that is considered to be representative of fecal odor, and results showed that the volatilization suppression rate of this substance was not sufficient.

On the other hand, the systems of KS-22-α1 and KS-22-α1 powder type + silver salt showed a suppression rate of about 75% for both indole and skatole on the fourth hour, subsequently slightly increased, and showed a suppression rate of 80% to 90% for KS-22-α1 and 90% or more for KS-22-α1 powder type + silver salt.

The reference treatment agent 1 showed a low suppressive effect on indole and skatole, and showed volatilization concentrations equal to or higher than those of the Controls; however, KS-22-α1 suppressed indole and skatole to high levels of approximately 80% or higher. It is believed that this also causes a decrease in the odor intensity attributed to indole and skatole by about one level.

**[Table 27]**

| n-Butyric acid (concentration in gas) | | | | |
|---|---|---|---|---|
| | Test time (hour) | | | |
| | 4 | 28 | 172 | 340 |
| Control (ppm by volume) | 0.3389 | 0.7164 | 0.6341 | 0.8399 |
| Control +reference treatment agent 1 (ppm by volume) | 0.1472 | 0.0697 | 0.0393 | 0.0473 |
| KS-22α1 (ppm by volume) | 0.0291 | 0.0125 | 0.0090 | 0.0110 |
| KS-22α1+silver nitrate 0.5% (ppm by volume) | 0.0105 | 0.0082 | 0.0071 | 0.0075 |

**[Table 28]**

| n-Butyric acid (suppression rate) | | | | |
|---|---|---|---|---|
| | Test time (hour) | | | |
| | 4 | 28 | 172 | 340 |
| Control +reference treatment agent 1 (%) | 56.6 | 90.3 | 93.8 | 94.4 |
| KS-22-α1 (%) | 91.4 | 98.3 | 98.6 | 98.7 |
| KS-22-α1+silver nitrate 0.5% (%) | 96.9 | 98.9 | 98.9 | 99.1 |

**[Table 29]**

| n-Valeric acid (concentration in gas) | | | | |
|---|---|---|---|---|
| | Test time (hour) | | | |
| | 4 | 28 | 172 | 340 |
| Control (ppm by volume) | 0.4024 | 1.2746 | 1.1948 | 1.4829 |
| Control +reference treatment agent 1 (ppm by volume) | 0.0778 | 0.0281 | 0.0138 | 0.0168 |
| KS-22α1 (ppm by volume) | 0.0136 | 0.0032 | 0.0015 | 0.0025 |
| KS-22α1+silver nitrate 0.5% (ppm by volume) | 0.0044 | 0.0019 | 0.0015 | 0.0011 |

**[Table 30]**

| n-Valeric acid (suppression rate) | | | | |
|---|---|---|---|---|
| | Test time (hour) | | | |
| | 4 | 28 | 172 | 340 |
| Control +reference treatment agent 1 (%) | 80.7 | 97.8 | 98.8 | 98.9 |
| KS-22-α1 (%) | 96.6 | 99.7 | 99.9 | 99.8 |
| KS-22-α1+silver nitrate 0.5% (%) | 98.9 | 99.8 | 99.9 | 99.9 |

**[Table 31]**

| Isovaleric acid (concentration in gas) | | | | |
|---|---|---|---|---|
| | Test time (hour) | | | |
| | 4 | 28 | 172 | 340 |
| Control (ppm by volume) | 0.3324 | 1.0781 | 0.9848 | 1.2398 |
| Control +reference treatment agent 1 (ppm by volume) | 0.0449 | 0.0143 | 0.0080 | 0.0090 |
| KS-22α1 (ppm by volume) | 0.0049 | 0.0049 | 0.0027 | 0.0035 |
| KS-22α1+silver nitrate 0.5% (ppm by volume) | 0.0018 | 0.0023 | 0.0020 | 0.0022 |

**[Table 32]**

| Isovaleric acid (suppression rate) | | | | |
|---|---|---|---|---|
| | Test time (hour) | | | |
| | 4 | 28 | 172 | 340 |
| Control +reference treatment agent 1 (%) | 86.5 | 98.7 | 99.2 | 99.3 |
| KS-22α1 (%) | 98.5 | 99.5 | 99.7 | 99.7 |
| KS-22α1+silver nitrate 0.5% (%) | 99.5 | 99.8 | 99.8 | 99.8 |

**[Table 33]**

| Indole (concentration in gas) | | | | |
|---|---|---|---|---|
| | Test time (hour) | | | |
| | 4 | 28 | 172 | 340 |
| Control (ppm by volume) | 0.0172 | 0.0467 | 0.0549 | 0.0900 |
| Control (i) (ppm by volume) | 0.0100 | 0.0152 | 0.0232 | 0.0268 |
| KS-22α1 (ppm by volume) | 0.0042 | 0.0056 | 0.0079 | 0.0096 |
| KS-22-α1+silver nitrate 0.5% (ppm by volume) | 0.0020 | 0.0024 | 0.0054 | 0.0061 |

**[Table 34]**

| Indole (suppression rate) | | | | |
|---|---|---|---|---|
| | Test time (hour) | | | |
| | 4 | 28 | 172 | 340 |
| Control +reference treatment agent 1 (%) | 41.9 | 67.4 | 57.8 | 70.2 |
| KS-22-α1 (%) | 75.6 | 87.9 | 85.5 | 89.4 |
| KS-22-α1+silver nitrate 0.5% (%) | 88.6 | 94.9 | 90.1 | 93.3 |

**[Table 35]**

| Skatole (concentration in gas) | | | | |
|---|---|---|---|---|
| | Test time (hour) | | | |
| | 4 | 28 | 172 | 340 |
| Control (ppm by volume) | 0.0054 | 0.0130 | 0.0175 | 0.0285 |
| Control +reference treatment agent 1 (ppm by volume) | 0.0045 | 0.0091 | 0.0163 | 0.0151 |
| KS-22-α1 (ppm by volume) | 0.0014 | 0.0022 | 0.0027 | 0.0024 |
| KS-22-α1+silver nitrate 0.5% (ppm by volume) | 0.0012 | 0.0007 | 0.0008 | 0.0007 |

**[Table 36]**

| Skatole (suppression rate) | | | | |
|---|---|---|---|---|
| | Test time (hour) | | | |
| | 4 | 28 | 172 | 340 |
| Control +reference treatment agent 1 (%) | 17.0 | 29.7 | 6.6 | 47.1 |
| KS-22-α1 (%) | 74.2 | 83.4 | 84.3 | 91.7 |
| KS-22-α1+silver nitrate 0.5% (%) | 77.0 | 94.9 | 95.2 | 97.7 |

While it was clarified that the reference treatment agent 1 is effective against fatty acids that are included in the fecal odor, the reference treatment agent 1 was less effective against alkaline compounds such as ammonia, indole, and skatole, and showed a possibility that depending on the situation, the odorous substances may increase compared to the case where the reference treatment agent 1 is not used.

Incidentally, the measurement results for the concentrations of n-butyric acid, n-valeric acid, isovaleric acid, indole, and skatole, as well as the suppression rates for odorous substances with respect to the Control obtained after 24 hours and after one week are summarized below.

**[Table 37]**

| Item | Evaluation target | 24 hours | | 1 week | |
|---|---|---|---|---|---|
| | | KS-22-α1 | KS-22-α1 +silver nitrate 0.5% | KS-22-α1 | KS-22-α1+ silver nitrate 0.5% |
| Volatilization concentration | Indole | 67.4%⇒ 87.9% | 67.4%⇒ 94.9% | 57.8%⇒ 85.5% | 57.8%⇒ 90.1% |
| | Skatole | 29.7%⇒ 83.4% | 29.7%⇒ 94.9% | 29.7%⇒ 83.4% | 29.7%⇒ 94.9% |
| | n-Butyric acid | 90.3%⇒ 98.3% | 90.3%⇒ 98.9% | 93.8%⇒ 98.6% | 93.8%⇒ 98.9% |
| | n-Valeric acid | 97.8%⇒ 99.7% | 97.8%⇒ 99.8% | 98.8%⇒ 99.9% | 98.8%⇒ 99.9% |
| | i-Valeric acid | 98.7%⇒ 99.5% | 98.7%⇒ 99.5% | 99.2%⇒ 99.7% | 99.2%⇒ 99.8% |

To summarize the above-described test results, the basic performance of the treatment agent containing slaked lime and pepper can promote a significant improvement in the volatilization concentrations of indole and skatole, which are the targets, and further improvement was also recognized for fatty acids, for which the reference treatment agent 1 also had satisfactory performance.

Subsequently, in order to verify the effect of the combination of slaked lime and pepper, the number of types of the constituent components was reduced, and an investigation was conducted.

Specifically, the following components were incorporated into the Control (i), and the odor pleasantness was determined in the same manner as described above.

**[Table 38]**

| Slaked lime (g) | Black pepper (g) | Water-absorbing polymer (g) | Number of test days | Odor pleasantness |
|---|---|---|---|---|
| 0.4 | 0 | 0 | 3 | -0.33 |
| 0.4 | 0 | 0 | 7 | - 1.00 |
| 04 | 0 | 0 | 14 | -0.33 |

**[Table 39]**

| Slaked lime (g) | Black pepper (g) | Water-absorbing polymer (g) | Number of test days | Odor pleasantness |
|---|---|---|---|---|
| 0 | 0 | 0.75 | 3 | - 1.67 |
| 0.1 | 0 | 0.75 | 3 | - 1.33 |
| 0 | 0 | 0.75 | 7 | - 2.00 |
| 0.1 | 0 | 0.75 | 7 | - 1.67 |
| 0.3 | 0 | 0.75 | 7 | - 0.17 |
| 0 | 0 | 0.75 | 14 | -2.67 |
| 0.1 | 0 | 0.75 | 14 | -2.67 |
| 0.3 | 0 | 0.75 | 14 | - 0.17 |

**[Table 40]**

| Slaked lime (g) | Black pepper (g) | Water-absorbing polymer (g) | Number of test days | Odor pleasantness |
|---|---|---|---|---|
| 0 | 0.05 | 0.75 | 3 | - 0.67 |
| 0.4 (33.3% by mass) | 0.05% (4.2% by mass) | 0.75 (62.5% by mass) | 3 | 0.00 |
| 0 | 0.05 | 0.75 | 7 | - 1.67 |
| 0.4 (33.3% by mass) | 0.05% (4.2% by mass) | 0.75 (62.5% by mass) | 7 | 0.00 |
| 0 | 0.05 | 0.75 | 14 | - 2.00 |
| 0.4 (33.3% by mass) | 0.05% (4.2% by mass) | 0.75 (62.5% by mass) | 14 | 0.00 |

**[Table 41]**

| Slaked lime (g) | Black pepper (g) | Water-absorbing polymer (g) | Number of test days | Odor pleasantness |
|---|---|---|---|---|
| 0 | 0.05 | 0.75 | 3 | - 0.67 |
| 0 | 0.3 | 0.75 | 3 | -0.33 |
| 0 | 0.05 | 0.75 | 7 | - 1.67 |
| 0 | 0.3 | 0.75 | 7 | -2.00 |
| 0 | 0.05 | 0.75 | 14 | -2.00 |
| 0 | 0.3 | 0.75 | 14 | - 2.33 |

**[Table 42]**

| Slaked lime (g) | Black pepper (g) | Water-absorbing polymer (g) | Number of test days | Odor pleasantness |
|---|---|---|---|---|
| 0.3 | 0 | 0.75 | 7 | - 0.17 |
| 0.3 (27.3% by mass) | 0.05 (4.5% by mass) | 0.75 (68.2% by mass) | 7 | 0.00 |
| 0.3 | 0 | 0.75 | 14 | - 0.17 |
| 0.3 (27.3% by mass) | 0.05 (4.5% by mass) | 0.75 (68.2% by mass) | 14 | 0.00 |
| 0.3 (25% by mass) | 0.15 (12.5% by mass) | 0.75 (62.5% by mass) | 14 | 0.00 |
| 0.3 (22.2% by mass) | 0.3 (22.2% by mass) | 0.75 (55.6% by mass) | 14 | 0.00 |

**[Table 43]**

| Slaked lime (g) | Black pepper (g) | Water-absorbing polymer (g) | Number of test days | Odor pleasantness |
|---|---|---|---|---|
| 0.3 | 0 | 0 | 3 | -0.33 |

As can be seen from the above results, it was found that the combination of slaked lime and pepper has a remarkable or disparate effect.

Next, the antibacterial/sterilizing effects of samples A to C were evaluated below.

### <Evaluation of antibacterial/sterilizing effects>

### (Sample preparation)

Each component of the following compositions was uniformly mixed to prepare samples A to C.

**[Table 44]**

| Sample A | |
|---|---|
| Substance name | % |
| Water-absorbing polymer (ST-573) | 50.0% |
| Lignin ST-202 | 3.5% |
| Slaked lime | 14.0% |
| KUNIPIA F | 3.5% |
| Humic acid | 10.0% |
| Zinc oxide type 1 | 7.0% |
| Black pepper | 3.5% |
| Zeolite | 8.5% |
| | 100.0% |

| | |
|---|---|
| In the above-described table, Lignin: manufactured by Nippon Paper Group SAN-X P-202 average particle size: 60 µm Slaked lime: manufactured by Ube Material Industries, Ltd.; 100-mesh pass KUNIPIA F: average particle size: 139 µm Humic acid: manufactured by PIC BIO, Inc. Canadian humin (42 mm-through to 0.6 mm-on 9%, 0.6 mm-through to 0.3 mm-on 41%, 0.03 mm-through 49%) Zinc oxide type 1: manufactured by Hakusui Tech Co., Ltd. average particle size 0.6 µm Black pepper: SB FOOD Co., Ltd. table pepper Zeolite ZEOFILL W1 volume average particle size (D50): 13 µm | |

**[Table 45]**

| Sample B | |
|---|---|
| Substance name | % |
| KC200 (KC FLOCK) | 38.0% |
| Sodium bicarbonate | 8.0% |
| Disodium hydrogen phosphate | 3.0% |
| Slaked lime | 12.0% |
| Zeolite | 2.0% |
| Zinc oxide | 0.5% |
| Water-absorbing polymer | 35.5% |
| Calcium stearate | 1.0% |
| | 100.0% |

| Sample C | |
|---|---|
| Substance name | % |
| Sodium bicarbonate | 13.0% |
| Disodium hydrogen phosphate | 5.0% |
| Slaked lime | 19.5% |
| Zeolite | 3.0% |
| Zinc oxide | 1.0% |
| Water-absorbing polymer | 57.0% |
| Calcium stearate | 1.5% |
| | 100.0% |

| | |
|---|---|
| In the above-described tables, KC200 (KC FLOCK): manufactured by Nippon Paper Group average particle size 32 µm Sodim bicarbonate: manufactured by Tosoh Corporation average particle size 90 µm Disodium hydrogen phosphate: manufactured by Mitejima Chemical Co., Ltd. average particle size 90 µm Slaked lime: manufactured by Ube Material Industries, Ltd.; 100-mesh pass Zeolite ZEOFILL W1 volume average particle size (D50): 13 µm Zinc oxide type 1: manufactured by Hakusui Tech Co., Ltd. average particle size 0.6 µm Water-absorbing polymer (SANFRESH ST-573): average particle size: 380 µm (more than 850 µm about 1% by mass, more than 106 µm 850 µm Calcium stearate: SUNACE SAK-CS-P average particle size 7 µm (including a portion of particles having a size of 50 µm or more) | |

### (Bacterial species)

The following three species, namely, Escherichia coli, Vibrio cholerae, and Clostridioides difficile, were selected as targets of measurement of the antibacterial/sterilizing effects. Escherichia coli is a facultative anaerobic, gram-negative bacillus, and is the most frequently detected bacterium in hospital laboratories. Furthermore, Vibrio cholerae is a gram-negative bacillus like Escherichia coli; however, it is a bacterial species that is often of concern for spread in low-hygienic environments during disasters. Clostridioides difficile is a gram-positive bacillus that is representative of opportunistic infection causative bacteria, and at the same time, this bacterium forms spores, is resistant to various disinfection and sterilization methods, and requires special attention in hospital infection control. The following five strains belonging to these three bacterial species were subjected to measurement.
· Escherichia coli:
   Standard strain ATCC25922
   Clinical isolate (2022 1-1110, blood isolate)

· Vibrio cholerae:
   Clinical isolate 1 (2009 7-1320, pus isolate)
   Clinical isolate 2 (2010 8-1057, fecal isolate)
· Clostridioides difficile:
   Clinical isolate (2021 8-68, fecal isolate)

The above-described clinical isolates were obtained from undefined patients and maintained at the Med-Tech Link Center, Chiba University Hospital. The above-described clinical isolates are not novel microorganisms. Therefore, the clinical isolates were not deposited with a depository institution. However, the present applicant is prepared to distribute the clinical isolates according to the present invention to a third party when it falls under any of the items of Article 27-3 of the Enforcement Regulations of the Patent Act of Japan, provided that the relevant laws and regulations are observed.

### (Preparation of bacterial sample)

### · Clostridioides difficile:

With regard to Clostridioides difficile, using sterile saline obtained by autoclaving sterile saline to eliminate dissolved oxygen, a bacterial solution to be tested of 0.5 Mcf (McFarland turbidimetry) was produced. Next, 30 mg of each of the samples A to C prepared as described above was placed in a 1.5-mL microtube, subsequently 1 mL each of the bacterial solution to be tested was added thereto and mixed, and the mixture was left to stand at room temperature (25°C; hereinafter, the same) for the reaction time (30 minutes or 24 hours) shown in the following Table 45 to prepare a test bacterial solution. The McFarland turbidimetry (Mcf) is a technique for estimating the number concentration of viable cells in a bacterial solution from turbidity, and 0.5 Mcf corresponds to 1×10⁸ CFU/mL of the spore-forming bacterium. In this case, the unit CFU (colony forming unit) is the number of colonies that form when bacteria are inoculated into a solid medium for growth, and indicates the number of proliferative microbial cells included in the inoculated microorganisms.

After being left to stand at room temperature for a predetermined time (30 minutes or 24 hours), the test bacterial solution was mixed again and then centrifuged at 3,000 rpm for 10 minutes to separate a supernatant. 10 µL of this supernatant was collected using a micropipette, inoculated into a medium by a semi-quantitative method using a platinum loop, and cultured (anaerobic culture) for 48 hours at 35°C in an anaerobic atmosphere (H₂ [10%]:N₂ [10%] :CO₂ [80%]). Furthermore, regarding a control bacterial solution to which no sample was added, immediately after the production of the bacterial solution to be tested, 10 µL thereof was collected using a micropipette, inoculated into a medium by a semi-quantitative method using a platinum loop, and cultured for 48 hours at 35°C in an anaerobic atmosphere (H₂ [10%]:N₂ [10%]:CO₂ [80%]). Incidentally, culture was carried out in Anaero Columbia RS blood agar medium (RS medium) (manufactured by Nippon Becton Dickinson Co., Ltd.). Furthermore, each test was performed in duplicate.

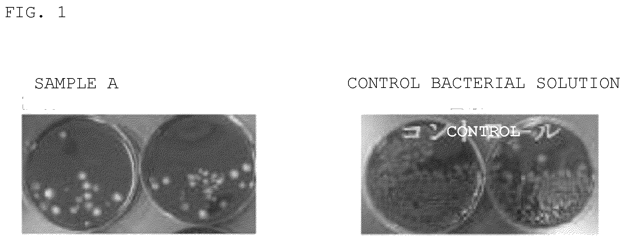

When the bacterial solution to be tested was subjected to the action of the sample A for 30 minutes and cultured, and the resultant was compared with a control bacterial solution, a decrease in the number of bacteria was recognized (see Fig. 1). For this reason, in order to quantify the decrease in the number of bacteria, a separate culture measurement for calculating the number of bacteria was carried out by the following method.

In the same manner as described above, a control bacterial solution immediately after production, and control bacterial solutions and test bacterial solutions that had been left to stand at room temperature for 30 minutes and 24 hours were prepared. After each of these bacterial solutions was mixed again and then centrifuged at 3,000 rpm for 10 minutes, and a supernatant was separated. A 10-fold dilution series (diluted bacterial solutions) were produced from this supernatant using sterile saline that had been autoclaved in advance in the same manner as described above, 100 µL of each diluted bacterial solution was collected from 1 mL and inoculated onto a medium (manufactured by Nippon Becton Dickinson Co., Ltd., Anaero Columbia RS blood agar medium (RS medium)), and culture (anaerobic culture) was carried out at 35°C for 4 days in an anaerobic atmosphere (H₂ [10%]:N₂ [10%]:CO₂ [80%]). After culturing for 4 days, the number of viable bacteria in the samples was evaluated from the number of colonies visually inspected on each medium. The results are shown in the following Table 45. In Table 45, a reaction time of 0 minutes indicates the sample immediately after the preparation of bacterial solution (the sample was subjected to neither reaction nor centrifugation). Furthermore, the figure in parentheses indicates the proportion of the number of bacteria obtained after the reaction of each sample with respect to the number of bacteria obtained from the control.

### · Escherichia coli and Vibrio cholerae:

With regard to Escherichia coli and Vibrio cholerae, bacterial solutions to be tested of 0.5 Mcf (McFarland turbidimetry) were produced using sterile saline. Next, 30 mg of each of the samples A to C prepared as described above was placed in a 1.5-mL microtube, subsequently 1 mL each of the bacterial solution to be tested was added thereto and mixed, and the mixture was left to stand at room temperature for the reaction time (30 minutes or 24 hours) shown in the following Table 45 to prepare a test bacterial solution. The McFarland turbidimetry (Mcf) is a technique for estimating the number concentration of viable cells in a bacterial solution from turbidity, and 0.5 Mcf corresponds to 1×10⁸ CFU/ml of Escherichia coli and 1×10⁷ CFU/ml of Vibrio cholerae, respectively. In this case, the unit CFU (colony forming unit) is the number of colonies that form when bacteria are inoculated into a solid medium for growth, and indicates the number of proliferative microbial cells included in the inoculated microorganisms.

After being left to stand at room temperature for a predetermined time (30 minutes or 24 hours), the test bacterial solution was mixed again and then centrifuged at 3,000 rpm for 10 minutes to separate a supernatant. 10 µL of this supernatant was collected using a micropipette, inoculated into a medium by a semi-quantitative method using a platinum loop, and cultured (aerial culture) at 35°C in an air atmosphere for the period indicated in the following table A (1 day or 4 days). Furthermore, regarding a control bacterial solution to which no sample was added, immediately after the production of the bacterial solution to be tested, 10 µl thereof was collected using a micropipette, inoculated into a medium, and cultured at 35°C in an air atmosphere (aerial culture) for 1 day or 4 days. Incidentally, culture of Escherichia coli was carried out in POREMEDIA (registered trademark) Drigalski improved medium (BTB medium) (manufactured by Eiken Chemical Co., Ltd.), and culture of Vibrio cholerae was carried out in Trypticase(TM) soy 5% sheep blood agar medium (BA medium) (manufactured by Nippon Becton Dickinson Co., Ltd.). Furthermore, each test was performed in duplicate.

After culturing for a predetermined time (1 day or 4 days), the presence or absence of the number of colonies visually inspected was examined. The results are shown in the following Table 46. In Table 46, the symbol "-" indicates that no growth was recognized.

**[Table 46]**

| Evaluation results for antibacterial properties in spore-forming bacterium | | | | | | | | | |
|---|---|---|---|---|---|---|---|---|---|
| Strain subjected to test | | Reaction time | | Culture period | | Control | Sample A | Sample B | Sample C |
| Clinical isolate (2021 8-68) | | 0 minutes | | 4 days | | 7.1×10⁶ | - | - | - |
| | | 30 minutes | | 4 days | | 1.6×10⁴ | 5.8×10³ (0.36) | 4.0×10³ (0.25) | 1.2×10⁴ (0.75) |
| | | 24 hours | | 4 days | | 1.7×10³ | 1.2×10² (0.07) | 5.5×10² (0.32) | 3.8×10² (0.22) |

| Evaluation results for antibacterial properties in Escherichia coli | | | | | | | | | |
|---|---|---|---|---|---|---|---|---|---|
| Strain subjected to test | | | Reaction time | | Culture period | | Sample A | Sample B | Sample C |
| Standard strain (ATCC25922) | | | 30 minutes | | 1 day | | -/- | -/- | -/- |
| | | | | | 4 days | | -/- | -/- | -/- |
| | | | 24 hours | | 1 day | | -/- | -/- | -/- |
| | | | | | 4 days | | -/- | -/- | -/- |
| Clinical isolate (2022 1-1110) | | | 30 minutes | | 1 day | | -/- | -/- | -/- |
| | | | | | 4 days | | -/- | -/- | -/- |
| | | | 24 hours | | 1 day | | -/- | -/- | -/- |
| | | | | | 4 days | | -/- | -/- | -/- |

| Evaluation results for antibacterial properties in Vibrio cholerae | | | | | | | | | |
|---|---|---|---|---|---|---|---|---|---|
| Strain subjected to test | Reaction time | Culture period | Sample A | Sample B | | Sample C | | | |
| Clinical isolate 1 (2009 7-1320) | 30 minutes | 1 day | -/- | -/- | | -/- | | | |
| | | 4 days | -/- | -/- | | -/- | | | |
| | 24 hours | 1 day | -/- | -/- | | -/- | | | |
| | | 4 days | -/- | -/- | | -/- | | | |
| Clinical isolate 2 (2010 8-1057) | 30 minutes | 1 day | -/- | -/- | | -/- | | | |
| | | 4 days | -/- | -/- | | -/- | | | |
| | 24 hours | 1 day | -/- | -/- | | -/- | | | |
| | | 4 days | -/- | -/- | | -/- | | | |

As shown in the above-described Table 46, in the spore-forming bacterium, the decrease in the number of bacteria compared to the control remained at 25% to 82% when each sample was reacted for 30 minutes, and a decrease in the number of bacteria of 68% to 99.94% was confirmed when each sample was reacted for 24 hours. These results suggest that the antibacterial effect is greater than that of samples of the current manufactured products. In contrast, in Escherichia coli (E. coli) and Vibrio cholerae (V. cholerae), no bacterial growth was recognized at all after reaction of the reagent for 30 minutes. Above all, in the case of E. coli, since 0.5 Mcf = 10⁸ CFU/mL, it is believed that the number concentration of viable bacteria in the bacterial solution at the start of culture had decreased to 10⁶ CFU/mL; however, since no bacterial growth was recognized at all after the culture, it is believed that at least an effect of controlling to 10⁶ CFU/mL (99.9999%) or more was obtained. Furthermore, in the case of V. cholerae, since 0.5 Mcf = 10⁷ CFU/mL, it is believed that the number concentration of viable bacteria decreased to a maximum of 10⁵ CFU/mL due to centrifugation; however, as with E. coli, since no bacterial growth was recognized at all after the culture, it is believed that at least an effect of controlling to 10⁵ CFU/mL (99.999%) or more was obtained. In both cases of Escherichia (E. coli) and Vibrio cholerae (V. cholerae), since no bacterial growth was recognized even after four days of culture, it is believed that all the bacteria had been killed.

It is presumed that the differences in the results between the above-described spore-forming bacterium (C. difficile), Escherichia coli (E. coli), and Vibrio cholerae (V. cholerae) are mainly due to the influence of the spores possessed by the spore-forming bacterium. Considering that sterilization of spores using disinfectants such as alcohol and benzalkonium chloride is ineffective, and spores cannot be completely inactivated even by boiling at 100°C (that is, it is difficult to completely kill spores), sample A of the present invention is expected to be clinically useful.

In the present investigation, in order to allow the reagent to react with the bacterial solution, the reagent is present in a suspended state in the sample. For this reason, the centrifuged supernatant was used for the culture measurement. Due to this centrifugation, the number of bacteria was reduced to about 1/100 in Escherichia coli (E. coli), to about 1/10 to 1/100 in Vibrio cholerae (V. cholerae), and to about 1/500 in the spore-forming bacterium (C. difficile). Furthermore, by leaving the bacterial solutions to stand for 24 hours, the number of bacteria was reduced to about 1/1 to 1/10 in E. coli, to about 1/10 to 1/100 in V. cholerae, and to about 1/10 in C. difficile.

Although embodiments of the present invention have been described in detail, it is apparent that these were explanatory, illustrative, and not limitative, and the scope of the present invention should be definitely interpreted by the appended claims.

The present invention includes the following aspects and configurations.
1. A treatment agent for treating waste liquid, the treatment agent including slaked lime and pepper.
2. The treatment agent according to 1., wherein the pepper is not in a form of piperine extract.
3. The treatment agent according to 1. or 2., further including a water-absorbing polymer.
4. The treatment agent according to any one of 1. to 3., wherein a content ratio (% by mass) of the slaked lime is 0.1% to 40% by mass, and a content ratio (% by mass) of the pepper is 0.05% to 30% by mass.
5. The treatment agent according to any one of 1. to 4., further including zinc oxide.
6. The treatment agent according to any one of 1. to 5., further including at least one selected from the group consisting of lignin, bentonite, and zeolite.
7. The treatment agent according to any one of 1. to 6., further including humic acid.
8. The treatment agent according to any one of 1. to 7., wherein the waste liquid includes excrement, blood, vomit, or body fluids discharged from a human being or an animal.
9. The treatment agent according to any one of 1. to 8., wherein the waste liquid includes at least one of indole and skatole as a foul odor component.
10. The treatment agent according to any one of 1. to 9., wherein the treatment agent is used to suppress or inhibit spore germination or proliferation of a spore-forming bacterium.
11. The treatment agent according to 10., wherein the spore-forming bacterium is Clostridioides difficile.
12. A method for suppressing or inhibiting spore germination or proliferation of a spore-forming bacterium, the method including bringing waste liquid into contact with the treatment agent according to any one of 1. to 11.

Thus, the first invention has been described above. Subsequently, a second invention will be described.

### <Second invention>

### TITLE OF THE INVENTION: WASTE LIQUID TREATMENT IMPLEMENT AND DOWNSTREAM-SIDE WASTE LIQUID TREATMENT IMPLEMENT

### TECHNICAL FIELD

The present invention relates to a waste liquid treatment implement and a downstream-side waste liquid treatment implement.

### BACKGROUND ART

Proper disposal of excrement is important for maintaining a hygienic and pleasant environment; however, that may be difficult in a variety of situations and places. Examples thereof include emergency evacuation sites in the event of an emergency disaster where a flushing toilet cannot be used, regions where infrastructure is insufficient, such as mountains and beaches, living rooms for persons who require nursing care and cannot use a toilet by themselves, ostomy pouches that hold the feces and the like of a patient who has received stoma creation surgery such as colostomy, and inside hospitals such as operation rooms and endoscopy rooms.

For example, to take the endoscopy room as an example, endoscopic examinations are divided into upper examinations, which are performed to examine the stomach and the like through the mouth or the nose, and lower examinations, which are performed to examine the rectum, large intestine, and small intestine through the anus. In both the upper and lower examinations, suction of body fluids is performed, and in some cases, since the examination is performed while rinsing with water, waste liquid mixed with excrement, blood, and body fluids is discharged. Waste liquid containing body fluids thus generated is subjected to waste disposal.

In Patent Document 1, a disposable collecting container made a synthetic resin has been proposed as one idea for waste disposal.

However, even in a case where disposal collecting containers are used, there is a problem that waste liquid in the container may leak out of the container due to damage or the like in the stage of transporting the container after collection to a processing facility, which not only poses hygiene problems but may also be unpreferable for the person who ultimately processes the waste liquid.

Thus, in Patent Document 2, in order to provide a treatment container for waste liquid containing body fluids, which allows the waste liquid containing body fluids stored in the container to be easily incinerated and which is excellent in terms of hygiene and safety, a treatment container for waste liquid containing body fluids has been proposed, in which a waste liquid inlet pipe and an exhaust pipe for exhausting air in the collecting container are provided in the upper part of a sealed collecting container made of a synthetic resin for storing waste liquid containing body fluids, characterized in that in the upper part of the collecting container, a coagulant container holding a coagulant whose entirety or main component is formed of a macromolecular water-absorbing polymer is provided, and an inputting means for inputting the coagulant inside the coagulant container into the waste liquid stored in the collecting container is provided.

### CITATION LIST

### PATENT DOCUMENT

Patent Document 1: JP 51-48589 A
Patent Document 2: JP 02-157083 A

In Patent Document 2, a container for storing body fluids is is connected to a pipe, the body fluids are held in the container by suction, and the body fluids are brought into contact with a treatment agent containing a macromolecular water absorbent in the container to coagulate the body fluids.

However, the present inventors found that with such a configuration, there is a risk that the material solidified in the container may be drawn into the downstream-side pipe and clog the downstream-side pipe. There is a risk that such situations may occur not only when endoscopes are used in medical settings, but also when devices such as manually operated pumps are used at disaster sites.

Therefore, it is an object of the invention to provide a novel technology related to waste liquid treatment, by which when a treatment agent is disposed in advance in a container, and when a tubular member is connected to the container to collect body fluids in the container, the body fluids that have come into contact with the treatment agent can be prevented from clogging the path of the tubular member connected to the container.

An aspect of the present invention is a waste liquid treatment implement. The waste liquid treatment implement has a container, a lid part, a partition member, and an excrement treatment agent. The container provides an internal space which can hold body fluids that are included in a human being or discharged from a human being, and an opening part through which body fluids can be introduced into the internal space by suction. The lid part can be attached to the container so as to close the opening part of the container, and includes an inlet part that allows body fluids to flow to the internal space by suction. The partition member is disposed in the internal space and is configured to partition the internal space into a first internal space that is proximal to the opening part, and a second internal space that is located distal to the opening part relative to the first internal space. The excrement treatment agent is disposed in the second internal space of the container and is configured to be capable of changing body fluids into a solid swollen material when brought into contact with the body fluids. The partition member is configured to allow body fluids that flow from the inlet part into the internal space to flow between the first internal space and the second internal space, and to block the flow of a swollen material produced when the body fluids that have flowed into the second internal space come into contact with the excrement treatment agent, into the first internal space.

Furthermore, an aspect of the present invention is a downstream-side waste liquid treatment implement that has a second container, a second lid part, the above-described excrement treatment agent, and a displacement member, and is connectable to the above-described waste liquid treatment implement through a tubular member. The second container is provided with the above-described internal space and the above-described opening part. The second lid part includes the above-described inlet part that can be attached to the second container so as to close the opening of the second container, and a second outlet part through which the gas discharged from the internal space by suction can flow to the downstream side. The excrement treatment agent is disposed in the internal space of the second container, and turns body fluids into a swollen material when brought into contact with the body fluids. The displacement member blocks the flow of body fluids and the swollen material to the downstream side when the swollen material produced upon contact of the excrement treatment agent with body fluids reaches a predetermined height in the internal space.

According to the waste liquid treatment implement and the downstream-side waste liquid treatment implement according to an aspect of the present invention, when a treatment agent is disposed in advance in a collecting container, and when a tubular member is connected to the container to collect body fluids in the container, the body fluids that have come into contact with the treatment agent can be prevented from clogging the path of the tubular member connected to the container.

### BRIEF DESCRIPTION OF DRAWINGS

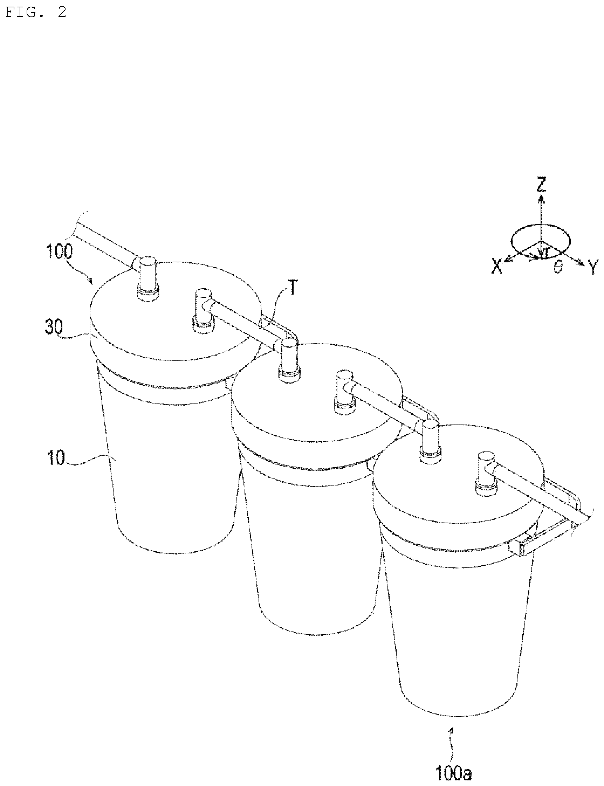

Fig. 2 is a schematic perspective view illustrating a waste liquid treatment implement and a downstream-side waste liquid treatment implement according to embodiments of the present invention;

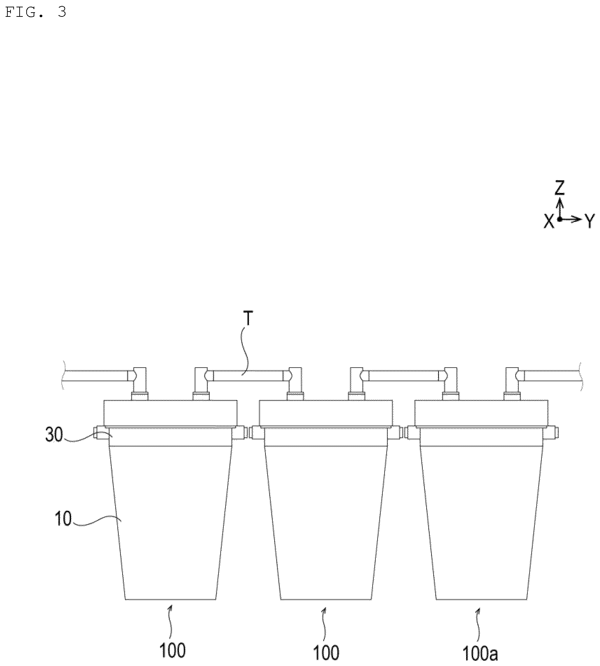

Fig. 3 is a front view of Fig. 2;

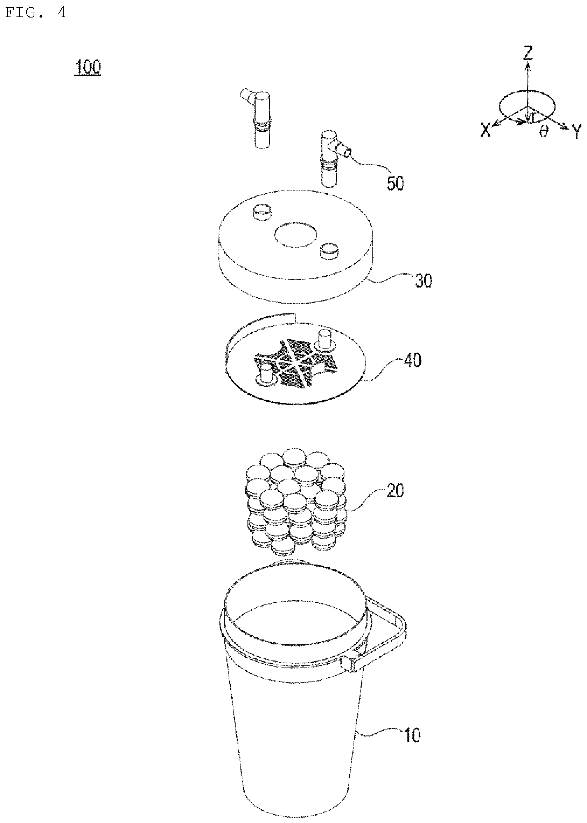

Fig. 4 is an exploded perspective view showing a configuration of the waste liquid treatment implement according to Fig. 2;

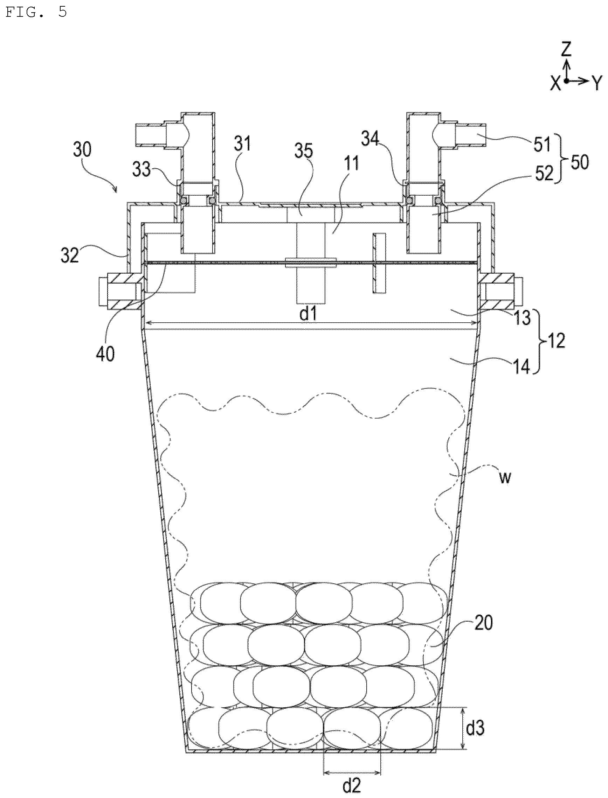

Fig. 5 is a cross-sectional view taken along the height direction, illustrating the inside of the waste liquid treatment implement according to Fig. 3;

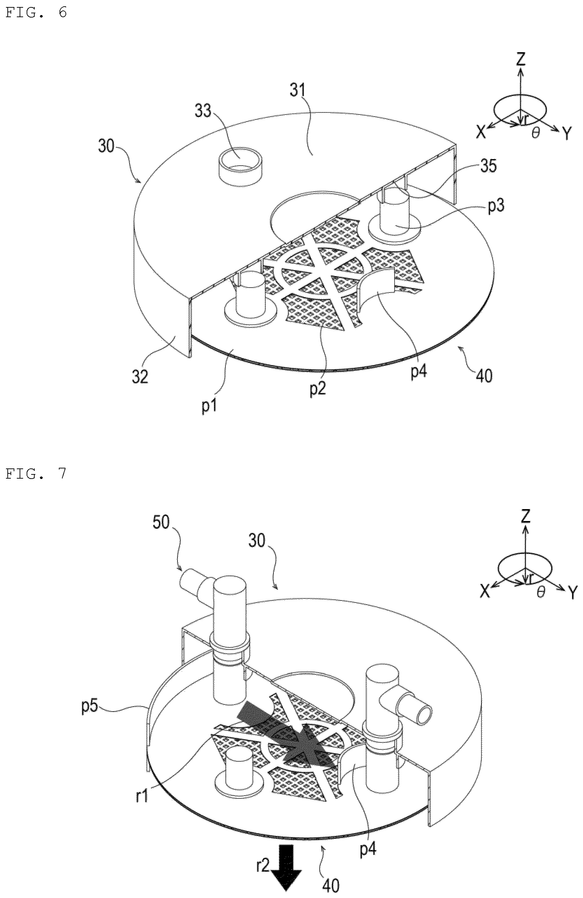

Fig. 6 is a perspective view illustrating a lid part and a partition member constituting the waste liquid treatment implement according to Fig. 3;
Fig. 7 is a perspective view illustrating a lid part and a partition member cut at a position different from that in Fig. 6;

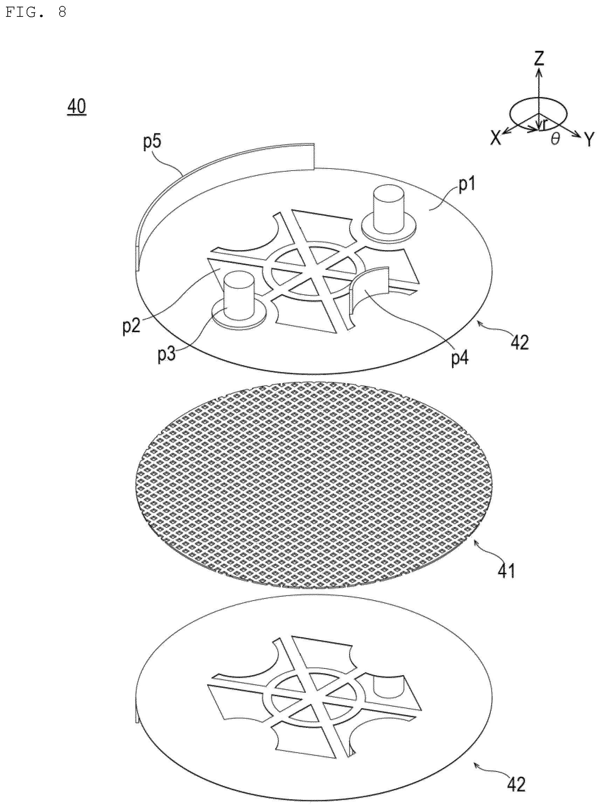

Fig. 8 is an exploded perspective view illustrating the configuration of the partition member;

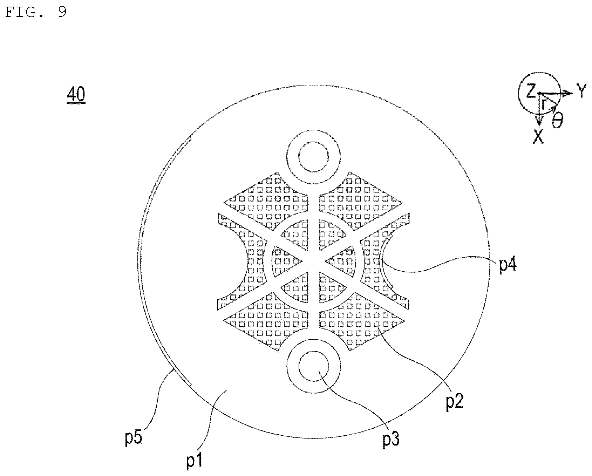

Fig. 9 is a plan view illustrating the partition member;

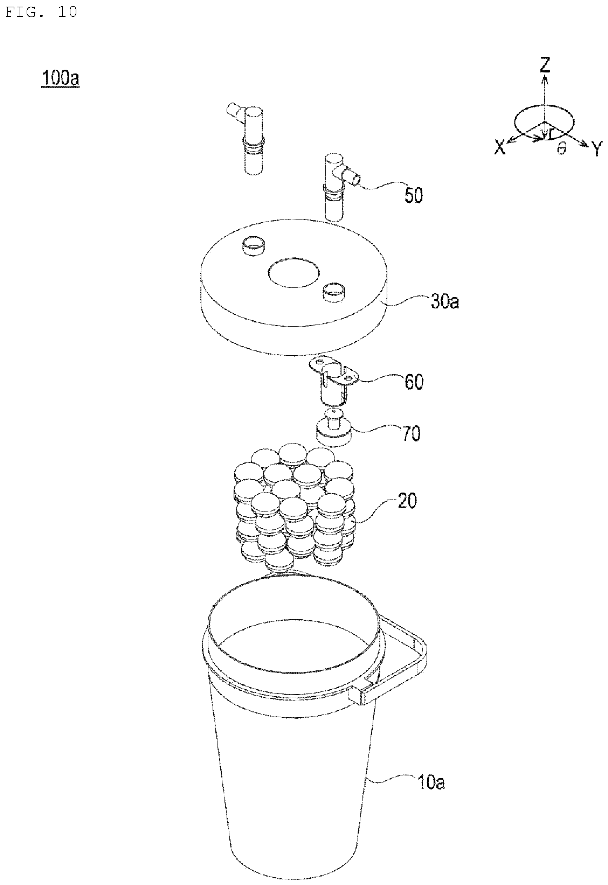

Fig. 10 is an exploded perspective view illustrating the configuration of the downstream-side waste liquid treatment implement shown in Fig. 2;

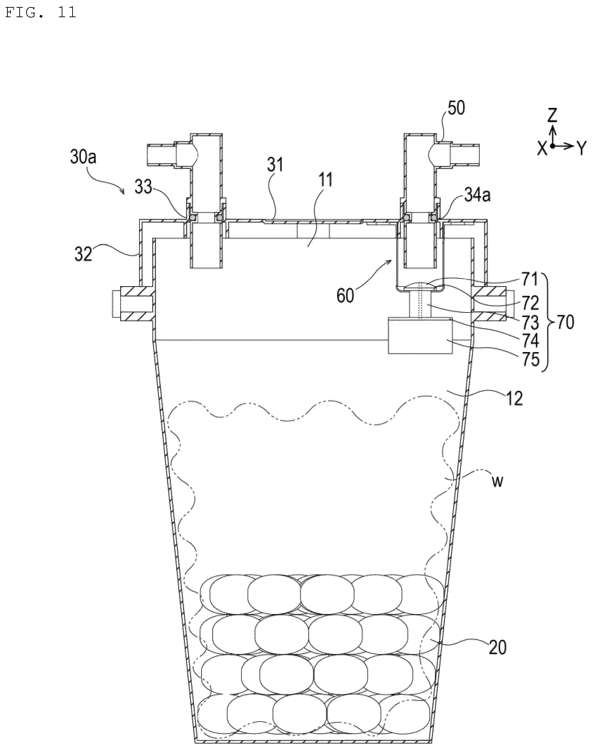

Fig. 11 is a cross-sectional view taken along the height direction, illustrating the inside of the downstream-side waste liquid treatment implement;

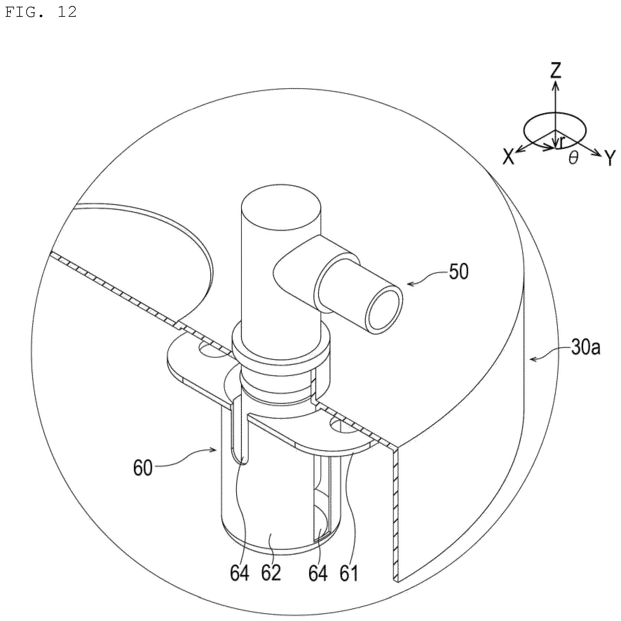

Fig. 12 a perspective view illustrating a lid part, a connecting member, and an attachment member constituting the downstream-side waste liquid treatment implement, with the lid part shown in a cutaway state;

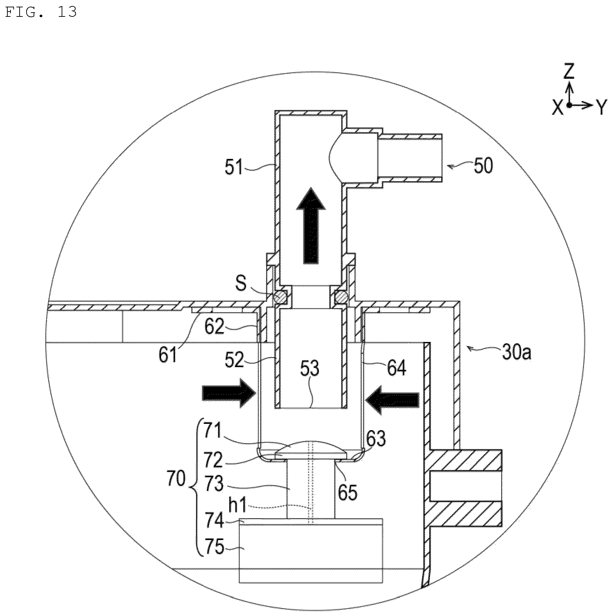

Fig. 13 is an enlarged view of Fig. 11 illustrating the lid part, the connecting member, the attachment member, and a displacement member, and showing a situation in which a gas or the like utilized for suction flows through the inside of the attachment member;

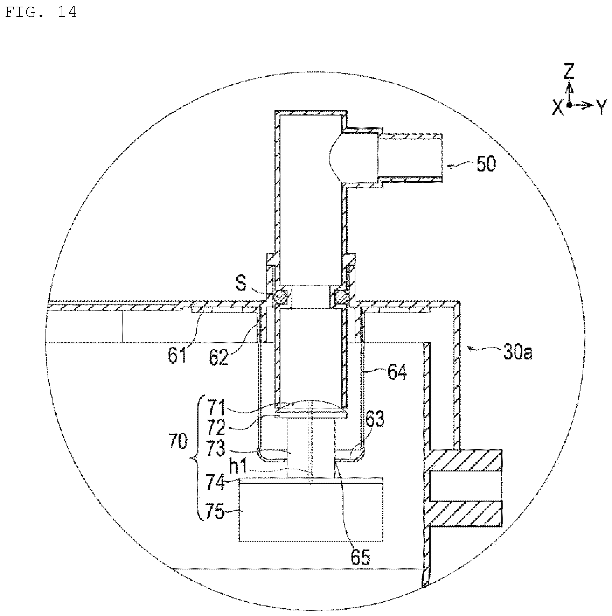

Fig. 14 is an enlarged view illustrating a state in which the position of the displacement member in Fig. 12 is different from that in Fig. 12;

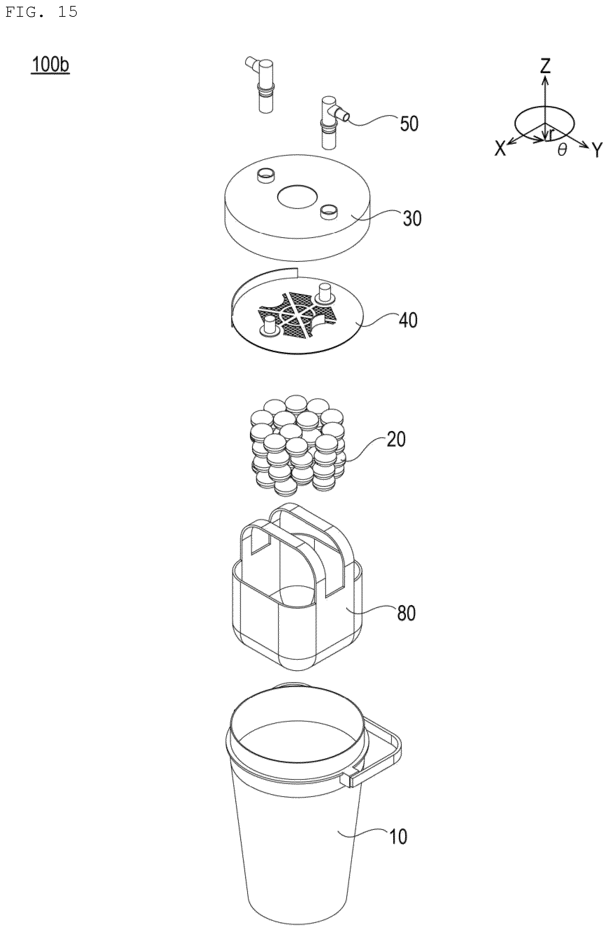

Fig. 15 is an exploded perspective view of a waste liquid treatment implement showing a modification example of Fig. 3;

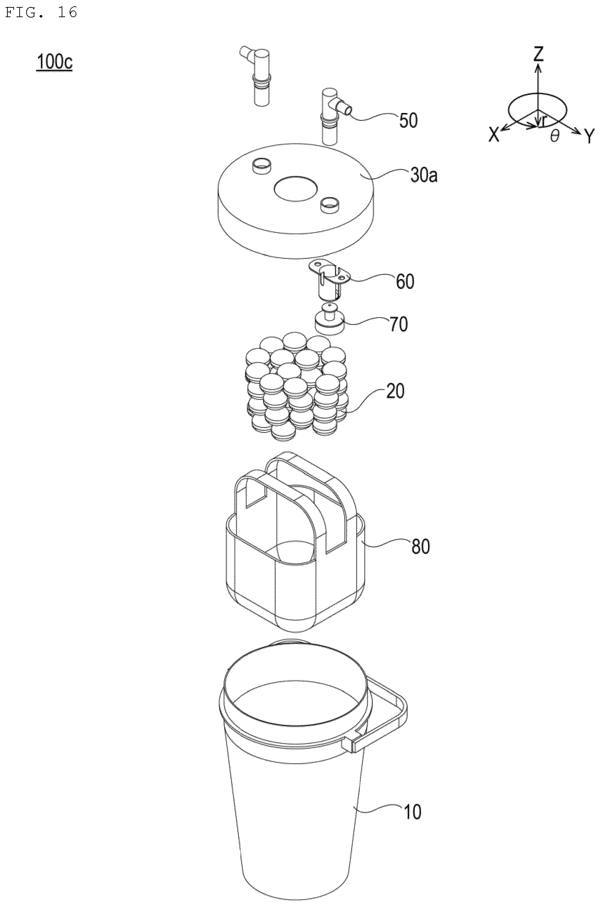

Fig. 16 is an exploded perspective view illustrating a downstream-side waste liquid treatment implement according to a modification example of Fig. 9;

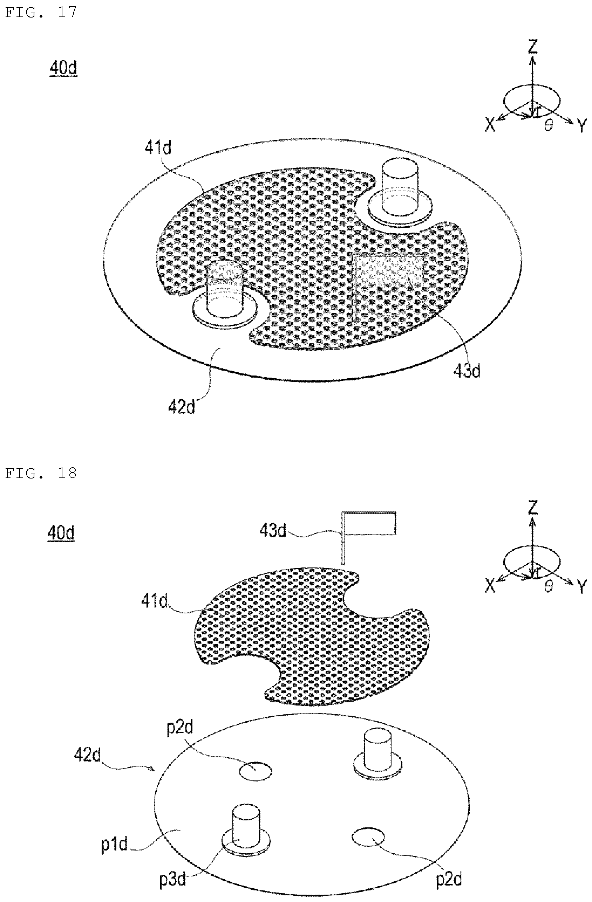

Fig. 17 is a perspective view illustrating a partition member according to a modification example;
Fig. 18 is an exploded perspective view of Fig. 17;

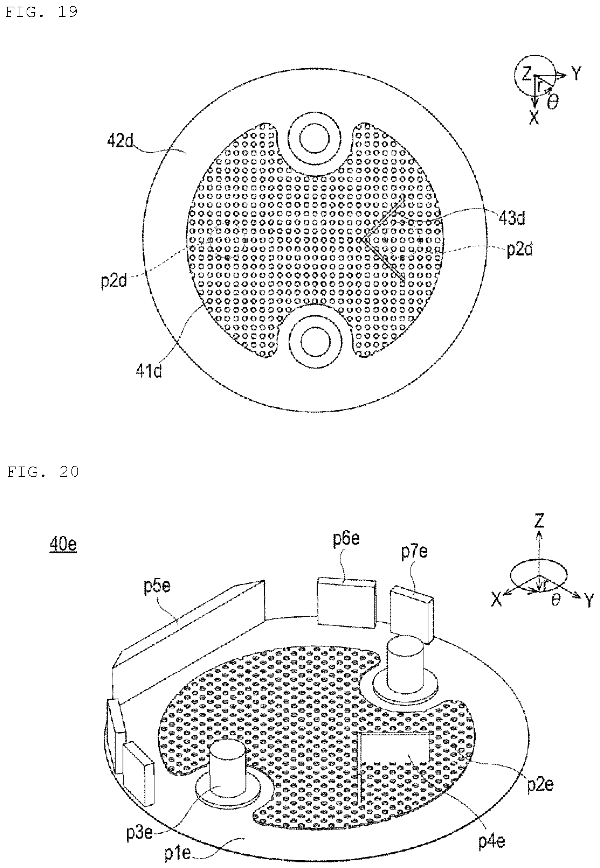

Fig. 19 is a plan view of Fig. 17;
Fig. 20 is a perspective view illustrating a partition member according to a modification example;

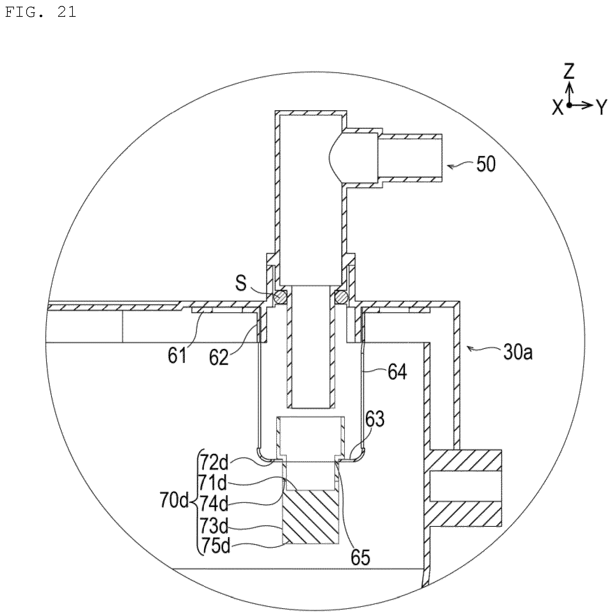

Fig. 21 is a diagram illustrating a modification example of Fig. 13;

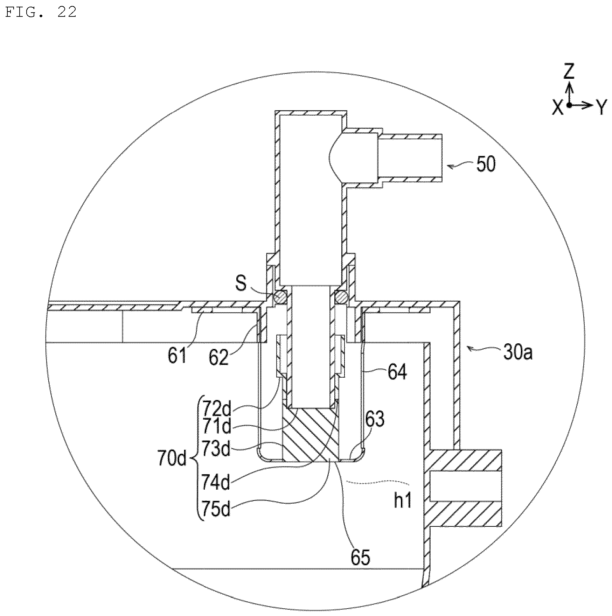

Fig. 22 is a diagram illustrating a modification example of Fig. 14;

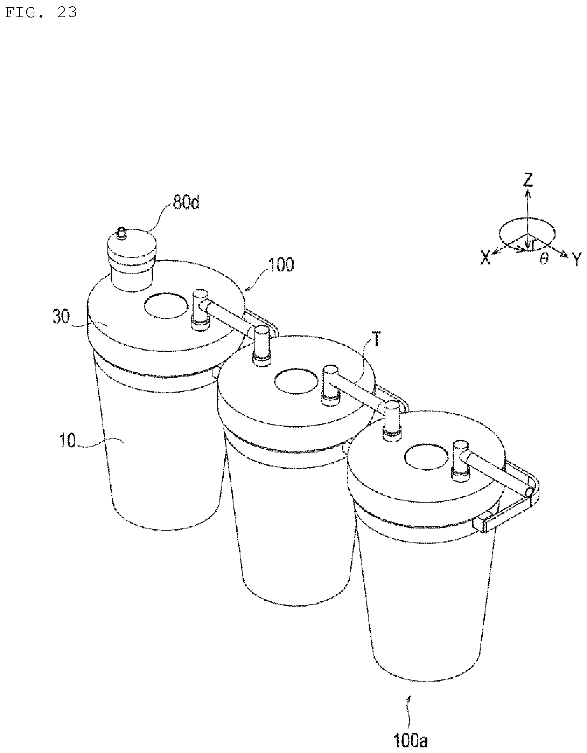

Fig. 23 is a perspective view illustrating a modification example of Fig. 2;

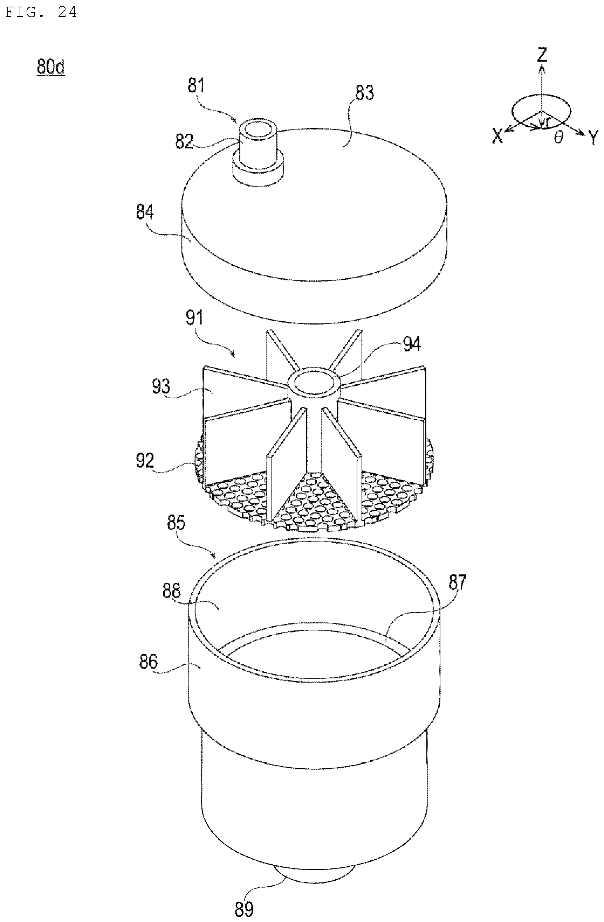

Fig. 24 is an exploded perspective view illustrating a capture assembly attached to a waste liquid treatment implement according to a modification example; and

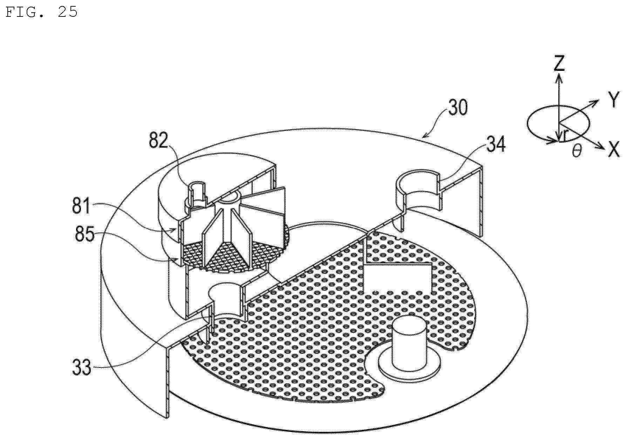

Fig. 25 is a diagram illustrating the inside of the capture assembly.

### BEST MODES FOR CARRYING OUT THE INVENTION

Embodiments for carrying out the second invention will be described below in detail with reference to the drawings. The embodiments shown herein are merely illustrative for embodying the technical ideas of the present invention, and are not intended to limit the present invention. Furthermore, all other possible embodiments, examples, operational technologies, and the like that can be conceived by those skilled in the art to the extent that does not deviate from the gist of the present invention are all included in the scope and gist of the present invention, and are also included in the scope of the inventions described in the claims and their equivalents.

In addition, for the convenience of illustration and ease of understand, the drawings attached to the present specification may be represented schematically with appropriate changes in scale, aspect ratio, shape, and the like from the actual objects; however, these are merely examples and are not intended to limit the interpretation of the present invention.

Furthermore, in the following description, the invention will be described by using ordinal numbers such as "first" and "second"; however, unless particularly stated otherwise, these ordinal numbers are used for convenience and do not dictate any order.

For ease of explanation, a coordinate system is indicated in the drawings. XY in the orthogonal coordinates is a plane parallel to the plane on which a waste liquid treatment implement 100 or a downstream-side waste liquid treatment implement 100a is placed, and for convenience, XY is referred to as planar direction XY. Z in the orthogonal coordinates is a direction orthogonal to the planar direction XY, and for convenience, Z is referred to as height direction Z. r in the cylindrical coordinates is intended to indicate a direction extending in the diameter direction or a radial direction from the center of any of the container 10 or 10a, or the lid 30 or 30a, and is referred to as radial direction r. θ in the cylindrical coordinates is intended to indicate a circumferential direction or an angular direction of the container 10, and for convenience, θ is referred to as circumferential direction θ**.**

The waste liquid treatment implement 100 and the downstream-side waste liquid treatment implement 100a according to the embodiments will be described below with reference to Fig. 2 to Fig. 14. Fig. 2 is a schematic perspective view showing the waste liquid treatment implement 100 and the downstream-side waste liquid treatment implement 100 according to the embodiments of the present invention. Fig. 3 is a front view of Fig. 2. Fig. 4 is an exploded perspective view showing the configuration of the waste liquid treatment implement 100 according to Fig. 2. Fig. 5 is a cross-sectional view taken along the height direction, showing the inside of the waste liquid treatment implement 100. Fig. 6 is a perspective view showing a lid part 30 and a partition member 40 constituting the waste liquid treatment implement 100 according to Fig. 3. Fig. 7 is a perspective view showing the lid part and the partition member cut at a position different from that in Fig. 6. Fig. 8 is an exploded perspective view showing the configuration of the partition member 40. Fig. 9 is a plan view showing the partition member 40.

Fig. 10 is an exploded perspective view showing the configuration of the downstream-side waste liquid treatment implement 100a. Fig. 11 is a cross-sectional view taken along the height direction, showing the inside of the downstream-side waste liquid treatment implement 100a. Fig. 12 a perspective view showing a lid part 30a, a connecting member 50, and an attachment member 60 constituting the downstream-side waste liquid treatment implement 100a. In Fig. 6, Fig. 7, and Fig. 12, for convenience of explanation, the lid parts 30 and 30a are depicted in a partially cutaway state. Fig. 13 is an enlarged view of Fig. 11 showing the connecting member 50, the attachment member 60, and a displacement member 70, and showing a situation in which a gas or the like utilized for suction flows through the inside of the connecting member 50. Fig. 14 is an enlarged view showing a state in which the position of the displacement member 70 in Fig. 13 is different from that in Fig. 13.

The waste liquid treatment implement 100 and the downstream-side waste liquid treatment implement 100a according to the present embodiment can be utilized when body fluids discharged from patients are treated using an endoscope or the like in medical institutions such as hospitals, or when body fluids discharged from people living in disaster-stricken areas or the like are treated in disaster-stricken areas or the like. In Fig. 2 and Fig. 3, two waste liquid treatment implements 100 and one downstream-side waste liquid treatment implements 100a are prepared, and each is connected by a tubular member T. The waste liquid treatment implement 100 and the downstream-side waste liquid treatment implement 100a will be described below.

### <Waste liquid treatment implement>

To briefly explain with reference to Fig. 4 and the like, the waste liquid treatment implement 100 has a container 10, an excrement treatment agent 20, a lid part 30, a partition member 40, and a connecting member 50. The implement will be described below in detail.

### <Container>

As shown in Fig. 5, the container 10 is configured to provide an internal space which can hold body fluids that are included in a human being or discharged from a human being, and an opening part 11 through which the body fluids can be introduced into the internal space 12. The specific configuration of the container 10 is not particularly limited as long as the container holds body fluids therein and is not damaged or the like in a state in which the inside is put under negative pressure by a pump or the like. The upper part of the container 10 can be provided with a fastening part (fastening shape) such as a male thread shape that engages with a thread shape provided on a flange part 32 of the lid part 30 that will be described below. For the container 10, a material such as a thermoplastic resin such as polyethylene, polypropylene, polycarbonate, or ABS (acrylonitrile-butadiene-styrene) can be used; however, the thickness, shape, and the like of the container 10 are not particularly limited as long as they are noticeably not suitable for holding body fluids.

### <Excrement treatment agent>

The excrement treatment agent 20 is disposed in a second internal space 14 that will be described below and constitutes the internal space 12 of the container 10, and is configured to be able to change body fluids into a solid swollen material w when brought into contact with the body fluids. The excrement treatment agent 20 is configured to be lump-like as shown in Fig. 5 and the like in the present embodiment, and is configured such that when disposed in the internal space 12 of the container 10, gaps can be formed between adjacent excrement treatment agents 20 to the extent that can be visually recognized. With such a configuration, when body fluids are introduced into the internal space 12, and the excrement treatment agent 20 comes into contact with the body fluids and swells, the excrement treatment agent 20 located in the lower part of the container 10 can be prevented from being unable to come into contact with the body fluids due to the excrement treatment agent 20 that is located in a relatively upper part and has swelled earlier.

The excrement treatment agent 20 contains a water-absorbing polymer. By containing a water-absorbing polymer, moisture and the like in the treatment target material are absorbed, and the treated material is likely to be solidified (likely to become jelly-like). In the present specification, the term "water-absorbing polymer (water-absorbing resin)" refers to a macromolecular gelling agent having a water swelling capacity (CRC) of 5 g/g or more as defined by ERT441.2-02, and containing 50% by mass or less of a water-soluble component (Ext) defined by ERT470.2-02.

According to an embodiment of the present invention, specific examples of the water-absorbing polymer include, for example, starch-based water-absorbing polymers such as a starch-acrylonitrile graft polymer hydrolysate, and a starch-acrylic acid graft polymer; cellulose-based water-absorbing polymers such as a cellulose-acrylonitrile graft polymer and a cellulose-styrene sulfonic acid graft copolymer; polysaccharide-based water-absorbing polymers such as pectin; protein-based water-absorbing polymers such as collagen; polyvinyl alcohol-based water-absorbing polymers such as a crosslinked polyvinyl alcohol polymer; acrylic water-absorbing polymers such as a sodium polyacrylate crosslinked body, an acrylic acid polymer partial sodium salt crosslinked product, and a sodium acrylate-vinyl alcohol copolymer; maleic anhydride-based water-absorbing polymers, vinylpyrrolidone-based water-absorbing polymers; and polyether-based water-absorbing polymers such as a crosslinked polyethylene glycol-diacrylate polymer. These water-absorbing polymers may be used singly, or two or more kinds thereof may be used in combination. Furthermore, these water-absorbing polymers may be synthesized, or commercially available products may be used. Examples of the commercially available products include, for example, AQUA KEEP (registered trademark) SA (manufactured by Sumitomo Seika Chemicals Co., Ltd.), AQUALIC (registered trademark) CA (manufactured by Nippon Shokubai Co., Ltd.), SANFRESH (ST-250, ST-100, ST-573), AQUAPEARL (manufactured by San-Dia Polymer, Ltd.), HIMOSAB HS-960 (manufactured by Hymo Corporation), and EF POLYMER (EF Polymer Private Limited).

In an embodiment of the present invention, the water-absorbing polymer may be carboxymethyl cellulose. Carboxymethyl cellulose is a derivative of cellulose, in which carboxymethyl groups (-CH₂-COOH) are bonded to some of the hydroxy groups of gluconopyranose monomers constituting the skeleton of cellulose. Furthermore, this carboxymethyl cellulose may also be a carboxymethyl cellulose salt. Carboxymethyl cellulose is a thickening agent that has a high affinity for water and becomes a gel-like high viscosity body when mixed with water. In the present invention, when the treatment agent thickens, the effect of suppressing foul odors is enhanced, and the suppressive effect can be further maintained.

According to an embodiment of the present invention, the average particle size of the water-absorbing polymer is 1 to 1200 µm, 50 to 1100 µm, 10 to 1000 µm, 80 to 850 µm, 100 to 600 µm, 150 to 500 µm, or 200 to 400 µm.

According to an embodiment of the present invention, the treatment agent may be granulated using water or binders such as PVA, cellulose, water-soluble cellulose, water-soluble carboxymethyl cellulose, and sodium alginate. That is, according to an embodiment of the present invention, the above-described treatment agent is a lump-like treatment agent. Furthermore, the treatment agent according to an embodiment of the present invention may be in the form of a granular treatment agent or a lump-like treatment agent using the technologies of JP 2013-6137 A and JP 2011/162244 A. According to an embodiment of the present invention, the treatment agent contains a lubricating agent. The lubricating agent is used when producing a lump-like treatment agent, particularly in order to facilitate smooth feeding into the mortar of a tableting machine. Therefore, regarding the type of the lubricating agent, conventionally known lubricating agents may be used by appropriately selecting or combining them. For example, ester-based agents, silicon-based agents, stearic acid esters, calcium stearate, potassium stearate, calcium, and zinc are used.

According to an embodiment of the present invention, the average diameter of the treatment agent is 3 to 100 mm, or 5 to 50 mm. In the present specification, unless particularly stated otherwise, the term "average diameter" means an average value obtained by arbitrarily selecting a statistically reliable number of particles (or 10, 100, 200, 300, or 1000 particles), measuring the longest particle size for each particle using a vernier caliper, and taking the arithmetic mean of those values. The excrement treatment agent 20 is not limited to a lump-like or granular form, and may also be formed in a powdered form. In this case, according to an embodiment, the average particle size of the treatment agent is 10 µm or more, 30 µm or more, 50 µm or more, 100 µm or more, 300 µm or more, 500 µm or more, 700 or more, 1 mm or more, or 2 mm or more. According to an embodiment, the average particle size of the treatment agent is 3 mm or less, 2 mm or less, 1000 µm or less, 700 µm or less, 500 µm or less, 300 µm or less, 100 µm or less, 50 µm or less, or 30 µm or less. According to an embodiment, the average particle size of the treatment agent is 10 µm to 30 µm, 30 µm to 50 µm, 50 µm to 100 µm, 100 µm to 300 µm, 300 µm to 500 µm, 500 µm to 700 to 1000 µm, 1 mm to 2 mm, or 2 mm to 3 mm.

### <Lid part>

The lid part 30 is configured to be attachable to the container so as to close the opening part 11 of the container 10. By configuring the lid part in this way, the internal space 12 of the container 10 can be enclosed in a state in which the user has attached the excrement treatment agent 20 and the partition member 40 to the internal space 12 of the container 10.

As shown in Fig. 5 and the like, the lid part 30 includes a covering part 31, a flange part 32, a connecting part 33 (corresponding to an inlet part), a connecting part 34 (corresponding to an outlet part), and an engagement recess part 35. The covering part 31 is configured to cover the opening part 11 of the container 10 in a state in which the lid part 30 is mounted on the container 10. The covering part 31 is formed in a shape provided with a level difference having a recessed shape approximately at the center, excluding the shapes of the connecting parts 33 and 34; however, as long as the covering part 31 can cover the opening part of the container 10 in a state of being mounted on the container 10, the specific shape is not limited to the above-described shape and may be a simple flat plane.

The flange part 32 is formed so as to fall in the height direction Z from the outer peripheral edge part of the covering part 31. The flange part 32 is configured so as to cover the upper outer surface of the container in a state of being mounted on the container 10. The flange part 32 is configured so as to provide a shape such as a female thread that can be screwed into a threaded part provided on the outer surface of the container 10. A fastening part (fastening shape) such as a female screw provided on the flange part 32 is not particularly limited to a specific shape, as long as the flange part can generate a suction force in the internal space 12 when the lid part 30 is mounted on the container 10, or the generated suction force is not drastically reduced due to gas leakage or the like is not drastically reduced due to unintended gas leakage or the like.

The connecting parts 33 and 34 are provided so as to pass through the inside and the outside of the lid part 30, and are configured to allow attachment of a coupling member 50 capable of connecting a tubular member T that allows body fluids to flow through the internal space 12 of the container 10 by suction. Through the connection between the coupling member 50 and the tubular member T, the connecting part 33 functions as an inlet part that allows body fluids and the like to flow to the internal space 12 by suction, and the connecting part 34 functions as an outlet part that allows at least one of gases and body fluids discharged from the internal space 12 by suction to flow out to the downstream side.

The engaging recess part 35 is configured to be able to engage with an engaging protrusion part p3 such that the partition member 40 can be positioned in the internal space 12 in a state in which the lid part 30 is mounted on the container 10. The engaging recess part 35 is configured so as to be provided on the side of the internal space 12 in a state in which the lid part 30 is mounted on the container 10. The engaging recess part 35 is configured so as to provide two cylindrical-shaped recesses that can engage with the engaging protrusion part p3 of the partition member 40 in the present embodiment (see Fig. 6). However, as long as the partition member can be positioned relative to the lid part, the engaging shape or the number of engaging shapes are not limited to those described above.

### <Partition member>

The partition member 40 is disposed in the internal space 12 as shown in Fig. 5 and the like, and is configured to partition the internal space 12 into a first internal space 13 that is proximal to the opening part 11, and a second internal space 14 that is located distal to the opening part 11 relative to the first internal space 13. The partition member 40 allows body fluids that flow from the connecting part 33 into the internal space 12, to flow between the first internal space 13 and the second internal space 14. Furthermore, the partition member 40 is configured to be capable of blocking the flow of the swollen material w (see dash-dot-dot line in Fig. 5) produced when body fluids that have flowed into the second internal space 14 come into contact with the excrement treatment agent 20, into the first internal space 13.

The partition member 40 is disposed closer to the opening part 11 than the excrement treatment agent 20 in the internal space 12. The partition member 40 includes a first member 41 and second members 42 as shown in Fig. 8. The first member 41 and the second members 42 are configured to have a small (thin) thickness dimension in the height direction Z and are laminated in the thickness direction to form an integrated structure.

The first member 41 allows body fluids to flow between the first internal space 13 and the second internal space 14.

The second members 42 are formed as a pair in the present embodiment so as to sandwich the first member 41. The second member 42 includes a covering part p1, a hole part p2, an engaging protrusion part p3, a first wall part p4, and a second wall part p5, as shown in Fig. 8 and the like.

The covering part p1 is configured as a portion that partially covers one side of the first member 41 without exposing the one side. The hole part p2 is configured as a portion at which one side of the first member 41 is exposed. In the present embodiment, it is configured that the covering part p1 is provided on a relatively outer side in the diameter direction r of the partition member 40, and the hole part p2 is provided on a relatively inner side in the diameter direction r of the partition member 40. Incidentally, the specific shape of the hole part p2 is not limited to the shape shown in the figure as long as the hole part can allow body fluids and the like to flow from the first internal space 13 to the second internal space 14 and inhibit the movement of the swollen material w from the second internal space 14 to the first internal space 13.

The engaging protrusion part p3 is configured to be capable of engaging with the engaging recession part 35 of the lid part 30 as described above. In the present embodiment, two engaging protrusion parts p3 are provided, similarly to the engaging recess parts 35 of the lid part 30; however, the specific number does not have to be two as described above, and the shape may be a shape other than that shown in Fig. 8 or the like. Furthermore, in Fig. 6, an engaging recess part 35 is provided on the lid part 30, and an engaging protrusion part p3 is provided on the partition member 40; however, as long as the two can be engaged with each other, a protrusion may be provided on the lid part, while a protrusion part may be provided on the lid part, and a recess part may be provided on the partition member in contrary to the above.

The first wall part p4 is disposed in the first internal space 13 in a state in which the partition member 40 is installed in the internal space 12, and is configured to be disposed between the connecting part 33 and the connecting part 34 and adjacent to the connecting part 34. In this way, body fluids that have flowed from the connecting part 33 into the internal space 12 can be prevented or suppressed from flowing to the downstream side through a flow path of the coupling member 50 mounted on the connecting part 34 as shown by arrow r1 in Fig. 7, without flowing into the second internal space 14 as shown by arrow r2 in Fig. 7.

The second wall part p5 is provided at the outer peripheral edge part of the coating part p1 or near the outer peripheral edge part. The second wall part p5 is configured to be disposed adjacent to the connecting part 33 in the first internal space 13 in a state in which the partition member 40 is installed in the internal space 12. There is a possibility that a gap may be formed between the partition member 40 and the container 10 in a state in which the partition member 40 is installed in the container 10; however, by providing the second wall part p5, the flow of body fluids flowing in from the connecting part 33 toward locations other than the boundary between the container 10 and the partition member 40, can be promoted. That is, in the present specification, the partition member 40 in the present specification allows a gap to be formed between the inner wall surface of the container 10 and the outer peripheral edge part of the partition member 40.

The second members 42 are disposed in a pair on the outside so as to sandwich the first member 41 therebetween. However, when the partition member 40 includes the first member, the first member does not have to be sandwiched between the second members. Furthermore, the second members 42 use the same component parts in the upper side and the lower side relative to the state of installation of the first member 41 into the container 10. However, when the second first wall part p4 and the second wall part p5 are provided on the upper second member in the installed state, the lower second member does not need to have the first wall part and the second wall part. The second member 42 is configured to have higher rigidity than the first member 41.

Regarding the material of the partition member 40, the first member 41 may include cotton, nonwoven fabric, woven fabric, cotton, paper, and the like. The second member 42 can include a material such as polyethylene or polypropylene. It is desirable that the pore size of the first member 41 such as a nonwoven fabric is smaller than the size of the swollen water-absorbing resin. Furthermore, the partition member 40 can be subjected to a hydrophilic surface treatment on the surface of the first internal space 13 side in order to promote the flow of body fluids flowing into the first internal space 13 to the second internal space 14. The surface treatment of the partition member 40 on the second internal space 14 side may or may not be performed; however, it is preferable that no surface treatment is provided.

Here, the dimensions of the partition member 40 and related portions such as the container 10, which prevent the swollen material w from flowing into he first internal space 13 when the swollen material w is produced in the second internal space 14, will be described with examples. The dimension d1 in the diameter direction r (see Fig. 5) of the opening part 11 of the container 10 can be configured to be about 135 mm. It is preferable that the size of the hole part p2 of the first member 41 is smaller than the swollen material w produced when brought into contact with the excrement treatment agent 20 in the second internal space 14 so that the swollen material 2 thus produced does not flow to the first internal space 13. Regarding the hole part p2 of the partition member 40, the dimensions of each portion of a plurality of hole shapes constituting the hole part p2 in the planar direction XY can be set such that the hole part p2 occupies 1% to 40% of the area of the covering part p1 when viewed in a plan view from the height direction Z in a case where it is assumed that the hole part p2 does not exist. The thickness of the first member 41 and the thickness of the second member 42 (see Fig. 8) of the partition member 40 can be configured to be approximately 1.4 mm. When the excrement treatment agent 20 is considered to approximate to a cylinder, the dimension d2 in the diameter direction r can be configured to be about 13 mm, and the dimension d3 in the height direction Z (see Fig. 5) can be configured to be 5 to 6 mm.

### <Coupling member>

The coupling member 50 is used to link adjacent waste liquid treatment implements 100 or downstream-side waste liquid treatment implements 100a to each other through a tubular member T. The coupling member 50 is mounted in the connecting parts 33 and 34 of the opening part 30 through which body fluids flow in and out of the internal space 12 in the lid part 30. The coupling member 50 includes an outer side part 51 and an inner side part 52 as shown in Fig. 5. The coupling member 50 is formed into a hollow cylinder shape such that in a state in which the lid part 30 is attached, the connecting part 33 allows body fluids from the upstream side to flow to the internal space 12, and the connecting part 34 allows body fluids located in the internal space 12 of the container 10 to flow to the downstream side.

The outer side part 51 corresponds to a portion that is disposed on the outer side with respect to the internal space 12 in a state of being attached to the lid part 30. The outer side part 51 is formed to bend in a substantially L-shape in the present embodiment, and is configured to have a tubular member T connected thereto at the tip part. Here, regarding the shape of the outer side part, the specific shape is not limited to an L-shape as long as a tubular member T can be connected thereto, and the shape may also be a linear shape or the like.

The inner side part 52 corresponds to a portion that is located in the internal space 12 in a state of being attached to the lid part 30. The outer side part 51 and the inner side part 52 are provided with a flow path through which body fluids can flow to the internal space 12 and the outside. The inner side part 52 is configured such that sealing members such as rubber or elastomer are provided at the connecting parts 33 and 34 to prevent the leakage of body fluids from portions other than the flow path. The coupling member 50 can abut against a displacement member 70 of the downstream-side waste liquid treatment implement 100a that will be described above at the lower end of the inner side part 52, and the portion that abuts against the displacement member 70 and becomes a portion of the flow path is referred to as an opening end part 53 (see Fig. 13). The details will be described below.

The tubular member T that is connected to the connecting parts 33 and 34 of the lid part 30 can be formed from known flexible materials such as plastics so that the tubular member T can be connected to the connecting parts 33 and 34 in various positions.

### <Downstream-side waste liquid treatment implement>

The downstream-side waste liquid treatment implement 100a is connected to the waste liquid treatment implement 100 through a tubular member T, and is configured to be disposed on the downstream side of the waste liquid treatment implement 100. The downstream-side waste liquid treatment implement 100a has a container 10a (corresponding to a second container), an excrement treatment agent 20, a lid part 30a (corresponding to a second lid part), a coupling member 50, an attachment member 60, and a displacement member 70, as shown in Fig. 10. Since the container 10a is similar to the container 10 of the waste liquid treatment implement 100, and the excrement treatment agent 20 and the coupling member 50 are similar to the respective constituent components of the waste liquid treatment implement 100, detailed description will not be repeated herein.

### <Lid part>

The lid part 30a is configured to be attachable to the container 10a so as to close the opening part 11 of the container 10a. The lid part 30a includes a covering part 31, a flange part 32, a connecting part 33 (corresponding to an inlet part), and a connecting part 34a (corresponding to a second outlet part), as shown in Fig. 11. Unlike the lid part 30, the lid part 30a does not include an engaging recess part 35; however, the lid part 30a includes a covering part 31, a flange part 32, and a connecting part 33 similarly to the lid part 30. Furthermore, the connecting part 34a is configured to allow the flow of gas discharged from the internal space 12 by suction to the downstream side while not allowing the flow of the swollen material w; however, unlike the connecting part 34, the connecting part 34a is configured not to allow the flow of body fluids to the downstream side by suction. However, the specific shape of the connecting part 34a can be configured similarly to that of the connecting part 34. Therefore, the description of the covering part 31, the flange part 32, and the connecting parts 33 and 34 will not be repeated.

### <Attachment member>

The attachment member 60 is configured so as to be attachable to the lid part 30a, allow the flow of gas and the like suctioned by a pump, and place a displacement member 70 that will be described below when the displacement member 70 does not block the flow of the swollen material w. As shown in Fig. 12, Fig. 13, and the like, the attachment member 60 includes a fixing part 61, a hanging part 62, an abutment part 63, a flow hole 64, and an insertion hole 65.

The fixing part 61 includes a flange provided with a hole that can be engaged by, for example, fitting with a protrusion shape (not shown in the diagram) provided on the lid part 30a in order to attach the attachment member 60 to the lid part 30a. The fixing part 61 is configured to include a flange that extends horizontally in the planar direction XY from the end part of the hanging part 62.

The hanging part 62 is configured to extend downward from the inner end part of the flange of the fixing part 61. The hanging part 62 extends linearly in parallel to the height direction Z in the present embodiment; however, as long as the flow hole 64 that will be described below can be provided, the hanging part 62 may be inclined or curved with respect to the height direction Z.

The abutment part 63 maintains the displacement member 70 in the vicinity of the connecting part 34a of the internal space 12 when the displacement member 70 does not block the flow path of the coupling member 50 mounted on the connecting part 34a. The abutment part 63 is configured to extend from the lower end part of the hanging part 62 toward the inside of the hanging part 62 in the planar direction XY, as shown in Fig. 13 and the like. The abutment part 63 is formed to be substantially flat so as to be able to abut against a drop preventing part 72 of the displacement member 70. When the displacement member 70 does not at least partially close the opening end part 53 of the coupling member 50, the displacement member 70 can be placed on the abutment part 63.

The flow hole 64 is a notch provided in the hanging part 62 as shown in Fig. 12, and is configured that gas and the like suctioned by a pump or the like to flow between the internal space 12 and the outside through the flow path of the coupling member 50. Regarding the flow hole 64, a plurality of them can be provided on the lateral surface of the hanging part 62, particularly in the circumferential direction θ as shown in Fig. 12. The insertion hole 65 is configured such that the displacement member 70 that will be described below can move in the height direction Z relative to the attachment member 60 by inserting the insertion part 73 of the displacement member 70 through the insertion hole 65. The insertion hole 65 is configured to be provided approximately at the center of the abutment part 63.

### <Displacement member>

The displacement member 70 is configured to abut against or move away from the opening end part 53 of the coupling member 50 provided in the connecting part 34a. The displacement member 70 includes a closure part 71, a drop preventing part 72, an insertion part 73, an opposite end part 74, and a floating part 75 as shown in Fig. 13.

The closure part 71 is configured to be capable of at least partially clogging the flow path of the coupling member 50 mounted on the connecting part 34a. The closure part 71 is configured to include a curved surface provided on top of the displacement member 70. By abutting this curved surface against the opening end part 53 of the inner side part 52 of the coupling member 50, the body fluids and swollen material w that may be present in the internal space 12 can be prevented from flowing through the flow path of the coupling member 50 mounted on the connecting part 34a.

The drop preventing part 72 is provided on the opposite side of the curved surface of the closure part 71 in the present embodiment, and is configured to be able to abut against the abutment part 63 of the attachment member 60. The drop preventing part 72 is configured such that by abutting against the abutment part 63, the attachment member 60 can maintain the displacement member 70 at a position in the vicinity of the connecting part 34a in the internal space 12. The drop preventing part 72 can be configured to include a flat surface or the like provided on the opposite side of the curved surface of the closure part 71.

The insertion part 73 can be inserted into the insertion hole 65 of the attachment member 60, and in the present embodiment, the insertion part 73 is configured to include a cylindrical shape. This allows the displacement member 70 to be moved to a position at which the closure part 71 of the displacement member 70 clogs the flow path of the coupling member 50, or to be moved to to a position at which the drop preventing part 72 abuts against the abutment part 63. Incidentally, the specific shape of the insertion part 73 is not limited to the above-mentioned cylindrical shape as long as the closure part 71 can move to a position that clogs the flow path of the coupling member 50 or move to a position that does not clog the flow path.

The opposite side end 74 is configured to be provided on the opposite side of the closure part 71 with respect to the insertion part 73. As the opposite end part 74 is formed so as to protrude outward from the center of the insertion part 73 in the planar direction XY due to the shape of the insertion part 73, the opposite end part 74 can function as a stopper preventing the displacement member 70 from moving too far upward relative to the attachment member 60.

The floating part 75 is provided at the lower end in the direction of gravity in an installed state, and is configured to be able to float the displacement member 70 above the liquid surface in a state of being brought into contact with the liquid surface of the body fluids in the internal space 12. Here, the liquid surface formed by the fluid flowing into the internal space 12 may contain not only the body fluids but also the swollen material w; however, hereinafter, the liquid surface will be described simply as "liquid surface of body fluids". The floating part 75 can be joined to the opposite end part 74 by adhesion, fusion, or the like. The floating part 75 can be constructed from a relatively lightweight member separately from the members that provide the closure part 71, the drop preventing part 72, the insertion part 73, and the opposite end part 74. The floating part 75 can be configured to include urethane or a material called foamed plastic such as styrene foam. Incidentally, in Fig. 13 and the like, the displacement member 70 is formed such that the opposite end part 74 protrudes outward in the diameter direction r so as to prevent the displacement member 70 from escaping upward from the insertion hole 65 of the attachment member 60. However, as long as the floating part 75 has a shape that does not pass through the insertion hole 65 when moving upward, the opposite end part 74 does not need to have a shape protruding outward in the diameter direction r so as to be unable to pass through the insertion hole 65.

Furthermore, the displacement member 70 can be provided with a flow mechanism that, during suction, suppresses the suction of liquid while allowing the suction of gas. As an example of the flow mechanism, an insertion hole h1 (see Figs. 13 and 14) having a size of about several millimeters is provided on the members that provide the closure part 71, the drop preventing part 72, the insertion part 73, and the opposite end part 74, and a large number of extremely tiny holes can be provided by foamed plastic or the like in the floating part 75.

When the drop preventing part 72 abuts against the abutment part 73 of the attachment member 60 as shown in Fig. 13, the displacement member 70 is in a state that does not close the opening end part 53 of the inner part 52 of the coupling member 50. This allows the gas or the like present in the internal space 12 of the container 10a to flow through the flow path of the coupling member 50 from the opening end part 53 of the coupling member 50 through the flow hole 64 as shown by the arrows in Fig. 12. On the other hand, when the liquid surface of the body fluids present in the internal space 12 further rises from a state of being in contact with the floating part 75, the drop preventing part 72 separates apart from the abutment part 63, the displacement member 70 moves upward, and the blocking preventing part 71 abuts against the opening end part 53 of the inner part 52 of the coupling member 50. This allows the opening end part 53 to be in a state of being blocked by the closure part 71. As a result, liquids such as body fluids and the swollen material w present in the internal space 12 are in a state in which the liquids cannot flow through the opening end part 53 of the coupling member 50. On the other hand, gas present in the internal space 12 can flow downstream through the tiny holes in the floating part 75 and the insertion hole h1 even in a state in which the closure part 71 abuts against the opening end part 53 of the inner part 52.

As described above, in the present embodiment, the waste liquid treatment implement 100 has a container 10, an excrement treatment agent 20, a lid part 30, and a partition member 40. The container 10 is provided with an internal space 12 capable of holding body fluids that are included in a human being or discharged from a human being, and an opening part 11 through which the body fluids can be introduced into the internal space 12 by suction. The lid part 30 can be attached so as to close the opening part 11 of the container 10, and includes a connecting part 33 that allows body fluids to flow to the internal space by suction. The partition member 40 is disposed in the internal space 12 and is configured to divide the internal space 12 into a first internal space 13 that is proximal to the opening part 11, and a second internal space 14 that is located distal to the opening part 11 relative to the first internal space 13. The excrement treatment agent 20 is disposed in the second internal space 14 of the container 10 and is configured to convert body fluids into a solid swollen material w when brought into contact with body fluids. The partition member 40 allows body fluids flowing from the connecting part 33 into the internal space 12 to flow between the first internal space 13 and the second internal space 14. Furthermore, the partition member 40 is configured so as to block the flow of the swollen material 2 produced when the body fluids that have flowed into the second internal space 14 is brought into contact with the excrement treatment agent 20, into the first internal space 13.

By configuring in this way, the material produced when body fluids come into contact with the excrement treatment agent 20 can be prevented from clogging the connecting part 34, which is a flow path on the discharge port side of the lid part 30. Furthermore, even when a specimen is taken from a patient's body in a medical setting or the like, and the taken specimen is mistakenly collected in the internal space 12 of the container 10, the partition member 40 allows the above-mentioned swollen material w to be disposed in an internal space different from the first internal space 13 (second internal space 14) containing the specimen. This can prevent the swollen material w from mixing with the specimen, and the specimen can be relatively easily taken out from the internal space 12 of the container 10.

Furthermore, the partition member 40 includes a first member 41 that allows body fluids to flow between the second internal space 14 and the first internal space 13, and second members 42 having higher rigidity than the first member 41. With such a configuration, the partition member 40 can be made difficult to be deformed by the swollen material w when the swollen material w is produced in the second internal space 14. In this way, the swollen material w can be prevented from clogging the flow path of the connecting part 34 of the lid part 30 when the excrement treatment agent 20 swells due to contact with body fluids.

Furthermore, the lid part 30 includes a connecting part 34 as an outlet part through which at least one of gas and body fluids discharged from the internal space 12 by suction can flow to the downstream side. The second members 42 are disposed in the first internal space 13 in a state in which the partition member 40 is installed in the internal space 12, and includes a first wall part p4 disposed between the connecting part 33 and the connecting part 34 and adjacent to the connecting part 34. With such a configuration, body fluids flowing in from the connecting part 33 can be prevented or suppressed from flowing from the connecting part 34 to the downstream side without moving to from the first internal space 13 to the second internal space 14 through the hole part p2.

Furthermore, the second member 42 includes a coating part p1 and a second wall part p5. The coating part p1 partially covers one side of the first member 41. The second wall part p5 is disposed at the outer peripheral edge part or in the vicinity of the outer peripheral edge part and adjacent to the connecting part 33 in the first internal space 13 in a state in which the partition member 40 is installed in the internal space 12. In this way, the body fluids that have flowed from the connecting part 33 into the internal space 12 can be suppressed from reacting only with the excrement treatment agent 20 disposed below the connecting part 33 int eh second internal space 14, and the flow of body fluids to the excrement treatment agent 20 disposed below the connecting part 34 can be promoted.

Furthermore, the second members 42 are disposed in a pair so as to sandwich the first member 41. With such a configuration, the partition member 40 can be made difficult to deform in the internal space when body fluids come into contact with the excrement treatment agent 20 in the second internal space 14, and a swollen material w is produced. In this way, the swollen material w can be prevented from clogging the flow path of the connecting part 34 of the lid part 30 when the excrement treatment agent 20 swells due to contact with body fluids.

Furthermore, in the present embodiment, it is configured that the partition member 40 is provided with an engaging protrusion part p3 so as to position the partition member 40 in the internal space 12 in a state in which the lid part 30 is mounted on the container 10, and the lid part 30 is provided with an engaging recess part 35. With such a configuration, the partition member 40 can be made difficult to shift from the intended position in the internal space 12, and thereby the swollen material w can be prevented from clogging the flow path of the connecting part 34 of the lid part 30 when the excrement treatment agent 20 swells due to contact with body fluids.

Furthermore, the downstream-side waste liquid treatment implement 100a has a container 10a, a lid part 30a that can be attached to the container 10a so as to close the opening part 11 of the container 10a, an excrement treatment agent 20, and a displacement member 70. The container 10a is provided with an internal space 12 and an opening part 11. The lid part 30a can be attached to the container 10a so as to close the opening part 11 of the container 10a, and includes a connecting part 33 and a connecting part 34a that allows gas discharged from the internal space 12 by suction to flow to the downstream side. The excrement treatment agent 20 is placed in the internal space 12 of the container 10a, and is configured to convert body fluids into a swollen material w when brought into contact with body fluids. The displacement member 70 is configured to block the flow of body fluids and the swollen material w to the downstream side when the excrement treatment agent 20 produced upon contact of the excrement treatment agent 20 with body fluids reaches a predetermined height in the internal space 12.

With such a configuration, the swollen material w in the internal space 12 of the downstream-side waste liquid treatment implement 100a can be collected, and the body fluids and the swollen material w can be prevented from flowing to the downstream side.

Furthermore, the downstream-side waste liquid treatment implement 100a includes an attachment member 60. The attachment member 60 can be attached to the lid part 30a and is configured to have an abutment part 63 that maintains the displacement member 70 in the vicinity of the connecting part 34a of the internal space 12 when the displacement member 70 does not block the flow of body fluids and the swollen material w. In this way, when the liquid surface of body fluids rises in the internal space 12 of the container 10a, the displacement member 70 can be put on standby so that the flow of the body fluids and the swollen material w through the flow path of the coupling member 50 mounted on the connecting part 34a can be interrupted by the displacement member 70.

Furthermore, the displacement member 70 includes a closure part 71 and a drop preventing part 72. The closure part 71 is configured to be able to partially block the flow path of the coupling member 50 mounted on the connecting part 34a. The drop preventing part 72 can abut against the abutment part 63 of the attachment member 60, and by this abutment, maintains the displacement member 70 at a position in the vicinity of the connecting part 34a in the internal space 12. In this way, the displacement member 70 can be put on standby at a position where the displacement member 70 can interrupt the flow of body fluids and the swollen material w, when body fluids flow into the internal space 12 of the container 10a, and the body fluids do not clog the flow path of the coupling member 50 mounted on the connecting part 34a.

Furthermore, the displacement member 70 is configured to include a floating part 75 that is provided at the lower end in the direction of gravity and is capable of floating the displacement member 70 on the liquid surface of body fluids in a state of being in contact with the liquid surface of the body fluids in the internal space 12. In this way, the displacement member 70 can be easily moved upward in accordance with the rise of the liquid surface of body fluids, and the displacement member 70 can be moved to a position that blocks the flow path of the coupling member 50 so that the body fluids and the swollen material w do not flow through the flow path of the coupling member 50 mounted on the connecting part 34a.

Furthermore, the displacement member 70 can be provided with a flow mechanism that, during suction, suppresses the suction of liquid while allowing the suction of gas. With such a configuration, even in a case where the closure part 71 blocks the opening end part 53 of the inner part 52 of the coupling member 50, gas and the like can be suctioned by a pump or the like disposed on the downstream side.

The present invention is not limited only to the above-described embodiments, and various modifications can be made within the scope of the claims. In Fig. 2 and the like, it has been described that two waste liquid treatment implements 100 are prepared, and one downstream-side waste liquid treatment implements 100a is prepared. However, as long as the body fluids and the like required in medical settings, disaster sites, or the like can be treated, the specific numbers of the waste liquid treatment implements and the downstream-side waste liquid treatment implements are not limited to those shown in Fig. 2 and the like. Furthermore, as long as the required body fluids can be treated, the waste liquid treatment implement 100 may be prepared while not preparing the downstream-side waste liquid treatment implement 100a.

Fig. 15 is an exploded perspective view of a waste liquid treatment implement 100b according to a modification example of Fig. 4, and Fig. 16 is an exploded perspective view of a downstream-side waste liquid treatment implement 100c according to a modification example of Fig. 10. In the above description, it has been described that the excrement treatment agent 20 and the like are held in the internal space 12 of the container 10 of the waste liquid treatment implement 100 or the downstream-side waste liquid treatment implement 100a, and when body fluids come into contact with the excrement treatment agent 20 to produce the swollen material w, the swollen material w and the like present in the internal space 12 can be discarded together with the container 10. However, as long as the swollen material w and the like can be disposed of, it is not necessary to discard the container 10 together with the swollen material w every time the swollen material w is produced. That is, a disposable bag 80 such as a plastic bag may be disposed in the internal space 12 of the container 10 of the waste liquid treatment implement 100b or the downstream-side waste liquid treatment implement 100c, and then the excrement treatment agent 20 may be placed inside the disposable bag 80 (see Fig. 15 and Fig. 16).

As the disposable bag 80, the above-mentioned plastic bag, a drawstring bag, or a bag with a zipper can be utilized. With such a configuration, when the swollen material w is produced, the disposable bag 80 can be discarded together with the swollen material w, and at least one of the containers 10 and 10a of the waste liquid treatment implement and the downstream-side waste liquid treatment implement can be reused.

Furthermore, it has been described above that the partition member 40 includes the first wall part p4 and the second wall part p5. However, as long as the body fluids that have flowed from the connecting part 33 into the internal space 12 can flow to the second internal space 14, the partition member 40 may be configured not to include the first wall part p4 and the second wall part p5, or to include only one of the first wall part p4 and the second wall part p5.

Furthermore, it has been described that the displacement member 70 includes a floating part 75; however, as long as the displacement member can come into contact with the liquid surface of body fluids in the internal space 12 of the container 10a and move upward along with the rise of the liquid surface, the displacement member does not have to include the floating part 75.

Fig. 17 is a perspective view showing the configuration of a partition member 40d according to a modification example, Fig. 18 is an exploded perspective view of Fig. 17, Fig. 19 is a plan view of Fig. 17, and Fig. 20 is a perspective view showing a partition member 40e according to a modification example.

In Fig. 8 and the like, the partition member 40 has a first member 41 and second members 42, and the first member 41 is configured to be sandwiched between two second members 42. However, the specific form of the partition member is not limited to the above-described form, as long as the partition member allows the body fluids that flow from the connecting part 33 into the internal space 12 to flow between the first internal space 13 and the second internal space 14, and prevents the swollen material w produced in the second internal space 14 from flowing to the first internal space 13.

In addition to the above description, in the partition member 40d, a first member 41d provided with a plurality of hole parts may be disposed on the second member 42d as shown in Fig. 17 and the like. The first member 41d has a substantially circular shape and is provided with a large number of hole parts.

As shown in Fig. 18, the second member 42d includes a coating part p1d, a hole part p2d, and an engaging protrusion part p3d. The coating part p1d is formed to have a peripheral shape that is substantially the same as the horizontal cross-sectional shape of the container 10 at the installation position of the container 10. The hole part p2d can be provided, in at least a portion of the first member 41d in a state in which the first member 41d is installed, with an inlet side hole part and an outlet side hole part that allow body fluids to flow from the first internal space 13 to the second internal space 14 (see Fig. 18). The engaging protrusion part p3d is configured in the same manner as the engaging protrusion part p3 of the partition member 40. The portion of the partition member 40 corresponding to the irst wall part p4 can be installed on the first member 41d as a wall member 43d. The wall member 43d is disposed closer to the connecting part 34 on the path between the connecting part 33 and the connecting part 34 of the lid part 30 through which body fluids flow in the internal space 12. The wall member 43d prevents or suppresses body fluids from flowing from the first internal space 13 to the downstream side without passing through the second internal space 14, similarly to the first wall part p4 of the partition member 40.

Furthermore, the partition member 40d is configured to have the first member 41d, the second member 42d, and the wall member 43 each as separate parts; however, these may be formed as a single part as in the case of the partition member 40e shown in Fig. 20. In this case, instead of providing the hole part p2d in the partition member 40d, a large number of hole parts of the first member 41d can be used as the hole part p2e. The covering part p1e is a flat portion outside the hole part p2e where the hole part p2e is not provided, and the engaging protrusion part p3e and the first wall part p4e can be configured similarly to the engaging protrusion part p3 and the first wall part p3d of the partition member 40d, respectively. The second wall parts p5e, p6e, and p7e include a second wall part p5e having a relatively larger dimension, and second wall parts p6e and p7e that have smaller dimensions compared to the second wall part p5e and are spaced apart from each other in the circumferential direction and disposed in a pair. However, as long as body fluids can flow through the hole part p2e to the second internal space 14 on the partition member 40, the second wall parts p6e and p7e do not need to be provided.

Fig. 21 is a diagram showing a modification example of Fig. 13, and Fig. 22 is a diagram showing a modification example of Fig. 14. In Fig. 13 and Fig. 14, the displacement member 70 of the downstream-side waste liquid treatment implement 100a includes a closure part 71, a drop preventing part 72, an insertion part 73, an opposite end part 74, and a floating part 75. The closure part 71 of the displacement member 70 is provided at the upper tip part of the displacement member 70; however, the configuration is not limited to this, the closure part 71d can be provided at the tip surface of the floating part 75d. The drop preventing part 72 of the displacement member 70 is provided on the opposite side of the closure part 71; however, the drop prevention part 72d of the displacement member 70d can be produced to have a step-shaped end surface that can be made of plastic or the like. Furthermore, the insertion part 73d can be produced to have a stepped cylinder-shaped vertical wall. Furthermore, the opposite end part 74d can be produced to have a cylindrical-shaped end part that comes into contact with the floating part 75. The floating part 75d is joined integrally with a cylindrical shape having the drop preventing part 72d and the opposite end part 74d, and can be constructed from a foam material such as urethane.

Fig. 23 is a perspective view showing a modification example of Fig. 2, Fig. 24 is an exploded perspective view showing a capture assembly 80d, and Fig. 25 is a diagram showing the inside of the capture assembly 80d. The waste liquid treatment implement 100 and the waste liquid treatment implement 100 can be connected to each other by a coupling member 50; however, a capture assembly 80d (corresponding to a capture part) may be attached to the connecting part 33 of the waste liquid treatment implement 100 located most upstream among the waste liquid treatment implements 100 shown in Fig. 2, instead of the coupling member 50 (see Fig. 23).

The capture assembly 80d includes a lid part 81, a container 85, and a compartment part 91 as shown in Fig. 24.

The lid part 81 includes an attachment part 82, an upper part 83, and a lateral part 84. The upper part 83 is configured to close an opening part 88 of the container 85. The lateral part 84 is provided to be continuous with the upper part 83 on the outer periphery of the upper part 83 and is configured to be connectable to the container 85. The inside of the lateral part 84 can be fitted to the container 85 or can be provided with thread shapes or groove shapes to be connected to the container 85. The attachment part 82 can be connected to a tubular member T or the like, and is provided with a hole part that allows body fluids, polyps, and the like discharged from a patient to flow therethrough.

The container 85 includes a wall surface 86, a level difference part 87, an opening part 88, and a discharge part 89. The wall surface 86 is configured to form a side wall and a bottom wall of the container 85. The level difference part 87 is provided at a middle position of the inner side wall in the wall surface 86 such that a compartment part 91 that will be described below can be installed. The opening part 88 is a portion that is blocked by the lid part 81, and is provided on top of the container 85. The discharge part 89 is provided in the lower part of the container 85 and is provided so as to allow the body fluids that have flowed in from the opening part 88 to flow to the downstream side.

The compartment part 91 includes a filter 92, partitions 93, and a support pillar 94. The filter 92 is provided with a large number of hole parts so as to separate solid components such as polyps included in the body fluids that have flowed in through the attachment part 82, from the body fluids. The partitions 93 are provided above the filter 92 at predetermined intervals in the angular direction. The partitions 93 are provided with a shape that divides the upper part of the filter 92 in the circumferential direction as viewed in a plan view so as to provide a plurality of places for disposing the solid components separated from the body fluids on the filter 92. The partition part 91 is configured to be rotatable relative to the container 85, and by rotating and changing the positions of the partitions 93 relative to the attachment part 82, for example, solid components can be captured on the filter 92 separately for each patient. The support pillar 94 is provided so as to be integrated with a plurality of partitions 93 approximately at the center of the partitions 93. With such a configuration, solid components such as polyps can be removed (separated) relatively quickly from the waste liquid to be treated.

The present invention includes the following aspects and forms.
1. A waste liquid treatment implement, including:
   a container provided with an internal space capable of holding body fluids included in a human being or discharged from a human being, and an opening part allowing the body fluids to be introduced into the internal space by suction;
   a lid part attachable to the container so as to close the opening part of the container, and having an inlet part allowing the body fluids to flow to the internal space by the suction;
   a partition member disposed in the internal space and dividing the internal space into a first internal space proximal to the opening part and a second internal space located distal to the opening part relative to the first internal space; and
   an excrement treatment agent disposed in the second internal space of the container and converting the body fluids into a solid swollen material when brought into contact with the body fluids,
   wherein the partition member allows the body fluids flowing from the inlet part into the internal space to flow between the first internal space and the second internal space, and allows blocking of the flow of the swollen material produced when the body fluids that have flowed into the second internal space come into contact with the excrement treatment agent, to the first internal space.
2. The waste liquid treatment implement according to the above-described item 1, wherein the partition member includes a first member allowing the body fluids to flow between the second internal space and the first internal space, and a second member having higher rigidity than the first member.
3. The waste liquid treatment implement according to the above-described item 2, wherein the lid part includes an outlet part allowing at least one of gas and the body fluids discharged from the internal space by the suction to flow to the downstream side, and
   the second member is disposed in the first internal space in a state in which the partition member is installed in the internal space, and includes a first wall part disposed between the inlet part and the outlet part and adjacent to the outlet part.
4. The waste liquid treatment implement according to the above-described item 2 or 3, wherein the second member includes a covering part partially covering one side of the first member, and a second wall part that is disposed at an outer peripheral edge part or in the vicinity of the outer peripheral edge part of the covering part and is adjacent to the inlet part in the first internal space in a state in which the partition member is installed in the internal space.
5. The waste liquid treatment implement according to any one of the above-described items 1 to 4, wherein the inlet part allows attachment of a capture part including a filter capable of separating solid components contained in the body fluids from the body fluids.
6. The waste liquid treatment implement according to any one of the above-described items 1 to 5, wherein a protrusion part is provided on one of the lid part and the partition member while a recess part capable of engaging with the protrusion part is provided on the other one, so as to position the partition member in the internal space in a state in which the lid part is mounted on the container.
7. A downstream-side waste liquid treatment implement, including:
   a second container provided with the internal space and the opening part;
   a second lid part attachable to the second container so as to close the opening part of the second container and including the inlet part and a second outlet part allowing gas discharged from the internal space by the suction to flow to the downstream side;
   the excrement treatment agent disposed in the internal space of the second container and converting the body fluids into the swollen material when brought into contact with the body fluids; and
   a displacement member blocking the flow of the body fluids and the swollen material to the downstream side when the swollen material produced upon contact of the excrement treatment agent with the body fluids reaches a predetermined height in the internal space,
   wherein the downstream-side waste liquid treatment implement is connectable to the waste liquid treatment implement according to any one of the above-described items 1 to 6 through a tubular member.
8. The downstream-side waste liquid treatment implement according to the above-described item 7, further including an attachment member that is attachable to the second lid part and includes an abutment part maintaining the displacement member in the vicinity of the second outlet part of the internal space when the displacement member does not block the flow of the body fluids and the swollen material.
9. The downstream-side waste liquid treatment implement according to the above-described item 8, wherein the displacement member includes a closure part capable of at least partially clogging a flow path of the second outlet part, and a drop preventing part capable of abutting against the abutment part and maintaining the displacement member at a position in the vicinity of the second outlet part in the internal space by abutment.
10. The downstream-side waste liquid treatment implement according to any one of the above-described items 7 to 9, wherein the displacement member is provided at a lower end in a direction of gravity and includes a floating part capable of floating the displacement member on a liquid surface in a state of being in contact with the liquid surface of the body fluids in the internal space.
11. The downstream-side waste liquid treatment implement according to any one of the above-described items 7 to 10, wherein the displacement member includes a flow mechanism suppressing the suction of liquid while allowing the suction of gas during the suction.

The above is the description of the second invention.

The present application is based on Japanese Patent Application No. 2022-173428 and Japanese Patent Application No. 2022-173431, filed on October 28, 2022, the entire disclosures of which are incorporated herein by reference.

### EXPLANATIONS OF LETTERS OR NUMERALS

- 10: CONTAINER,
- 10a: CONTAINER (SECOND CONTAINER),
- 11: OPENING PART,
- 12: INTERNAL SPACE,
- 13: FIRST INTERNAL SPACE,
- 14: SECOND INTERNAL SPACE,
- 20: EXCREMENT TREATMENT AGENT,
- 30: LID PART,
- 30a: LID PART (SECOND LID PART)
- 33: CONNECTING PART (INLET PART),
- 34: CONNECTING PART (OUTLET PART),
- 34a: CONNECTING PART (SECOND OUTLET PART),
- 35: ENGAGING RECESS PART,
- 40: PARTITION MEMBER,
- 41: FIRST MEMBER,
- 42: SECOND MEMBER,
- 50: COUPLING MEMBER,
- 60: ATTACHMENT MEMBER,
- 63: ABUTMENT PART,
- 70: DISPLACEMENT MEMBER,
- 71: CLOSURE PART,
- 72: DROP PREVENTING PART,
- 75: FLOATING PART,
- 80: CAPTURE ASSEMBLY (CAPTURE PART),
- 92: FILTER,
- 100: WASTE LIQUID TREATMENT IMPLEMENT,
- 100a: DOWNSTREAM-SIDE WASTE LIQUID TREATMENT IMPLEMENT,
- h1: INSERTION HOLE (FLOW MECHANISM),
- p1: COATING PART,
- p3: ENGAGING PROTRUSION PART,
- p4: FIRST WALL PART,
- p5: SECOND WALL PART,
- T: TUBULAR MEMBER,
- w: SWOLLEN MATERIAL.

## Claims

1. A treatment agent for treating waste liquid, the treatment agent comprising slaked lime and pepper.

2. The treatment agent according to claim **1,** wherein the pepper is not in a form of piperine extract.

3. The treatment agent according to claim **1,** further comprising a water-absorbing polymer.

4. The treatment agent according to claim **1,** wherein a content ratio (% by mass) of the slaked lime is 0.1% to 40% by mass, and a content ratio (% by mass) of the pepper is 0.05% to 30% by mass.

5. The treatment agent according to claim **1,** further comprising zinc oxide.

6. The treatment agent according to claim 5, further comprising at least one selected from the group consisting of lignin, bentonite, and zeolite.

7. The treatment agent according to claim 6, further comprising humic acid.

8. The treatment agent according to claim 1, wherein the waste liquid includes excrement, blood, vomit, or body fluids discharged from a human being or an animal.

9. The treatment agent according to claim 1, wherein the waste liquid includes at least one of indole and skatole as a foul odor component.

10. The treatment agent according to claim 1, wherein the treatment agent is used to suppress or inhibit spore germination or proliferation of a spore-forming bacterium.

11. The treatment agent according to claim 10, wherein the spore-forming bacterium is *Clostridioides difficile.*

12. A method for suppressing or inhibiting spore germination or proliferation of a spore-forming bacterium, the method comprising:
bringing waste liquid into contact with the treatment agent according to any one of claims 1 to 11.

13. A waste liquid treatment implement, comprising:
a container provided with an internal space capable of holding body fluids included in a human being or discharged from a human being, and an opening part allowing the body fluids to be introduced into the internal space by suction;
a lid part attachable to the container so as to close the opening part of the container, and having an inlet part allowing the body fluids to flow to the internal space by the suction;
a partition member disposed in the internal space and dividing the internal space into a first internal space proximal to the opening part and a second internal space located distal to the opening part relative to the first internal space; and
an excrement treatment agent disposed in the second internal space of the container and converting the body fluids into a solid swollen material upon contact with the body fluids,
wherein the partition member allows the body fluids that flow from the inlet part into the internal space to flow between the first internal space and the second internal space; and is capable of blocking of the flow of the swollen material, produced upon contact between the body fluids that have flowed into the second internal space and the excrement treatment agent, to the first internal space.

14. The waste liquid treatment implement according to claim 13, wherein the partition member includes a first member allowing the body fluids to flow between the second internal space and the first internal space, and a second member having higher rigidity than the first member.

15. The waste liquid treatment implement according to claim 14, wherein the lid part includes an outlet part allowing at least one of gas and the body fluids discharged from the internal space by the suction to flow to a downstream side, and
the second member is disposed in the first internal space in a state in which the partition member is installed in the internal space, and includes a first wall part disposed between the inlet part and the outlet part and adjacent to the outlet part.

16. The waste liquid treatment implement according to claim 14, wherein the second member includes a covering part partially covering one side of the first member, and a second wall part that is disposed at an outer peripheral edge part of the covering part or in a vicinity of the outer peripheral edge part and is adjacent to the inlet part in the first internal space in a state in which the partition member is installed in the internal space.

17. The waste liquid treatment implement according to claim 13, wherein a capture part is attachable to the inlet part, the capture part including a filter capable of separating solid components contained in the body fluids from the body fluids.

18. The waste liquid treatment implement according to claim 13, wherein a protrusion part is provided on one of the lid part and the partition member, and a recess part capable of engaging with the protrusion part is provided on the other, so as to position the partition member in the internal space in a state in which the lid part is mounted on the container.

19. A downstream-side waste liquid treatment implement, comprising:
a second container provided with the internal space and the opening part;
a second lid part attachable to the second container so as to close the opening part of the second container and including the inlet part and a second outlet part allowing gas discharged from the internal space by the suction to flow to a downstream side;
the excrement treatment agent disposed in the internal space of the second container and converting the body fluids into the swollen material upon contact with the body fluids; and
a displacement member blocking the flow of the body fluids and the swollen material to the downstream side when the swollen material produced upon contact between the excrement treatment agent and the body fluids reaches a predetermined height in the internal space,
wherein the downstream-side waste liquid treatment implement is connectable to the waste liquid treatment implement according to claim 13 through a tubular member.

20. The downstream-side waste liquid treatment implement according to claim 19, further comprising an attachment member that is attachable to the second lid part and includes an abutment part maintaining the displacement member in a vicinity of the second outlet part of the internal space when the displacement member does not block the flow of the body fluids and the swollen material.

21. The downstream-side waste liquid treatment implement according to claim 21, wherein the displacement member includes a closure part capable of at least partially clogging a flow path of the second outlet part, and a drop preventing part capable of abutting against the abutment part of the attachment member and maintaining the displacement member at a position in the vicinity of the second outlet part in the internal space by abutment.

22. The downstream-side waste liquid treatment implement according to claim 19, wherein the displacement member is provided at a lower end in a direction of gravity and includes a floating part capable of floating the displacement member on a liquid surface in a state of being in contact with the liquid surface of the body fluids in the internal space.

23. The downstream-side waste liquid treatment implement according to claim 19, wherein the displacement member includes a flow mechanism suppressing the suction of liquid while allowing the suction of gas during the suction.
